# EUROPEAN PATENT SPECIFICATION

(11) **EP 3 908 662 B1**
(45) Date of publication and mention of the grant of the patent: **20.11.2024**
(21) Application number: 20738372.0
(22) Date of filing: 09.01.2020
(51) Int. Cl.: C07K 14/47, C12N 5/077, A61K 35/17, A61P 11/06, A61P 31/00, A61P 33/00, A61P 35/00, A61P 37/02, A61K 38/00

(54) **ENGINEERED TUBEROUS SCLEROSIS COMPLEX 2 POLYPEPTIDES**
MANIPULIERTE POLYPEPTIDE DES TUBERÖSEN SKLEROSE-KOMPLEXES 2
POLYPEPTIDES DU COMPLEXE 2 DE LA SCLÉROSE TUBÉREUSE MODIFIÉS

(30) Priority: 09.01.2019 US 201962790216 P
(43) Date of publication of application: 17.11.2021
(73) Proprietor: The Johns Hopkins University, Baltimore, Maryland 21218 (US)
(72) Inventor: KASS, David A., Baltimore, Maryland 21218 (US); RANEK, Mark J., Baltimore, Maryland 21218 (US); KOKKONEN, Kristen, Baltimore, Maryland 21218 (US); DUNKERLY-EYRING, Brittany, Baltimore, Maryland 21218 (US)
(74) Representative: Murgitroyd & Company
(86) International application number: PCT/US2020/012924
(87) International publication number: WO 2020/146625

(56) References cited:
- US-A1- 2016 251 410
- US-B2- 9 149 506
- MONIKA LINKE ET AL: "mTORC1 and mTORC2 as regulators of cell metabolism in immunity", FEBS LETTERS, ELSEVIER, AMSTERDAM, NL, vol. 591, no. 19, 23 June 2017 (2017-06-23), pages 3089 - 3103, XP071256298, ISSN: 0014-5793, DOI: 10.1002/1873-3468.12711
- BALLIF BRYAN A ET AL: "Quantitative phosphorylation profiling of the ERKp90 ribosomal S6 kinase-signaling cassette and its targets, the tuberous sclerosis tumor suppressors", PNAS, 18 May 2005 (2005-05-18), pages 667 - 672, XP055953726, Retrieved from the Internet <URL:www.pnas.org/cgi/doi/10.1073/pnas.0409143102> [retrieved on 20220822], DOI: 10.1073/pnas.0409143102
- MANNING BRENDAN D ET AL: "Identification of the Tuberous Sclerosis Complex-2 Tumor Suppressor Gene Product Tuberin as a Target of the Phosphoinositide 3-Kinase/Akt Pathway", MOLECULAR CELL, vol. 10, no. 1, 1 July 2002 (2002-07-01), AMSTERDAM, NL, pages 151 - 162, XP055953666, ISSN: 1097-2765, DOI: 10.1016/S1097-2765(02)00568-3
- SANDRINE BENHAMRON ET AL: "Direct activation of mTOR in B lymphocytes confers impairment in B-cell maturation and loss of marginal zone B cells", EUROPEAN JOURNAL OF IMMUNOLOGY, WILEY-VCH, HOBOKEN, USA, vol. 41, no. 8, 24 June 2011 (2011-06-24), pages 2390 - 2396, XP071225722, ISSN: 0014-2980, DOI: 10.1002/EJI.201041336
- DANIEL H FOWLER: "Rapamycin-resistant effector T-cell therapy", IMMUNOLOGICAL REVIEWS, WILEY-BLACKWELL PUBLISHING, INC, US, vol. 257, no. 1, 13 December 2013 (2013-12-13), pages 210 - 225, XP071455754, ISSN: 0105-2896, DOI: 10.1111/IMR.12127
- RANEK MARK J. ET AL: "PKG1-modified TSC2 regulates mTORC1 activity to counter adverse cardiac stress", NATURE, vol. 566, no. 7743, 30 January 2019 (2019-01-30), London, pages 264 - 269, XP055953497, ISSN: 0028-0836, Retrieved from the Internet <URL:https://www.nature.com/articles/s41586-019-0895-y.pdf> DOI: 10.1038/s41586-019-0895-y
- BRITTANY L. JACOBS ET AL.: "Identification of mechanically regulated phos- phorylation sites on tuberin (TSC2) that control mechanistic target of rapamycin (mTOR) signaling", J. BIOL. CHEM., vol. 292, no. 17, 2017, pages 6987 - 6997, XP055564925
- BRIAN YORK ET AL.: "Tuberin Nuclear Localization Can Be Regulated by Phos- phorylation of Its Carboxyl Terminus", MOL. CANCER RES., vol. 4, no. 11, 2006, pages 885 - 897, XP055724530

## Description

### BACKGROUND

### 1. Technical Field

This document relates to engineered tuberous sclerosis complex 2 (TSC2) polypeptides in which the ability of a residue (*e.g*., a residue corresponding to a serine residue in a wild type TSC2 polypeptide) to be phosphorylated is altered. In some cases, an engineered TSC2 polypeptide cannot be phosphorylated (*e.g*., by substituting a serine residue with an alanine residue). In some cases, an engineered TSC2 polypeptide can act as if it is constitutively phosphorylated (*e.g*., by substituting a serine residue with a glutamic acid residue).

### 2. Background Information

The mechanistic target of rapamycin complex 1 (mTORC1) coordinates biosynthetic and recycling pathways to control cell growth and metabolic homeostasis (Dibble et al., 2013 Nat Cell Biol 15:555; and Wolfson et al., 2016 Science 351:43). mTORC1 stimulates anabolic growth and suppresses protein recycling by autophagy. In addition to its role in normal physiology, mTORC1 contributes to disease such as autoimmune disorders, cancer, and heart failure (Sciarretta et al., 2014 Circ Res 114:549), where its hyperactivation is a therapeutic target (Laplante et al., 2012 Cell 149:274).

Broad suppression, however, risks compromising the normal role of mTORC1, whereas disease-dependent modulation could provide a more nuanced and targeted approach. Many intrinsic regulators of mTORC1 do so by phosphorylating Tuberous Sclerosis Complex 2 ("TSC2," also referred to as "tuberin"), a GTPase activating protein that modifies Rheb-GTP binding to stimulate or suppress mTORC1 (Inoki et al., 2003 Genes Dev 17:1829).

TSC2 is constitutively inhibitory, as gene deletion and loss-of-function mutations induce mTORC1 hyperactivity, causing tumors and neurological disease.
Growth/metabolic stimulation of extra-cellular response kinase (ERK1/2), protein kinase B (Akt), and p90Rsk reduce TSC2 inhibition (Menon et al., 2014 Cell 156:771), whereas energy depletion-stimulated AMP-activated protein kinase (AMPK) or glycogen synthase kinase-3β (GSK-3β) enhances TSC2 inhibition of mTORC1 (Inoki et al., 2006 Cell 126:955). Each kinase targets 2-5 different residues, and in order to block a particular enzyme effect, all the related sites must be silenced (Inoki et al., 2006 Cell 126:955; and Zhang et al., 2009 PLoS One 4:e6189). Perhaps as a consequence, no models altering such regulation *in vivo* have been reported.

In immune cells, upon the activation of the T-Cell Receptor (TCR), or corresponding surface receptor that triggers the immune cell to perform a designated task, the mTORC1 signaling pathway is engaged and ultimately determines the outcome of antigen recognition and cellular signaling response to the immune microenvironment. Through genetic gain or loss of function studies of components of the mTORC1 protein complex and its regulating proteins, a prominent role for mTORC1 in T-cell and other immune and inflammatory cell activity, differentiation, and function has been identified.

In T-cells, stimulation of mTORC1 results in enhancement of their effector function. This can involve clonal expansion so that the population size of a specific antigen-receptive cell is amplified to combat a foreign (or perceived to be foreign as in the case of autoimmune disease) body. It can also involve the enhanced synthesis and release of cytokines that coordinate an immune response. Stimulation of mTORC1 also enhances cytotoxic responses controlled by T cells to target foreign cells (*e.g.* tumor cells, virus, bacteria, other foreign bodies) with the goal of eliminating these cells from the host.

In T-cells, sustained mTORC1 stimulation results in a suppression of immunological effector (cytotoxic) function, termed anergy or exhaustion. It also compromises cell memory (or persistence) of the T-cell to a prior immunological antigenrecognition event and response.

In T-cells, the suppression of mTORC1 activity allows cells to remain in a more undifferentiated state, where they can replicate while still maintaining full differentiation potential. Reducing activity also enhances memory/persistence in effector T-cells and reduces the development of immunological anergy and exhaustion.

mTORC1 stimulation and inhibition play roles in cells that regulate immunological self-recognition, *e.g*. control over the immune system to recognize self from foreign cells and cell products. This is principally controlled by regulatory T-cells (Treg) that are central for suppressing immune reactions to self-antigens.

mTORC1 stimulation and inhibition also play roles in the modulation of inflammatory cells, such as neutrophils and macrophages. These cells are commonly engaged in autoimmune disease where identification of a self-antigen has resulted in the activation of an immunological response, and local release of cytokines and other factors stimulates inflammatory cells to attack the body and cause disease. The regulation of neutrophil and macrophage function to the corresponding inflammatory response also depends on their ability to control cell growth, metabolism, and protein homeostasis, and mTORC1 plays a central role to these factors and thus the functionality of these inflammatory cells.

US2016/251410A1 describes methods of expressing a recombinant polypeptide and providing cells having been contacted with an agent which downregulates an expression of a tuberous sclerosis, Brittany L Jacobs et al., 2017, J Biol. Chem, 292(17): 6987-6997 is directed to the identification of mechanically regulated phosphorylation sites on tuberin (TSC2) that control mechanistic target of rapamycin (mTOR) signaling.

### SUMMARY

In an aspect of the present invention, is provided a polypeptide comprising any one of SEQ ID NO: 1; SEQ ID NO:20; SEQ ID NO:2 and SEQ ID NO:21. In another aspect of the present invention is provided a nucleic acid encoding a polypeptide comprising SEQ ID NO: 1; SEQ ID NO:20; SEQ ID NO:2 and SEQ ID NO:21..

In another aspect of the present invention is provided a cell comprising a vector comprising a nucleic acid encoding a polypeptide comprising SEQ ID NO: 1 ; SEQ ID NO:20; SEQ ID NO:2 and SEQ ID NO:21, wherein the nucleic acid encoding the polypeptides is operably linked to a nucleic acid that drives expression of the polypeptide in the cell. The cell can be an immune cell. The immune cell can be a cytotoxic T cell or a chimeric antigen receptor T cell (CAR-T cell). Upon activation, the cytotoxic T cell or CAR-T cell can exhibit a higher level of mTORC1 signaling than a reference cytotoxic T cell or a reference CAR-T cell that lacks the vector. Upon activation, the cytotoxic T cell or CAR-T cell can express one or more cytokines at a higher level than a reference cytotoxic T cell or a reference CAR-T cell that lacks the vector, where the one or more cytokines can be interferon gamma, tumor necrosis factor alpha, interleukin 2, or combinations thereof. The immune cell can be a helper T cell. Upon activation, the helper T cell can exhibit a higher level of mTORC1 signaling than a reference helper T cell that lacks the vector. The immune cell can be a regulatory T cell. Upon activation, the regulatory T cell can exhibit a higher level of mTORC1 signaling than a reference regulatory T cell that lacks the vector. The cell also can include a genetic alteration in which a wild type nucleic acid sequence encoding TSC2 has been rendered inactive.

In some embodiments, wherein the nucleic acid sequence encodes a polypeptide comprising SEQ ID NO: 1 orSEQ ID NO:2, the nucleic acid comprises the sequence of SEQ ID NO:3 or SEQ ID NO:4.The nucleic acid encoding the polypeptide comprising SEQ ID NO: 2 is operably linked to a nucleic acid that drives expression of the polypeptide in the cell. In some embodiments the cell can be an immune cell. The immune cell can be a memory T cell. The memory T cell can exhibit a lower level of mTORC1 signaling than a reference memory T cell that lacks the vector. Upon activation, the memory T cell can express one or more cytokines at a lower level than a reference memory T cell that lacks the vector, where the one or more cytokines can be interferon gamma, tumor necrosis factor alpha, interleukin 2, or combinations thereof. The cell also can include a genetic alteration in which a wild type nucleic acid sequence encoding TSC2 has been rendered inactive.

In another aspect of the present invention is provided an engineered immune cell comprising a vector for use in the treatment of a disease in a subject, wherein the vector comprises a nucleic acid encoding a polypeptide comprising SEQ ID NO: 1 or SEQ ID NO: 20 operably linked to a promoter that drives expression of the polypeptide in the T cell, and wherein upon recognizing an antigen associated with the disease, the engineered immune cell exhibits increased activity as compared to a reference immune cell that lacks the vector; wherein the increased activity comprises increased mTORC1 signalling. The engineered immune cell for use can be a cytotoxic T cell. The increased activity of the cytotoxic T cell can include increased mTORC1 signaling. The increased activity of the cytotoxic T cell can include increased expression of one or more cytokines selected from the group consisting of: interferon gamma, tumor necrosis factor alpha, interleukin 2, and combinations thereof. The engineered immune cell can be a helper T cell. The increased activity of the helper T cell can include increased mTORC1 signaling. The disease can be cancer, a viral disease, a bacterial disease, fungal disease, or a parasitic disease. The engineered immune cell can be a regulatory T cell. The increased activity of the regulatory T cell can include increased mTORC1 signaling. The disease can be asthma, an autoimmune disease, or graft vs. host disease. The engineered immune cell can include a genetic alteration in a wild type nucleic acid sequence encoding TSC2, where the genetic alteration renders the TSC2 inactive. The engineered immune cell can be derived from an endogenous immune cell obtained from the subject.

In another aspect of the present invention is provided, an engineered immune cell comprising a vector for use in generating a persistent T cell in a subject, wherein the the vector comprises a nucleic acid encoding a polypeptide comprising SEQ ID NO: 2 or SEQ ID NO: 21 operably linked to a nucleic acid promoter that drives expression of the polypeptide in the engineered immune cell, wherein the engineered immune cell recognizes an antigen, where, upon recognizing the antigen, the engineered immune cell exhibits decreased activity as compared to a reference immune cell that lacks the vector, wherein the decreased activity of the engineered immune cell comprises decreased mTORC1 signaling, and where, upon recognizing the antigen, the engineered immune cell becomes the persistent T cell.. The engineered immune cell can be derived from an endogenous immune cell obtained from the subject. The engineered immune cell can be a CD8+ T cell, and the persistent T cell can be a memory T cell. The CD8+ T cell can be further engineered to express a chimeric antigen receptor or a T cell receptor. The engineered immune cell can be a regulatory T cell, and the persistent T cell can be a persistent T regulatory cell.

In another aspect of the present invention is provided, a method of generating a persistent T cell *in vitro.* The method comprises introducing into an immune cell a nucleic acid encoding a polypeptide comprising SEQ ID NO: 2 or SEQ ID NO: 21 operably linked to promoter that drives expression of the polypeptide in the immune cell, thereby generating an engineered immune cell, where the engineered immune cell exhibits decreased mTORC1 signaling as compared to a reference immune cell that lacks the vector, contacting the engineered immune cell with an antigen that is recognized by the engineered immune cell, and culturing the engineered immune cell under conditions and for a time sufficient such that the engineered immune cell becomes the persistent T cell. The immune cell can be a CD8+ T cell, and the persistent T cell can be a memory T cell. The CD8+ T cell can be further engineered to express a chimeric antigen receptor or a T cell receptor. The memory T cell produced by the method of generating a persistent T-cell *in* vitro can be administered to a subject. The subject can exhibit a disease, and the administration of the memory T cell to the subject can treat the disease. The disease can be cancer, a viral disease, a bacterial disease, fungal disease, or a parasitic disease. The immune cell can be obtained from the subject. The immune cell can be a regulatory T cell, and the persistent T cell can be a persistent T regulatory cell. The persistent T regulatory cell produced by the method can be administered to a subject. The subject can exhibit a disease, and the administration of the persistent T regulatory cell to the subject can treat the disease. The disease can be asthma, an autoimmune disease, or graft vs. host disease. The immune cell can be obtained from the subject.

In some embodiments, wherein the
nucleic acid encoding a polypeptide comprises SEQ ID NO: 20, the nucleic acid can include the sequence of SEQ ID NO: 22. In another embodiment, the vector includes a nucleic acid encoding a polypeptide including SEQ ID NO: 20 (e.g., SEQ ID NO: 22).

In some embodiments, wherein the nucleic acid encoding a polypeptide comprises SEQ ID NO: 21, the nucleic acid can include the sequence of SEQ ID NO: 23. In another embodiment, is provided a vector comprising a nucleic acid encoding a polypeptide including SEQ ID NO: 21 (e.g., SEQ ID NO: 23).

The details of one or more embodiments of the invention are set forth in the accompanying drawings and the description below. Other features, objects, and advantages of the invention will be apparent from the description and drawings, and from the claims.

### DESCRIPTION OF THE DRAWINGS

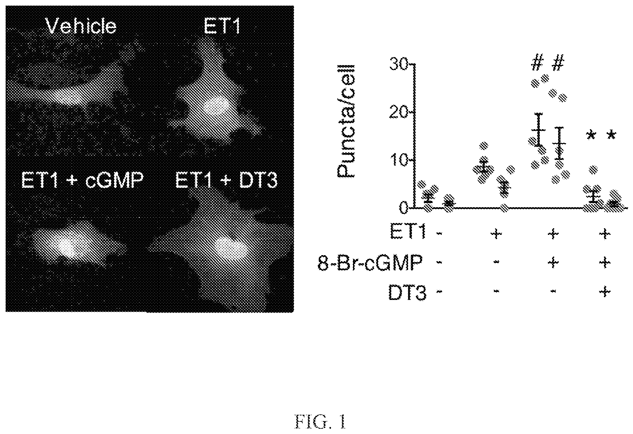

Figure 1 shows that protein kinase G (PKG) activates autophagic flux in isolated cardiac myocytes. Confocal images and summary data (n=6 biologically independent experiments; mean±SEM) for myocytes expressing an autophagic flux fluorescent reporter construct (LC3-GFP-RFP) with or without endothelin-1 (ET1) stimulation, and in turn treated with either a protein kinase G activator (cGMP) or protein kinase G inhibitor (DT3). ET1 stimulates myocyte growth (hypertrophy) and this is markedly reduced by protein kinase G stimulation. These cells display an increase in red dots indicating enhanced autophagy. By contrast, inhibiting PKG increases cell size and markedly reduces autophagy as reflected by the fall in both green and red dots. Summary results to the right, analysis by 1WANOVA, with Sidak multiple comparison test. For green and red dots: * p≤8E-5 vs corresponding Veh, red: †-p=0.014 vs Veh-SA and WT-ET1, p<1E-6 vs SE-ET1, # p<1E-6 vs WT-ET1.

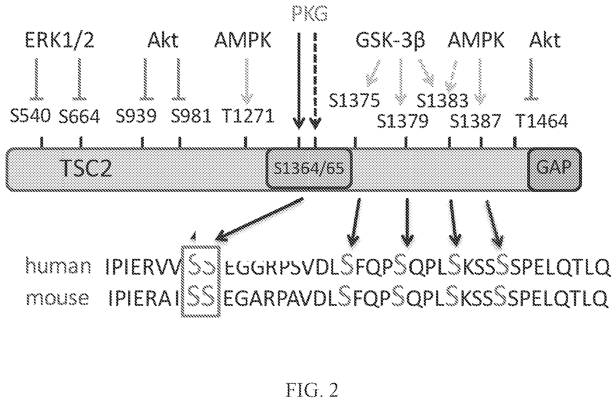

Figure 2 contains a phospho-site map for TSC2, with numbering based on the human polypeptide sequence. Sequences shown include residues 1357-1395 of the human TSC2 sequence set forth in SEQ ID NO: 5, and residues 1358 -1396 of the mouse TSC2 sequence set forth in SEQ ID NO: 6. These sites are all highly homologous between human and other mammalian species, though the numbering is slightly different for the T1271 AMP-kinase (AMPK) site (for mouse it is T1270), and for all sites identified at and above S1364 (mouse numbering is one higher for each; *e.g*., human S1364 (hsS1364) is equivalent to mouse S1365 (mmS1365)).

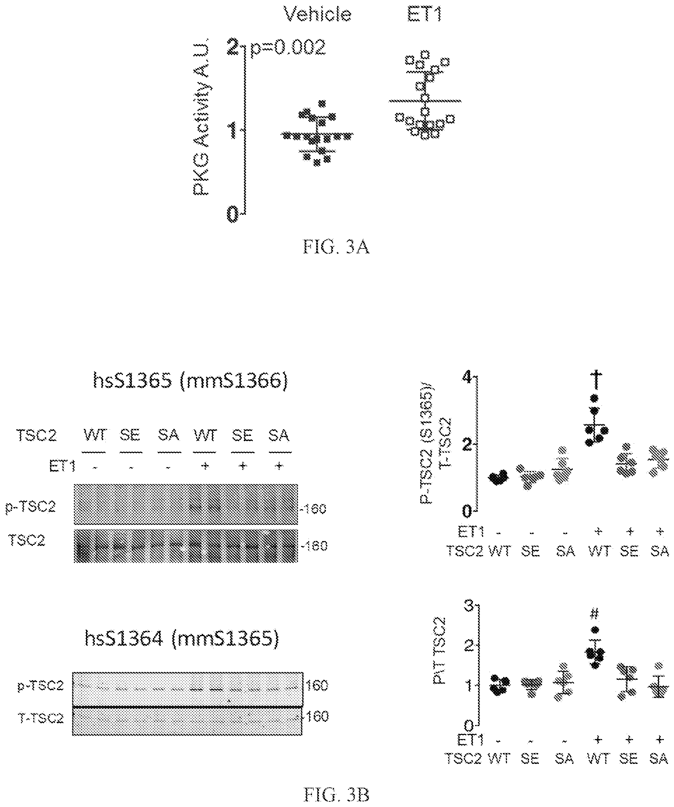

Figures 3A and 3B show that myocytes stimulated with a growth hormone - endothelin-1 exhibit increased protein kinase G activation, and that this results in the phosphorylation of TSC2 at either hsS1364 or hsS1365. The latter is equally blunted by site mutagenesis of the serine to either an alanine (SA) or glutamic acid (SE) at either hsS1364 or hsS1365. A) PKG activation was measured in myocytes overexpressing WT, SA, or SE forms of TSC2 and stimulated with endothelin-1 (ET1, 10 nM) or vehicle for 48 hours. Mean±SD, Unpaired t-test, 2-tailed; n=18 biologically independent samples. B) The same cell lysates were probed for phosphorylated TSC2 signal as detected by an antibody raised to mmS1365 (mouse; hsS1364 - human). Mutations of either hsS1365 or hsS1364 result in a loss of antibody-detected signal, indicating that either serine modification is sufficient to prevent phosphorylation of TSC2 protein at this location. Summary data are provided to the right for each set of serine mutations: n=6 biologically independent samples. Summary data below, Mean±SD. 1WANOVA, Tukey multiple comparisons test, † p=0.0002 vs SE, p=0.0007 vs SA, # p p=0.003 vs SE, p=0.0003 vs SA.

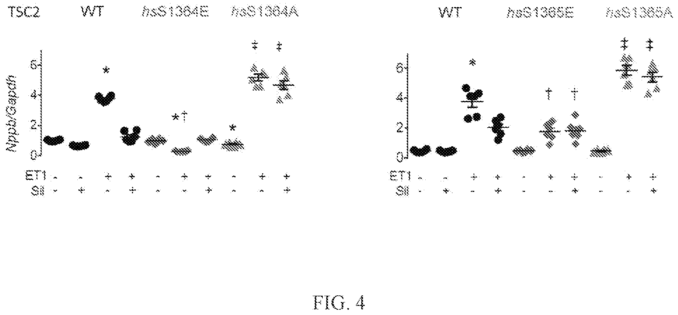

Figure 4 shows *Nppb* mRNA expression (pathological hypertrophy gene marker) in myocytes transfected with WT, SA, or SE TSC2 (huS1365 mutated), and then exposed to 48-hrs ET1 to induce hypertrophy. Activation of PKG by sildenafil (Sil) reduces Nppb increase in WT expressing cells, but not those with SA or SE. SE expression depresses *Nppb* rise, whereas SA expression enhances it. The results are shown for two sets of experiments. On the left, both the SA and SE mutations targeted hsS1364 (mmS1365). On the right, both the SA and SE mutations targeted hsS1365 (mmS1366). Mean±SEM, n=6 biologically independent experiments, 1-WANOVA with Tukey multiple comparisons test. *p<1E-5 vs other WT groups; † p=0.001 vs SE, p<1E-6 vs WT+ET1, ‡ p<1E-6 vs SA, WT-ET1, SE+ET1.

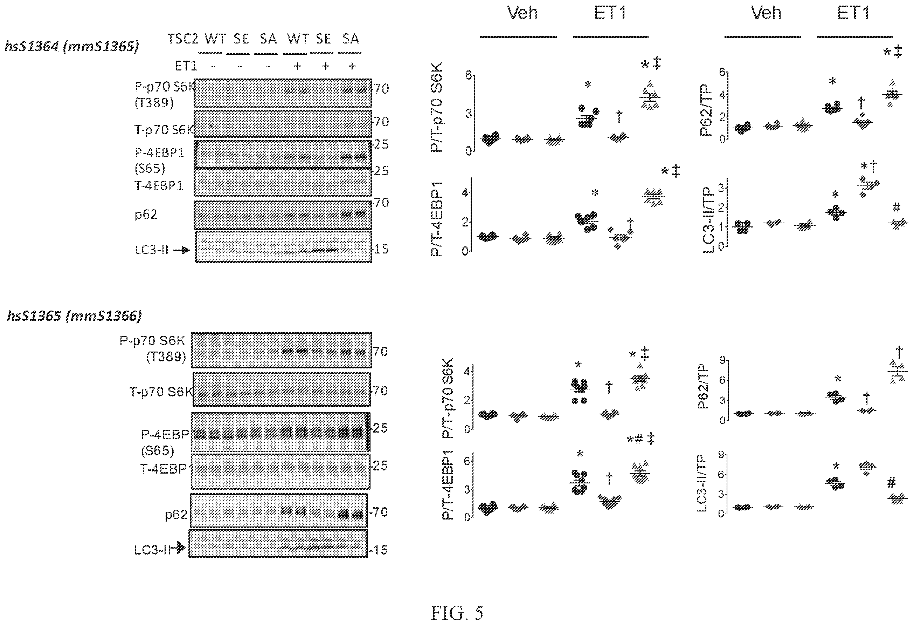

Figure 5 shows mTORC1 signaling proteins and for markers of autophagy (p62 and LC3-II) when hsS1364 (mmS1365) was mutated to either alanine (SA) or glutamate (SE) (upper set of data), and when hsS1365 (mmS1366) was mutated to either alanine (SA) or glutamate (SE) (lower set of data). Western blots are from the same experiment as shown in Figure 4. Expression of hsS1364E or hsS1365E reduces mTORC1 activation and p62, while further increasing LC3-II. By contrast, expression of hsS1364A or hsS1365A further increased mTORC1 activation (more p70S6K and 4EBP1 phosphorylation, increased p62, reduced LC3-II). Each experiment was replicated 2-4 times, providing n=4-8 biologically independent samples. Mean±SEM, 1WANOVA with Tukey multiple comparisons test. Symbols for upper panel set: *p≤ 7E-6 vs Vehicle control; † p<1E-6 vs SE-ET1; ‡p<5E-6, #p=0.01 vs WT-ET1. Symbols for lower panel set: *p≤1E-6 vs WT vehicle and SE-ET1, †p<1E-6 vs SA-ET1, §p=0.0001, ‡p=0.003, ¶ρ<1E-6 vs SA-ET1, #p=0.003 vs. SA-Veh.
Figure 6 shows that autophagic flux when hsS1365 (mmS1366) was mutated to either A (SA) or E (SE). Confocal image and summary data (n=6 biologically independent experiments; mean±SEM) for LC3-GFP-RFP reporter of autophagic flux in WT, SA, or SE-TSC2 expressing myocytes ± ET-1 stimulation. 1W ANOVA, Sidak multiple comparison test. For green and red dots: * p≤8E-5 vs corresponding Veh, red: †-p=0.014 vs Veh-SA and WT-ET1, p<1E-6 vs SE-ET1, # p<1E-6 vs WT-ET1.

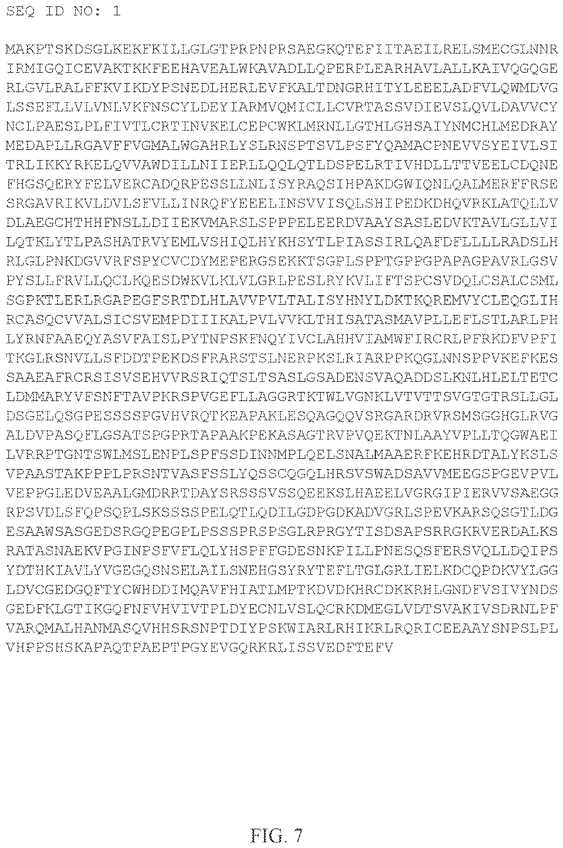

Figure 7 contains an amino acid sequence (SEQ ID NO: 1) of an exemplary engineered TSC2 polypeptide having an alanine substitution at the serine residue at position S1365 of the human TSC2 polypeptide sequence.

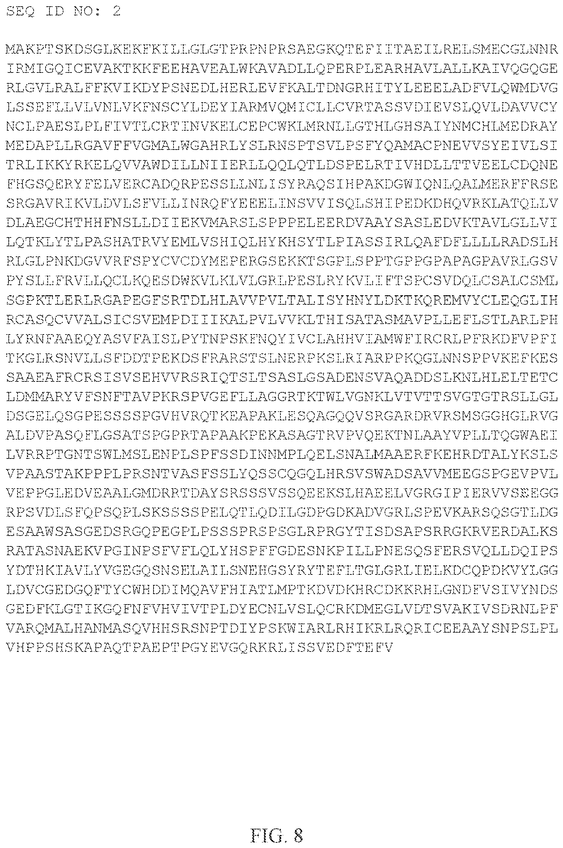

Figure 8 contains an amino acid sequence (SEQ ID NO: 2) of an exemplary engineered TSC2 polypeptide having a glutamic acid substitution at the serine residue at position S1365 of the human TSC2 polypeptide sequence.

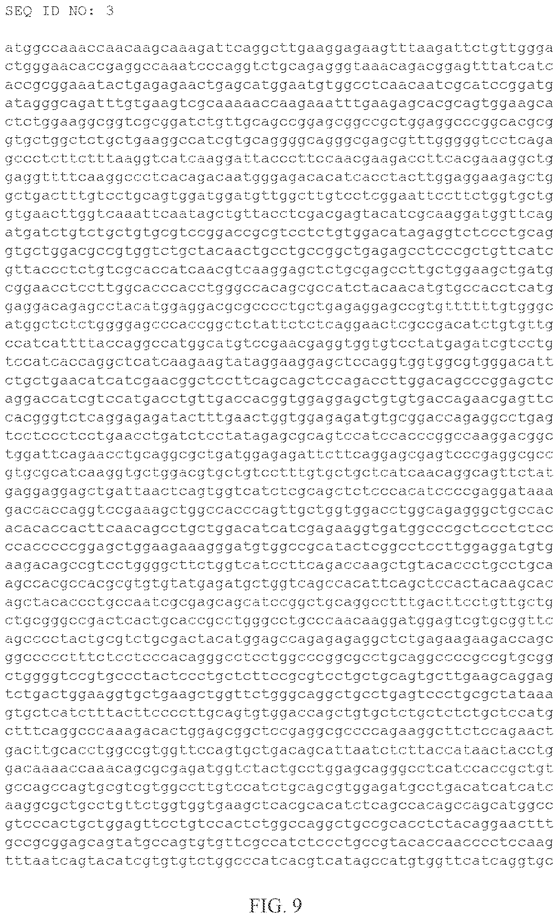

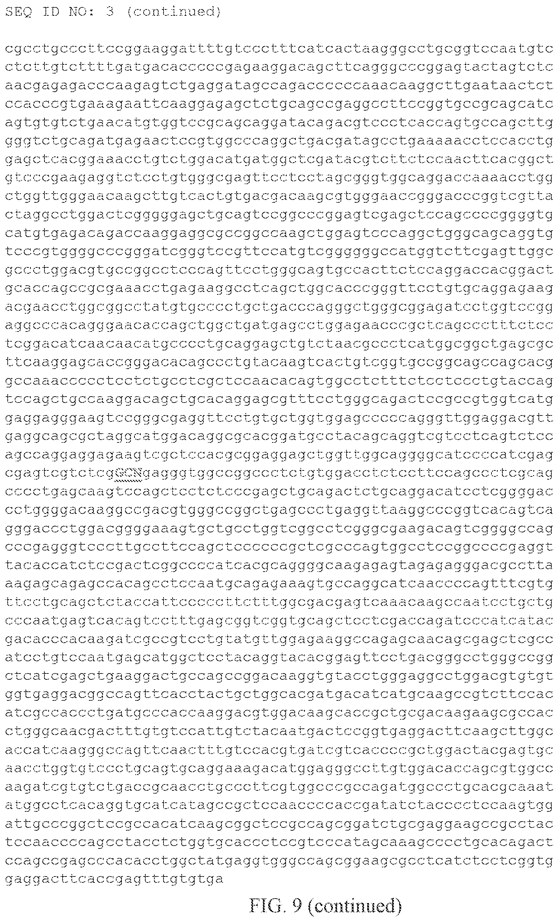

Figure 9 contains a nucleic acid sequence (SEQ ID NO: 3) encoding an engineered TSC2 polypeptide having an alanine substitution at the serine residue at position S1365 of the human TSC2 polypeptide sequence.

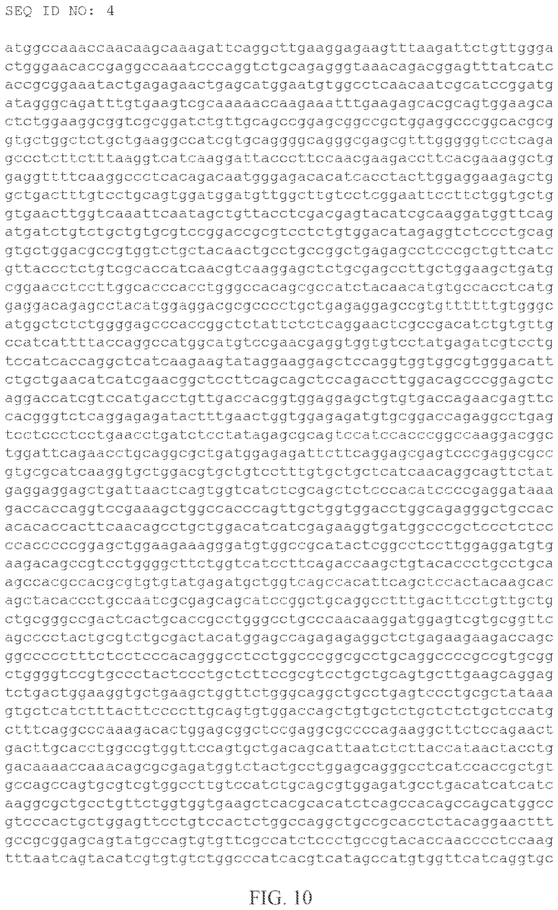

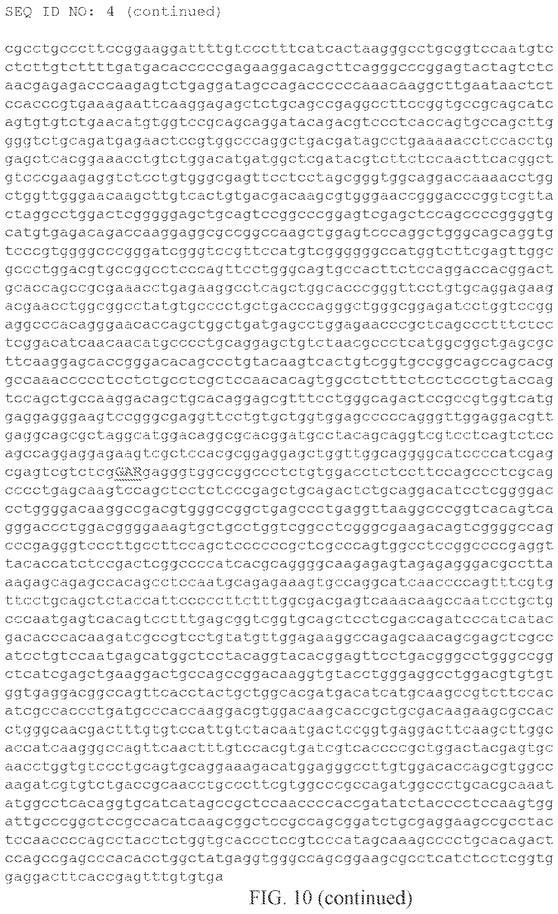

Figure 10 contains a nucleic acid sequence (SEQ ID NO: 4) encoding an engineered TSC2 polypeptide having a glutamic acid substitution at the serine residue at position S1365 of the human TSC2 polypeptide sequence.

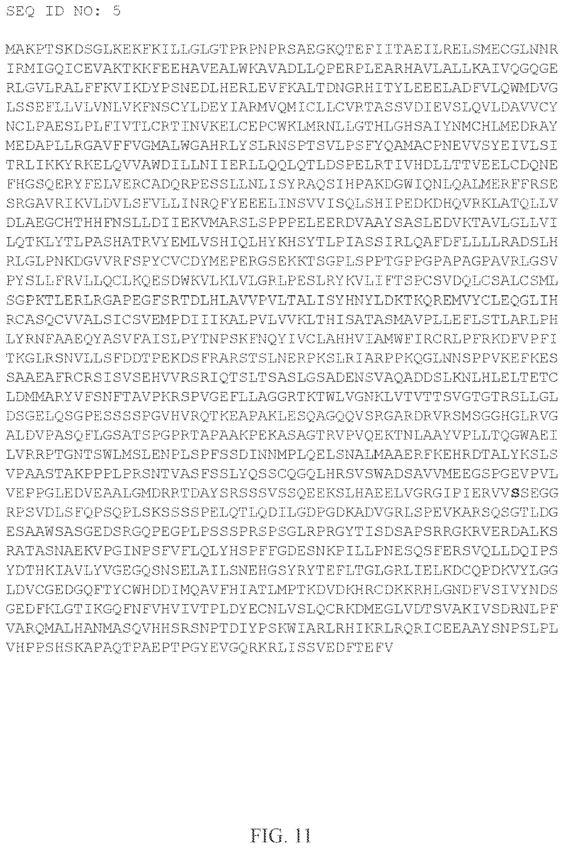

Figure 11 contains an amino acid sequence (SEQ ID NO: 5) of an wild-type human TSC2 polypeptide sequence.

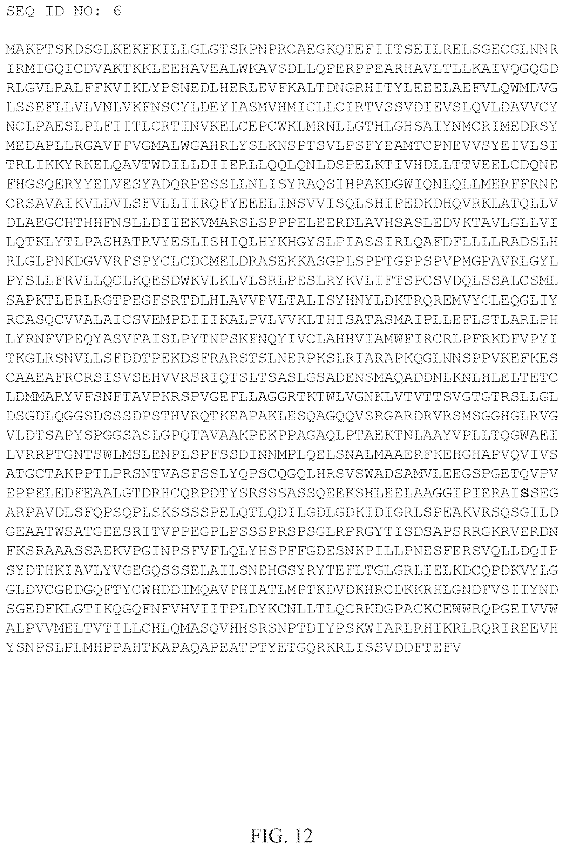

Figure 12 contains an amino acid sequence (SEQ ID NO: 6) of an wild-type mouse TSC2 polypeptide sequence.

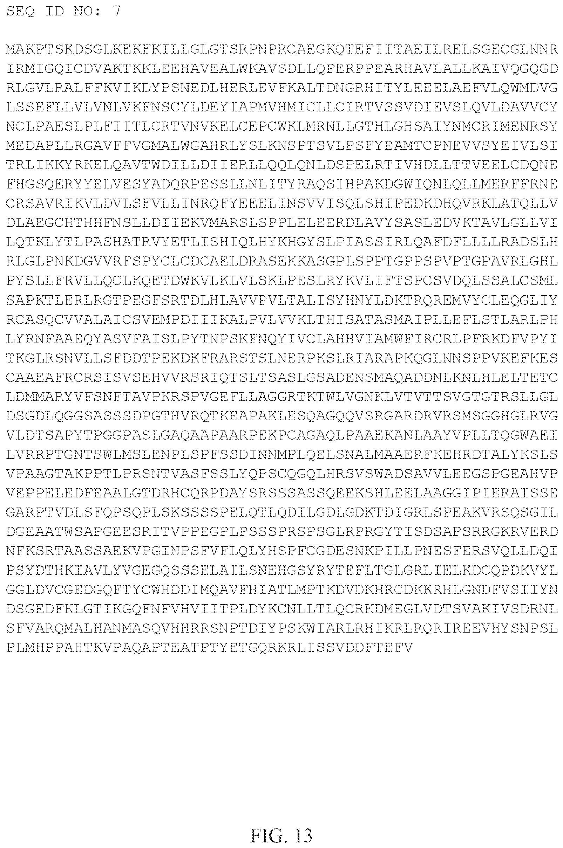

Figure 13 contains an amino acid sequence (SEQ ID NO: 7) of an wild-type rat TSC2 polypeptide sequence.

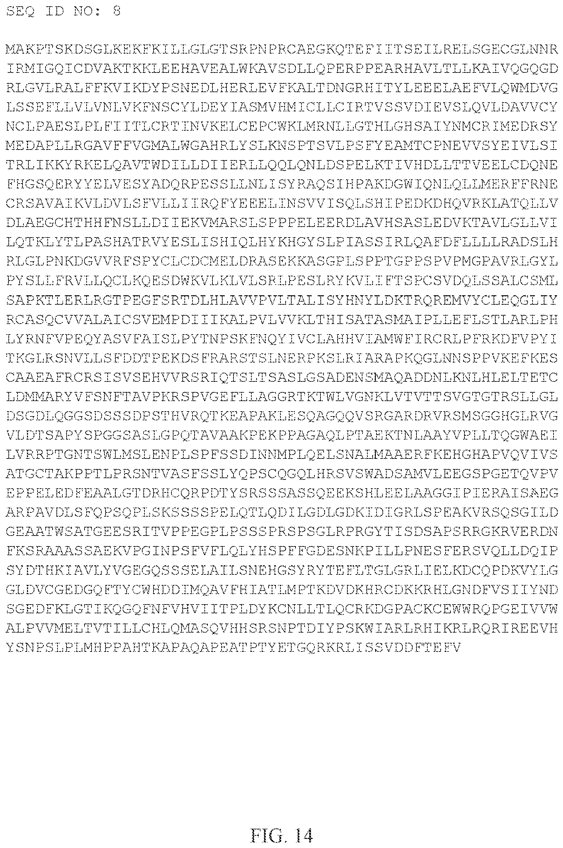

Figure 14 contains an amino acid sequence (SEQ ID NO: 8) of an engineered TSC2 polypeptide having an alanine substitution at the serine residue at position S1366 of the mouse TSC2 polypeptide sequence.

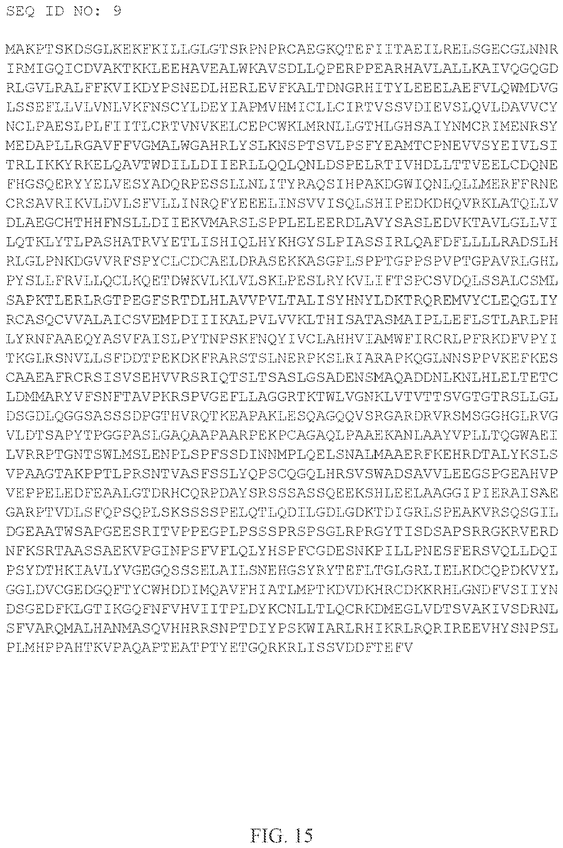

Figure 15 contains an amino acid sequence (SEQ ID NO: 9) of an e engineered TSC2 polypeptide having an alanine substitution at the serine residue at position S1367 of the rat TSC2 polypeptide sequence.

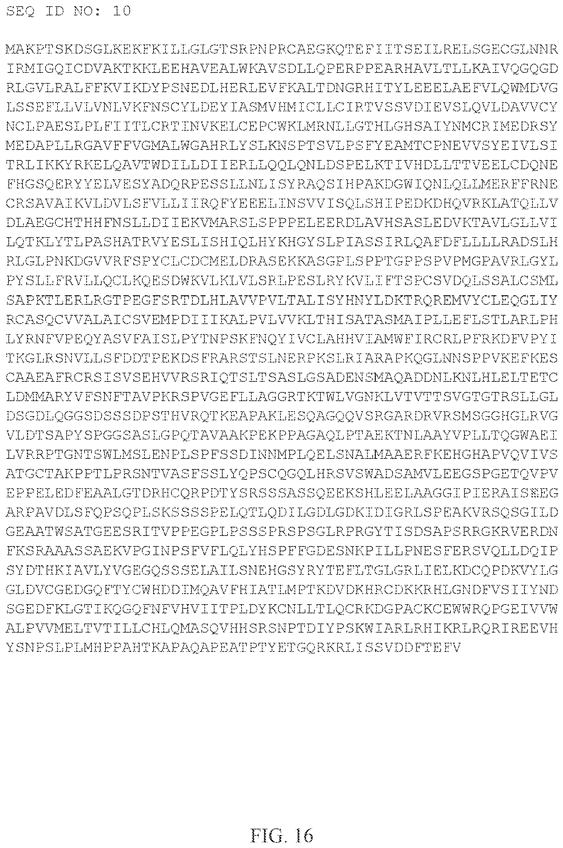

Figure 16 contains an amino acid sequence (SEQ ID NO: 10) of an engineered TSC2 polypeptide having a glutamic acid substitution at the serine residue at position S1366 of the mouse TSC2 polypeptide sequence.

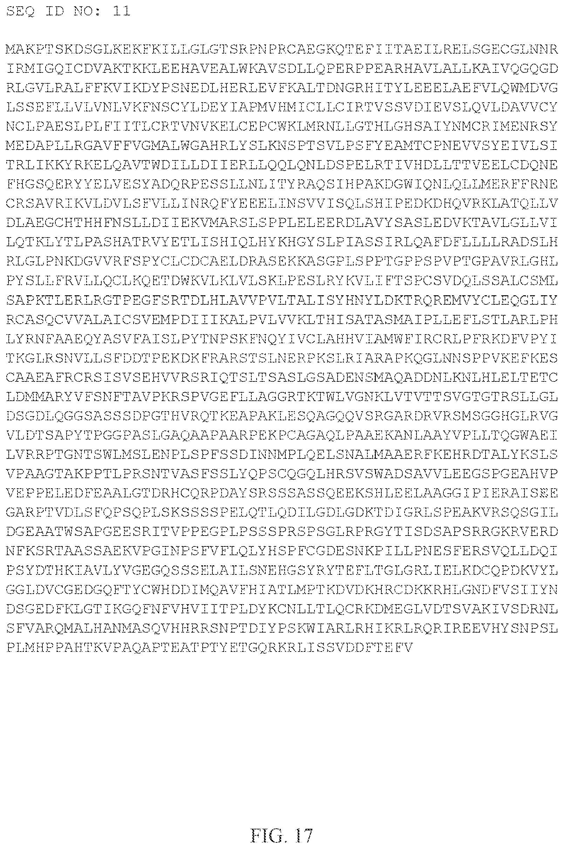

Figure 17 contains an amino acid sequence (SEQ ID NO: 11) of an engineered TSC2 polypeptide having a glutamic acid substitution at the serine residue at position S1367 of the rat TSC2 polypeptide sequence.

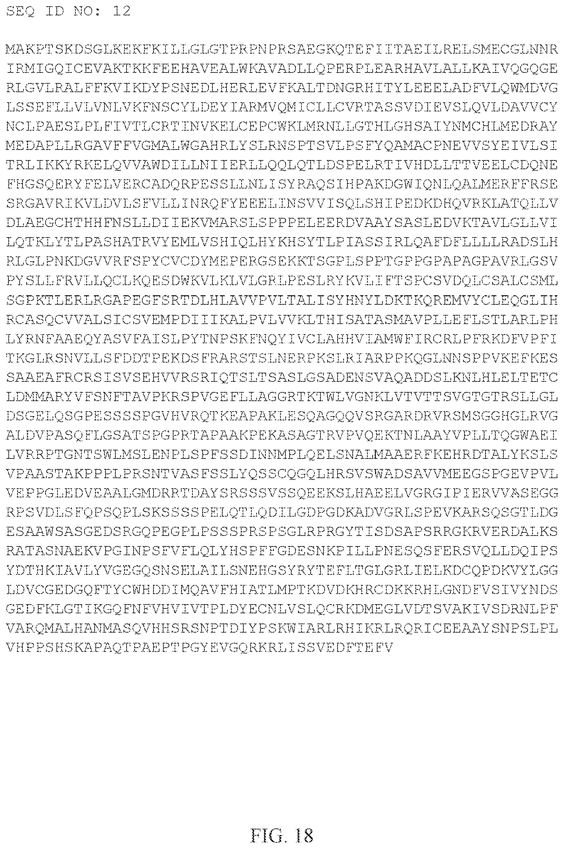

Figure 18 contains an amino acid sequence (SEQ ID NO: 12) of an engineered TSC2 polypeptide having an alanine substitution at the serine residue at position S1364 of the human TSC2 polypeptide sequence.

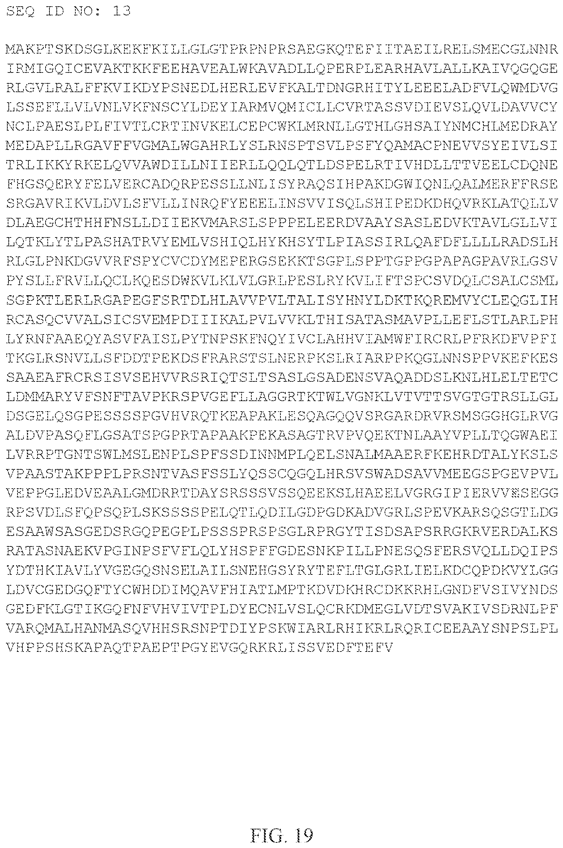

Figure 19 contains an amino acid sequence (SEQ ID NO: 13) of an engineered TSC2 polypeptide having a glutamic acid substitution at the serine residue at position S1364 of the human TSC2 polypeptide sequence.

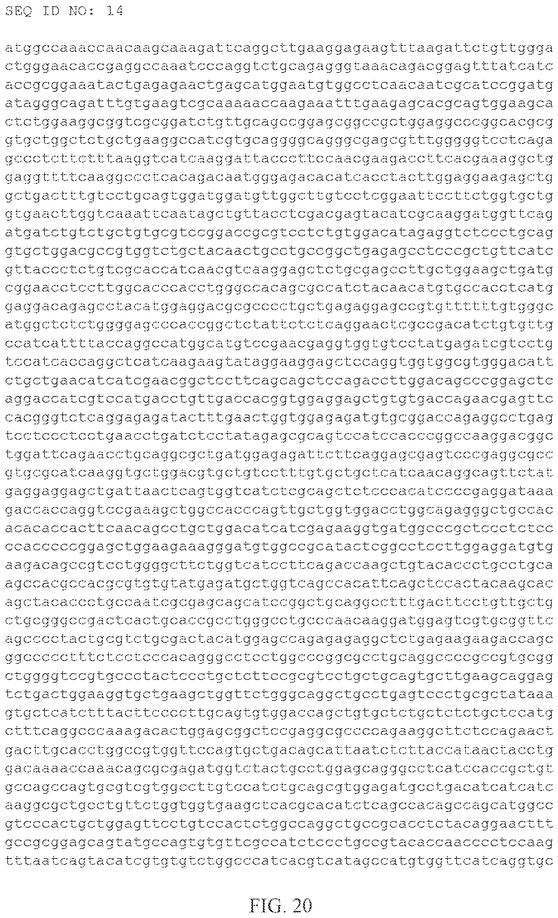

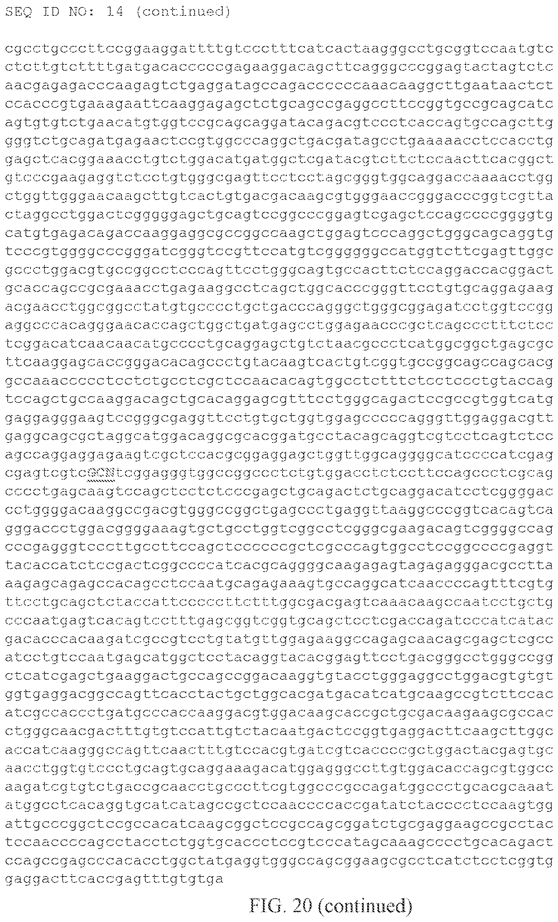

Figure 20 contains a nucleic acid sequence (SEQ ID NO: 14) encoding an amino acid sequence of an engineered TSC2 polypeptide having an alanine substitution at the serine residue at position S1364 of the human TSC2 polypeptide sequence.

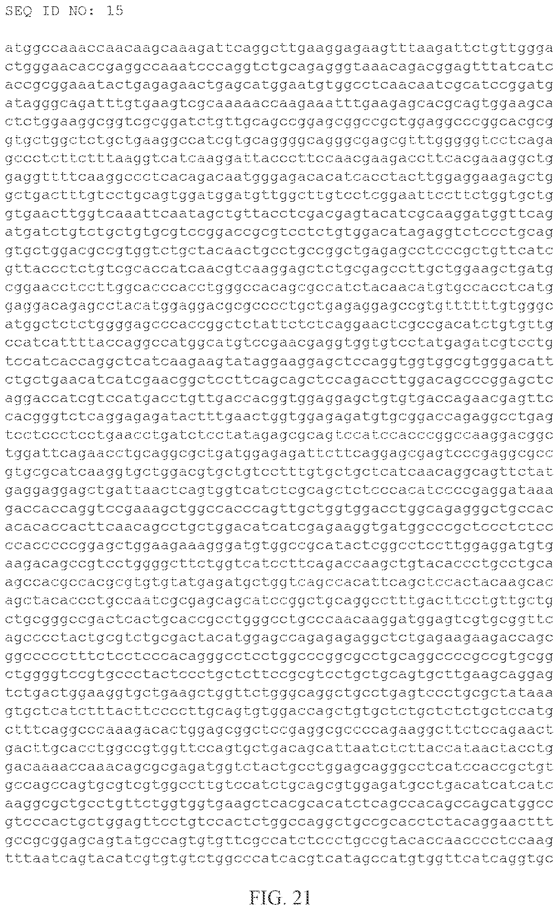

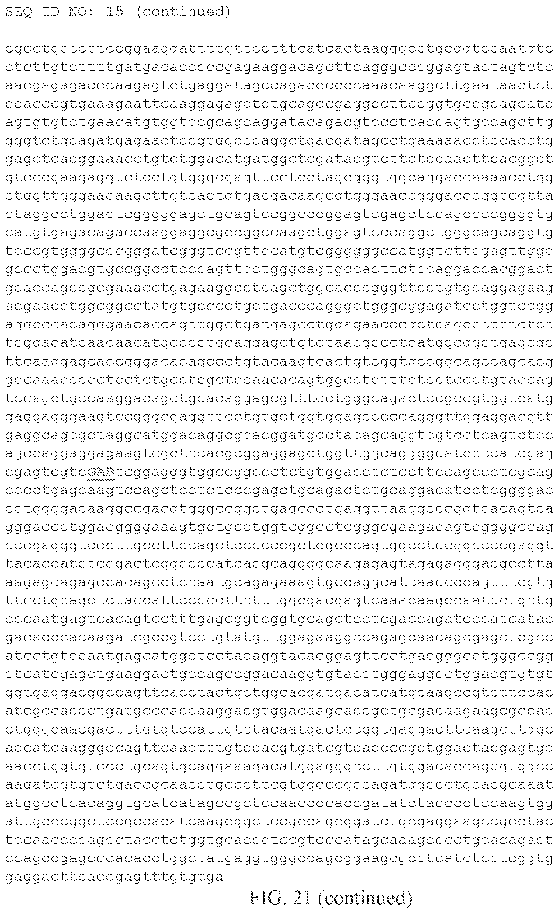

Figure 21 contains a nucleic acid sequence (SEQ ID NO: 15) encoding an amino acid sequence of an engineered TSC2 polypeptide having a glutamic acid substitution at the serine residue at position S1364 of the human TSC2 polypeptide sequence.

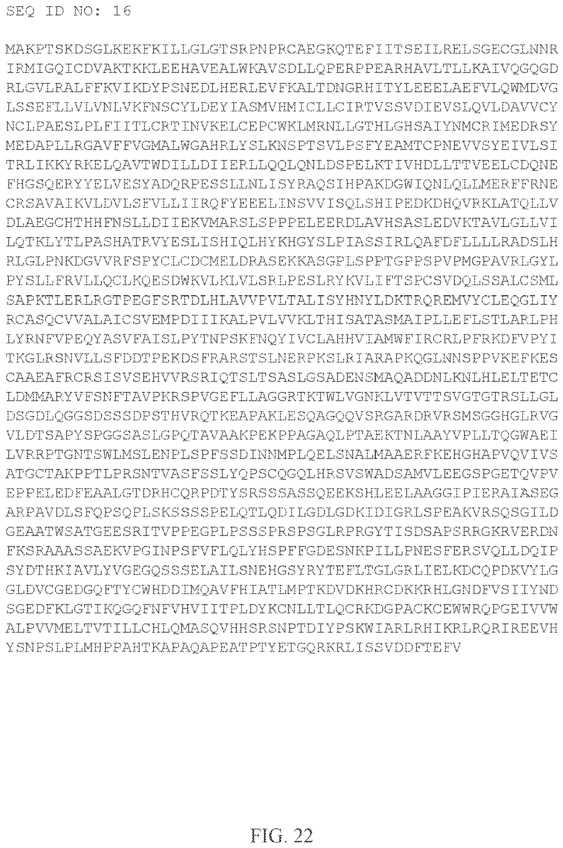

Figure 22 contains an amino acid sequence (SEQ ID NO: 16) of an engineered TSC2 polypeptide having an alanine substitution at the serine residue at position S1365 of the mouse TSC2 polypeptide sequence.

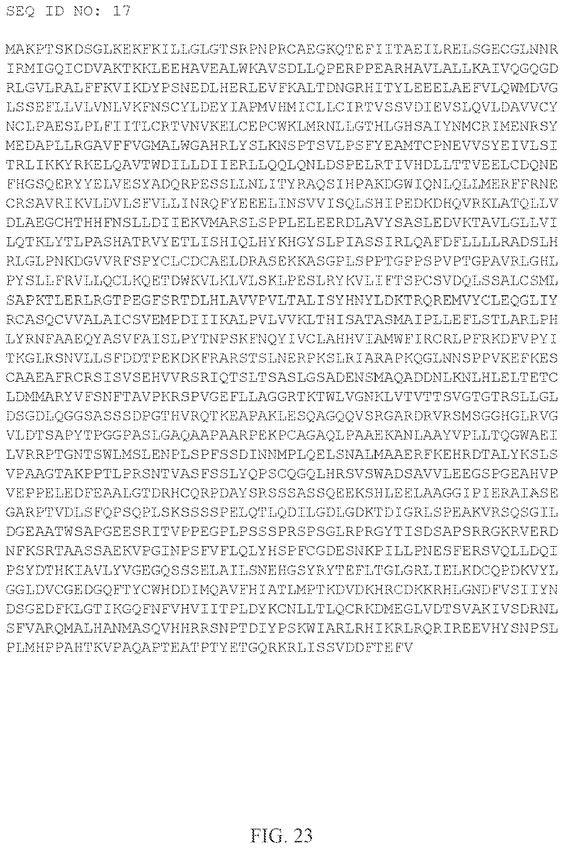

Figure 23 contains an amino acid sequence (SEQ ID NO: 17) of an engineered TSC2 polypeptide having an alanine substitution at the serine residue at position S1366 of the rat TSC2 polypeptide sequence.

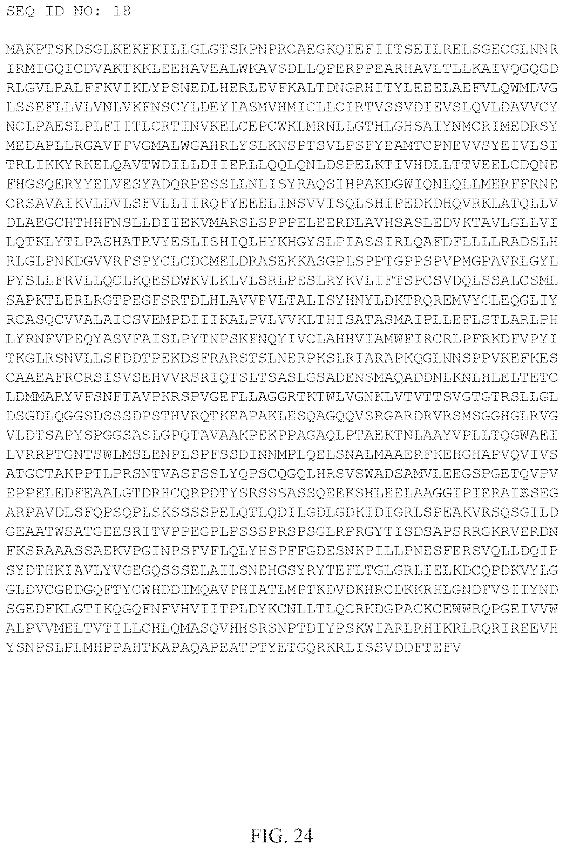

Figure 24 contains an amino acid sequence (SEQ ID NO: 18) of an engineered TSC2 polypeptide having a glutamic acid substitution at the serine residue at position S1365 of the mouse TSC2 polypeptide sequence.

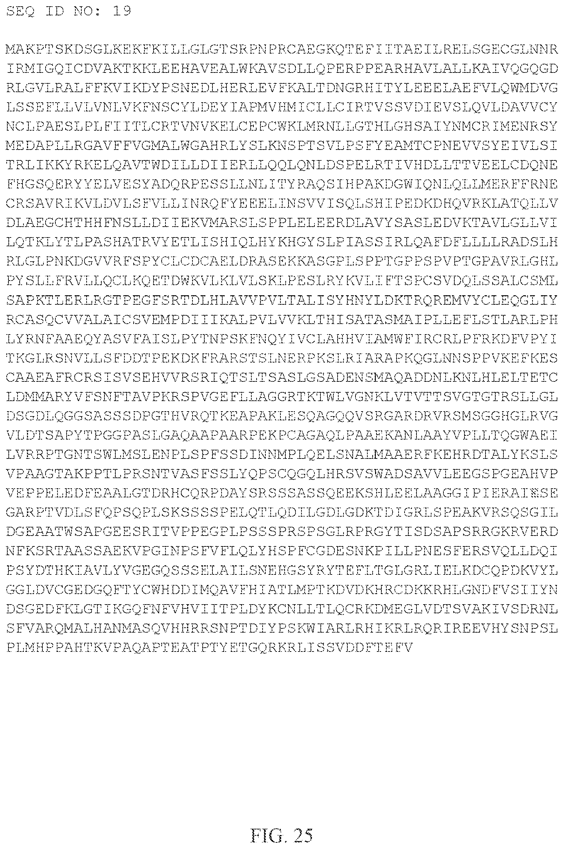

Figure 25 contains an amino acid sequence (SEQ ID NO: 19) of an engineered TSC2 polypeptide having a glutamic acid substitution at the serine residue at position S1366 of the rat TSC2 polypeptide sequence.

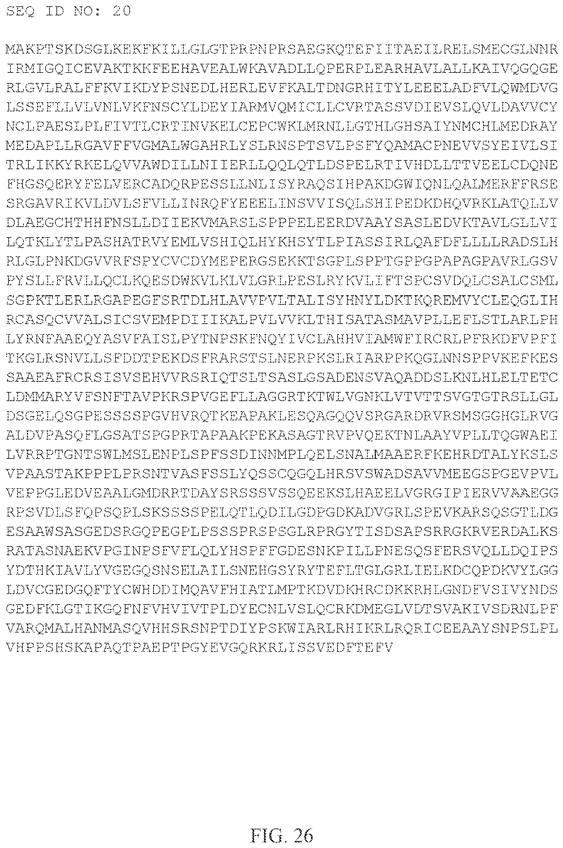

Figure 26 contains an amino acid sequence (SEQ ID NO: 20) of an exemplary engineered TSC2 polypeptide having an alanine substitution at the serine residue at position S1364 and having an alanine substitution at the serine residue at position S1365 of the human TSC2 polypeptide sequence.

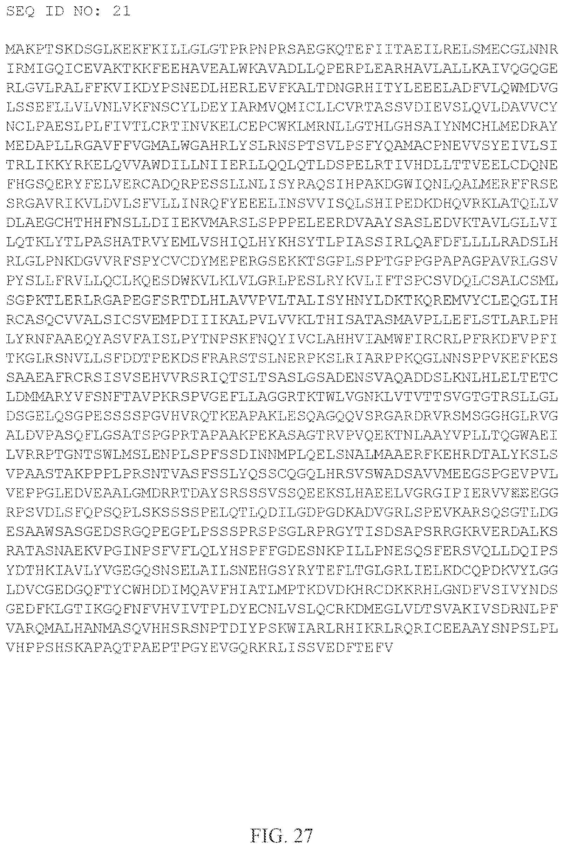

Figure 27 contains an amino acid sequence (SEQ ID NO: 21) of an exemplary engineered TSC2 polypeptide having a glutamic acid substitution at the serine residue at position S1364 and having a glutamic acid substitution at the serine residue at position S1365 of the human TSC2 polypeptide sequence.

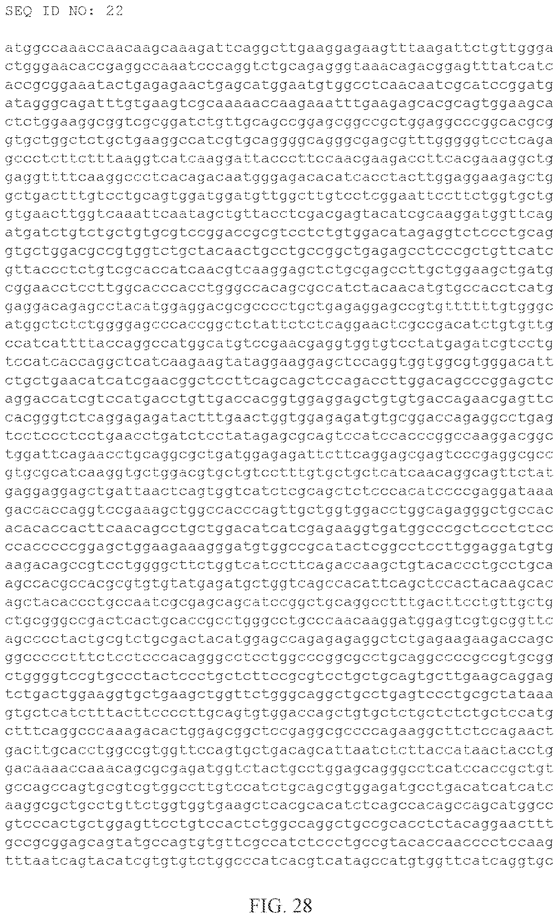

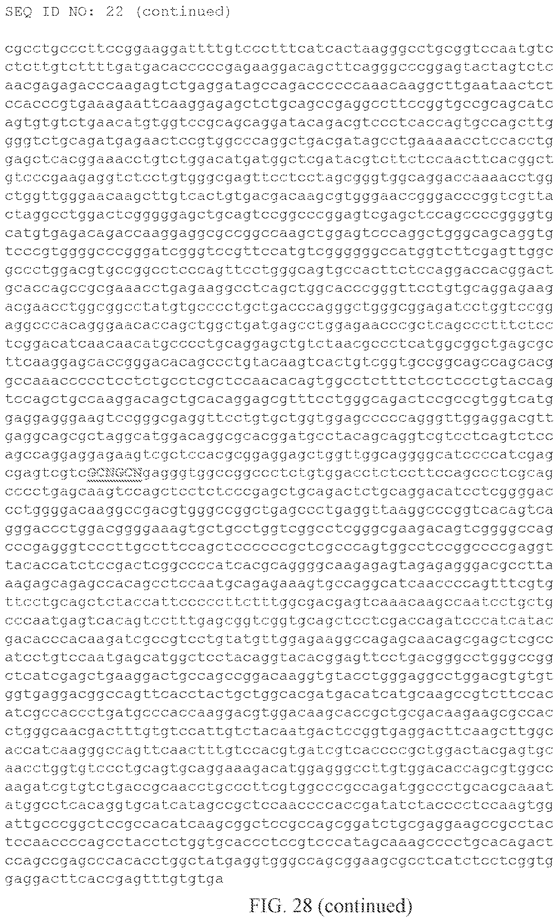

Figure 28 contains a nucleic acid sequence (SEQ ID NO: 22) encoding an amino acid sequence of an exemplary engineered TSC2 polypeptide having an alanine substitution at the serine residue at position S1364 and having an alanine substitution at the serine residue at position S1365 of the human TSC2 polypeptide sequence.

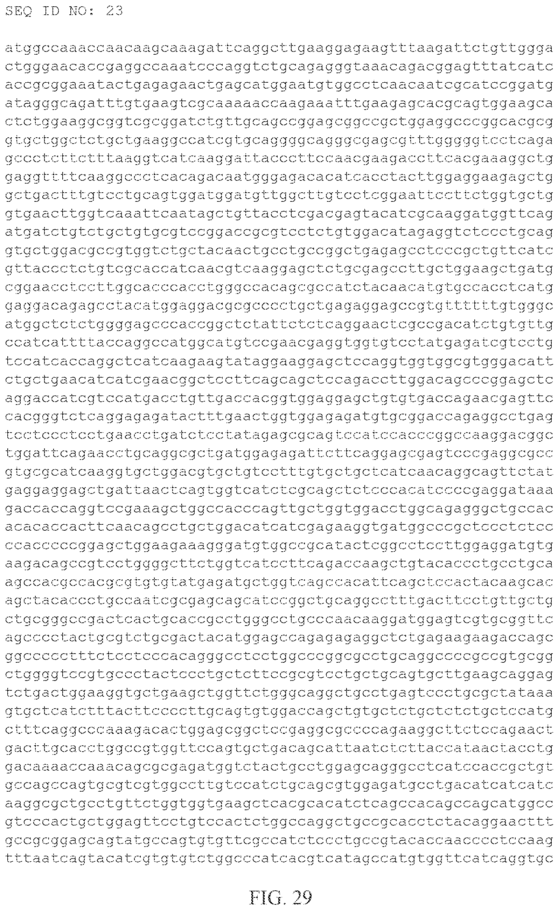

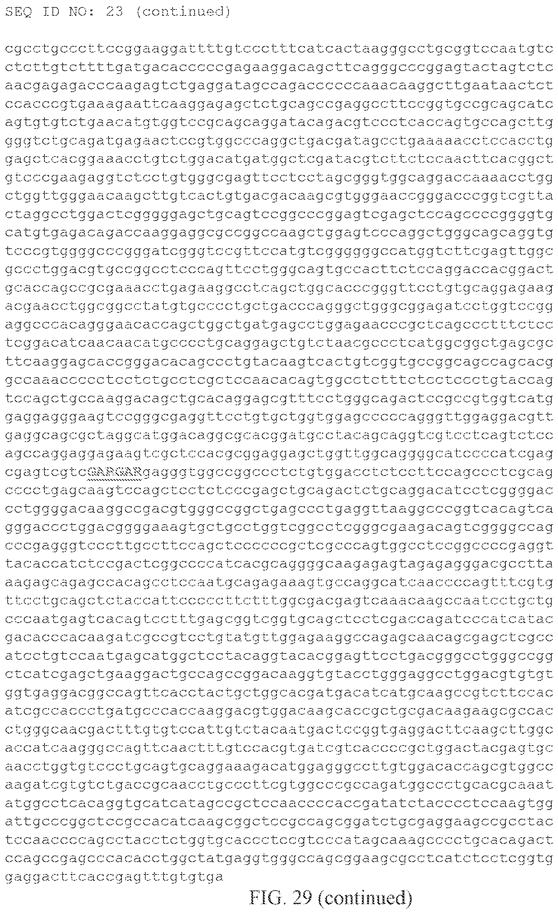

Figure 29 contains a nucleic acid sequence (SEQ ID NO: 23) encoding an amino acid sequence of an exemplary engineered TSC2 polypeptide having a glutamic acid substitution at the serine residue at position S1364 and having a glutamic acid substitution at the serine residue at position S1365 of the human TSC2 polypeptide sequence.

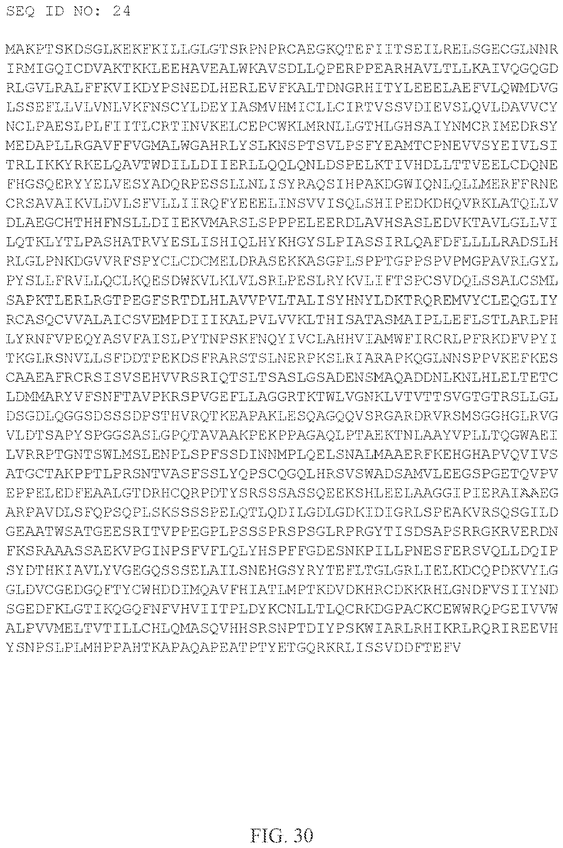

Figure 30 contains an amino acid sequence (SEQ ID NO: 24) of an engineered TSC2 polypeptide having an alanine substitution at the serine residue at position S1365 and having an alanine substitution at the serine residue at position S1366 of the mouse TSC2 polypeptide.

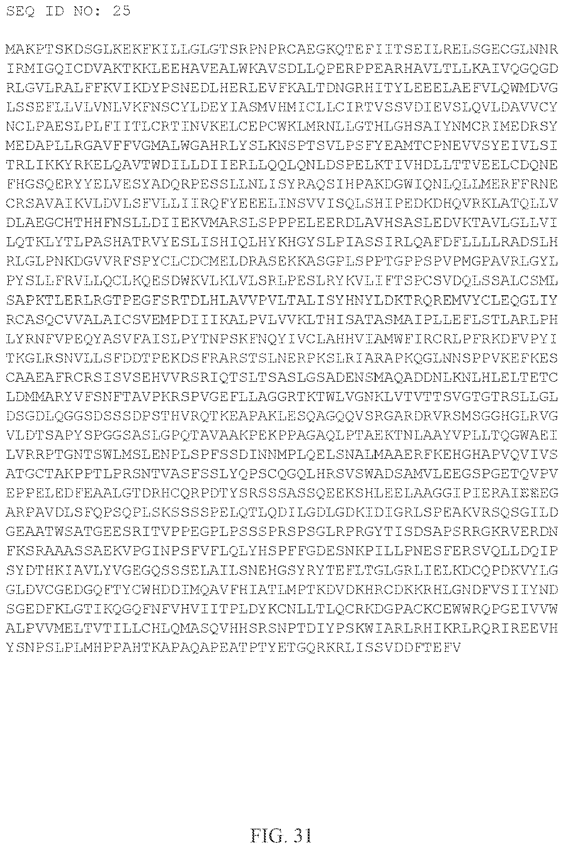

Figure 31 contains an amino acid sequence (SEQ ID NO: 25) of an engineered TSC2 polypeptide having an glutamic acid substitution at the serine residue at position S1365 and having an glutamic acid substitution at the serine residue at position S1366 of the mouse TSC2 polypeptide.

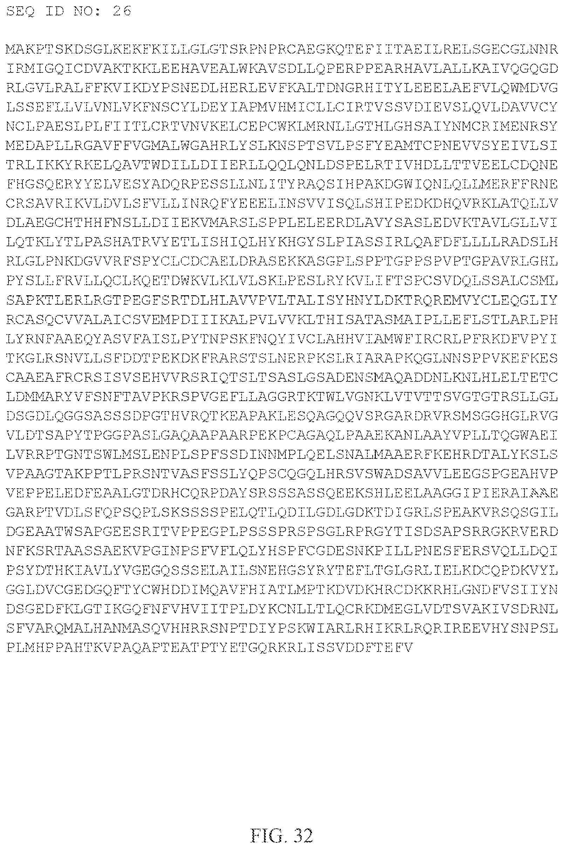

Figure 32 contains an amino acid sequence (SEQ ID NO: 26) of an engineered TSC2 polypeptide having an alanine substitution at the serine residue at position S1366 and having an alanine substitution at the serine residue at position S1367 of the rat TSC2 polypeptide.

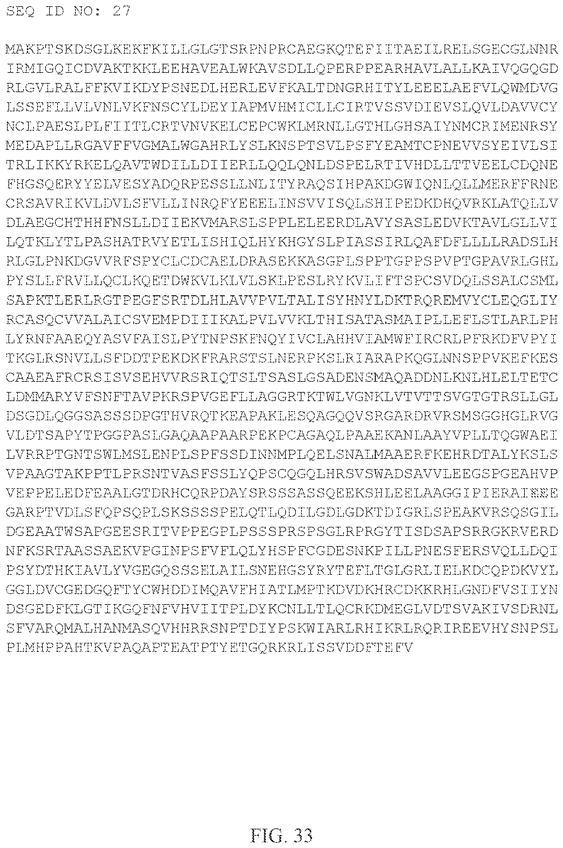

Figure 33 contains an amino acid sequence (SEQ ID NO: 27) of an engineered TSC2 polypeptide having an glutamic acid substitution at the serine residue at position S1366 and having an glutamic acid substitution at the serine residue at position S1367 of the rat TSC2 polypeptide.

### DETAILED DESCRIPTION

This document provides engineered TSC2 polypeptides in which the ability of a residue (*e.g*., a residue corresponding to a serine residue in a wild type TSC2 polypeptide) to be phosphorylated (*e.g*., as compared to a wild-type TSC2 polypeptide) is altered. For example, a TSC2 polypeptide can be engineered at a serine residue corresponding to S1365 of the human TSC2 polypeptide sequence (SEQ ID NO: 5), and, optionally, can also be engineered at a serine residue corresponding to S1364 of the human TSC2 polypeptide sequence (SEQ ID NO: 5).

In some cases, an engineered TSC2 polypeptide can exhibit a decreased ability to be phosphorylated. In some embodiments, an engineered TSC2 polypeptide having a substitution at residue S1365 (*e.g*., a substitution of the serine residue with an alanine residue (S1365A)), and, optionally, a substitution at residue S13645 (e.g., a substitution of the serine residue with an alanine residue (S1364A)), can exhibit a decreased ability to be phosphorylated (*e.g*., cannot be phosphorylated).The present invention provides, an engineered TSC2 polypeptide having a substitution at residue S1365 (S1365A or S1365E), and, optionally, also having a substitution at residue S1364 (S1365A or S1365E), which can exhibit a decreased ability to be phosphorylated (e.g., cannot be phosphorylated).

In some embodiments, an engineered TSC2 polypeptide can be pseudophosphorylated. An engineered TSC2 polypeptide having a substitution at residue S1365 (*e.g*., a substitution of the serine residue with a glutamic acid residue (S1365E)), and, optionally, a substitution at residue S1364 (e.g., a substitution of the serine residue with a glutamic acid residue (S1364E)) can be pseudo-phosphorylated (*e.g*., can act as if it is constitutively phosphorylated). For example, an engineered TSC2 polypeptide having a substitution at residue S1365E, and, optionally, also having a substitution at residue S1364E can be pseudo-phosphorylated (e.g., can act as if it is constitutively phosphorylated).

This document also provides *in vitro* methods for making and using the engineered TSC2 polypeptides described herein, as well as engineered immune cells including engineered TSC2 polypeptides described herein and/or nucleic acid sequences encoding engineered TSC2 polypeptides described herein and methods of making and using such engineered immune cells.

As used herein, the word "a" before a noun represents one or more of the particular noun. For example, the phrase "a genetic alteration" encompasses "one or more genetic alterations."

As used herein, the term "about" means approximately, in the region of, roughly, or around. When used in conjunction with a numerical range, the term "about" modifies that range by extending the boundaries above and below the numerical values set forth. In general, the term "about" is used herein to modify a numerical value above and below the stated value by a variance of 10%.

As used herein, the term "subject" means a vertebrate, including any member of the class mammalia, including humans, domestic and farm animals, and zoo, sports or pet animals, such as mouse, rabbit, pig, sheep, goat, cattle, horse *(e.g.,* race horse), and higher primates. In some embodiments, the subject is a human. In some embodiments, the subject has a disease. In some embodiments, the subject has cancer. In some embodiments, the subject has a viral disease. In some embodiments, the subject has a bacterial disease. In some embodiments, the subject has a fungal disease. In some embodiments, the subject has a parasitic disease. In some embodiments, the subject has asthma. In some embodiments, the subject has an autoimmune disease. In some embodiments, the subject has graft vs. host disease.

### Engineered TSC2 Polypeptides that Exhibit Decreased Ability to be Phosphorylated

Provided herein are engineered TSC2 polypeptides that cannot be phosphorylated at a residue that has been substituted for a serine residue, or that cannot be phosphorylated to the extent that a non-engineered TSC2 polypeptide having the serine residue can be phosphorylated. For example, provided herein are engineered TSC2 polypeptides having an amino acid substitution at a residue corresponding to the serine residue at position S1365 of the human TSC2 polypeptide sequence (SEQ ID NO: 5). As disclosed above, the present invention provides engineered TSC2 polypeptides having an amino acid substitution to alanine or glutamic acid at a residue corresponding to the serine residue at position S1365 of the human TSC2 polypeptide sequence (SEQ ID NO: 5) and optionally a further substitution to alanine or glutamic acid at a residue corresponding to the serine residue at position S1364 of the human TSC2 polypeptide sequence. Substitutions at other positions or to different amino acids are disclosed herein for reference.

In some disclosures, the serine residue at position S1365 of SEQ ID NO: 5 (or a corresponding amino acid residue in a different TSC2 polypeptide) is substituted with an amino acid with an aliphatic side chain other than alanine or glutamic acid. In some disclosures, the serine residue at position S1364 and/or S1365 of SEQ ID NO: 5, or a serine residue in a TSC2 polypeptide that corresponds to these serine residues, is/are substituted with an amino acid with an aliphatic side chain other than alanine or glutamic acid. In some disclosures, the serine residue at position S1365 of SEQ ID NO: 5 is substituted with a methionine residue, a valine residue, a leucine residue, an isoleucine residue, or a phenylalanine residue. In some disclosuress, the serine residue at position 51364 and/or S1365 of SEQ ID NO: 5, or a serine residue in a polypeptide that corresponds to these serine residues, is/are substituted with a methionine residue, a valine residue, a leucine residue, an isoleucine residue, or a phenylalanine residue.

In some embodiments of the present invention, an engineered TSC2 polypeptide having an alanine substitution at the serine residue at position S1365 of the human TSC2 polypeptide sequence is provided (*e.g*., SEQ ID NO: 1). In some embodiments of the present invention, an engineered TSC2 polypeptide having alanine substitutions at the serine residue positions S1364 and S1365 of the human TSC2 polypeptide sequence is provided (e.g., SEQ ID NO: 20). In some embodiments, engineered TSC2 polypeptides disclosed herein cannot be phosphorylated to the extent that a non-engineered TSC2 polypeptide having the serine residue(s) can be phosphorylated. In some embodiments, engineered TSC2 polypeptides disclosed herein are phosphorylated to an extent that is about 5%, 10%, 15%, 20%, 25%, 30%, 35%, 40%, 45%, 50%, 55%, 60%, 65%, 70%, 75%, 80%, 85%, 90%, 95% or less than a non-engineered TSC2 polypeptide having the serine residue(s). In some embodiments, engineered TSC2 polypeptides disclosed herein cannot be phosphorylated.

Disclosed herein for reference are also engineered TSC2 polypeptides having an amino acid substitution at a residue corresponding to the serine residue at position S1366 of the mouse TSC2 polypeptide sequence (SEQ ID NO: 6) such that engineered TSC2 polypeptides cannot be phosphorylated at that residue, or cannot be phosphorylated to the extent that a non-engineered TSC2 polypeptide having the serine residue can be phosphorylated. In some disclosure, engineered TSC2 polypeptides provided herein have an amino acid substitution at a residue corresponding to the serine residue at position S1365 and/or S1366 of the mouse TSC2 polypeptide sequence (SEQ ID NO: 6) such that engineered TSC2 polypeptides cannot be phosphorylated at that residue, or cannot be phosphorylated to the extent that a non-engineered TSC2 polypeptide having the serine residue can be phosphorylated. In some disclosures, the serine residue at position S1366 of SEQ ID NO: 6 is substituted with an amino acid with an aliphatic side chain. In some embodiments, the serine residue at position S1365 and/or S1366 of SEQ ID NO: 6, or a serine residue in a TSC2 polypeptide that corresponds to these serine residue, is/are substituted with an amino acid with an aliphatic side chain. In some disclosures, the serine residue at position S1366 of SEQ ID NO: 6 is substituted with a methionine residue, an alanine residue, a valine residue, a leucine residue, an isoleucine residue, or a phenylalanine residue. In some disclosures, the serine residue at position S1365 and/or S1366 of SEQ ID NO: 6, or a serine residue in a polypeptide that corresponds to these serine residues, is substituted with a methionine residue, an alanine residue, a valine residue, a leucine residue, an isoleucine residue, or a phenylalanine residue. In some disclosures, an engineered TSC2 polypeptide having an alanine substitution at the serine residue at position S1366 of the mouse TSC2 polypeptide sequence is provided (*e.g*., SEQ ID NO: 8). In some disclosures, an engineered TSC2 polypeptide having alanine substitutions at the serine residue positions S1365 and S1366 of the mouse TSC2 polypeptide sequence is provided (e.g., SEQ ID NO: 24). In some disclosures, engineered TSC2 polypeptides disclosed herein cannot be phosphorylated to the extent that a non-engineered TSC2 polypeptide having the serine residue(s) can be phosphorylated. In some embodiments, engineered TSC2 polypeptides disclosed herein are phosphorylated to an extent that is about 5%, 10%, 15%, 20%, 25%, 30%, 35%, 40%, 45%, 50%, 55%, 60%, 65%, 70%, 75%, 80%, 85%, 90%, 95% or less than a non-engineered TSC2 polypeptide having the serine residue(s). In some embodiments, engineered TSC2 polypeptides disclosed herein cannot be phosphorylated.

Disclosed herein for reference are also engineered TSC2 polypeptides having an amino acid substitution at a residue corresponding to the serine residue at position S1367 of the rat TSC2 polypeptide sequence (SEQ ID NO: 7) such that engineered TSC2 polypeptides cannot be phosphorylated at that residue, or cannot be phosphorylated to the extent that a non-engineered TSC2 polypeptide having the serine residue can be phosphorylated. In some disclosures, engineered TSC2 polypeptides have an amino acid substitution at a residue corresponding to the serine residue at position S1366 and/or S1367 of the rat TSC2 polypeptide sequence (SEQ ID NO: 7) such that engineered TSC2 polypeptides cannot be phosphorylated at that residue, or cannot be phosphorylated to the extent that a non-engineered TSC2 polypeptide having the serine residue can be phosphorylated. In some disclosures, the serine residue at position S1367 of SEQ ID NO: 7 is substituted with an amino acid with an aliphatic side chain. In some disclosures, the serine residue at position S1366 and/or S1377 of SEQ ID NO: 7, or a serine residue in a TSC2 polypeptide that corresponds to these serine residues, is/are substituted with an amino acid with an aliphatic side chain. In some disclosures, the serine residue at position S1367 of SEQ ID NO: 7 is substituted with a methionine residue, an alanine residue, a valine residue, a leucine residue, an isoleucine residue, or a phenylalanine residue. In some disclosures, the serine residue at position S1366 and/or S1367 of SEQ ID NO: 7, or a serine residue in a polypeptide that corresponds to these serine residues, is/are substituted with a methionine residue, an alanine residue, a valine residue, a leucine residue, an isoleucine residue, or a phenylalanine residue. In some disclosures, an engineered TSC2 polypeptide having an alanine substitution at the serine residue at position S1367 of the rat TSC2 polypeptide sequence is provided (SEQ ID NO: 9). In some disclosures, an engineered TSC2 polypeptide having alanine substitutions at the serine residue positions S1366 and S1367 of the rat TSC2 polypeptide sequence is provided (SEQ ID NO: 26). In some disclosures, engineered TSC2 polypeptides disclosed herein cannot be phosphorylated to the extent that a non-engineered TSC2 polypeptide having the serine residue(s) can be phosphorylated. In some embodiments, engineered TSC2 polypeptides disclosed herein are phosphorylated to an extent that is about 5%, 10%, 15%, 20%, 25%, 30%, 35%, 40%, 45%, 50%, 55%, 60%, 65%, 70%, 75%, 80%, 85%, 90%, 95% or less than a non-engineered TSC2 polypeptide having the serine(s). In some disclosuress, engineered TSC2 polypeptides disclosed herein cannot be phosphorylated.

Disclosed herein for reference are also vectors that include nucleic acid sequences that encode polypeptides that cannot be phosphorylated at an amino acid position, S1365 and/or S1366 of the mouse TSC2 polypeptide sequence (SEQ ID NO: 6), or S1366 and/or S1367 of the rat TSC2 polypeptides sequence (SEQ ID NO: 7), or that cannot be phosphorylated to the extent that a TSC2 polypeptide having a serine residue at these positions can be phosphorylated, and cells having such vectors.

A vector that includes nucleic acid sequences that encode polypeptides that cannot be phosphorylated at position corresponding to S1364 and/or S1365 of the human TSC2 polypeptide sequence (SEQ ID NO: 5), S1365 and/or S1366 of the mouse TSC2 polypeptide sequence (SEQ ID NO: 6), or S1366 and/or S1367 of the rat TSC2 polypeptide sequence (SEQ ID NO: 7), or that cannot be phosphorylated to the extent that a TSC2 polypeptide having a serine residue at these positions can be phosphorylated can be any appropriate type of vector.

The present invention provides cells comprising a vector of the present invention (i.e., a vector comprising a nucleic acid sequence encoding a polypeptide comprising any one of SEQ ID NO: 1, SEQ ID NO: 2, SEQ ID NO: 20, and SEQ ID NO:21), wherein the nucleic acid encoding the polypeptide comprising any one of SEQ ID NO:1, SEQ ID NO: 2, SEQ ID NO: 20, and SEQ ID NO:21 is operably linked to a nucleic acid that expression of the polypeptide in the cell.

Examples of vectors include, without limitation, plasmids (*e.g*., expression plasmids) and vectors (*e.g*., viral vectors such as lentiviral vectors, retroviral vectors, adenovirus vectors, and adeno-associated virus vectors). In some cases, a vector that includes nucleic acid sequences that encode polypeptides that cannot be phosphorylated at position corresponding to S1364 and/or S1365 of the human TSC2 polypeptide sequence (SEQ ID NO: 5), S1365 and/or S1366 of the mouse TSC2 polypeptide sequence (SEQ ID NO: 6), or S1366 and/or S1367 of the rat TSC2 polypeptides sequence (SEQ ID NO: 7), or that cannot be phosphorylated to the extent that a TSC2 polypeptide having a serine residue at these positions can be phosphorylated can be a lentiviral vector. A vector that includes nucleic acid sequences that encode polypeptides that cannot be phosphorylated at position corresponding to S1364 and/or S1365 of the human TSC2 polypeptide sequence (SEQ ID NO: 5), S1365 and/or S1366 of the mouse TSC2 polypeptide sequence (SEQ ID NO: 6), or S1366 and/or S1367 of the rat TSC2 polypeptides sequence (SEQ ID NO: 7), or that cannot be phosphorylated to the extent that a TSC2 polypeptide having a serine residue at these positions can be phosphorylated also can include one or more additional features (*e.g*., one or more additional features to modulate polypeptide expression). Examples of features that can modulate polypeptide expression include, without limitation, an origin of replication, a promoter, a polyA tail, a terminator, and a microRNA response element. In some cases, when a vector described herein also includes a promoter, the promoter can be operably linked to a nucleic acid sequence that encodes polypeptides that cannot be phosphorylated at position corresponding to S1364 and/or S1365 of the human TSC2 polypeptide sequence (SEQ ID NO: 5), S1365 and/or S1366 of the mouse TSC2 polypeptide sequence (SEQ ID NO: 6), or S1366 and/or S1367 of the rat TSC2 polypeptides sequence (SEQ ID NO: 7), or that cannot be phosphorylated to the extent that a TSC2 polypeptide having a serine residue at these positions can be phosphorylated (*e.g*., such that the promoter can drive expression of the nucleic acid sequence). A promoter can be any appropriate promoter. In some cases, a promoter can be a constitutive promoter. In some cases, a promoter can be a viral promoter. In some cases, a promoter can be an inducible promoter. In some cases, a promoter can be a cell-specific and/or tissue-specific promoter. Examples of promoters that can be used to drive expression of nucleic acid sequences that encode polypeptides that cannot be phosphorylated at position corresponding to S 1364 and/or S1365 of the human TSC2 polypeptide sequence (SEQ ID NO: 5), S1365 and/or S1366 of the mouse TSC2 polypeptide sequence (SEQ ID NO: 6), or S1366 and/or S1367 of the rat TSC2 polypeptides sequence (SEQ ID NO: 7), or that cannot be phosphorylated to the extent that a TSC2 polypeptide having a serine residue at these positions can be phosphorylated include, without limitation, CMV In some embodiments, a promoter can be as described elsewhere (see, *e.g.,* Morgan et al., 2016 Biomedicines. 4:9).

In some embodiments, cells having vectors that include nucleic acid sequences that encode engineered TSC2 polypeptides that cannot be phosphorylated at a position that corresponds to a wild-type serine residue in any of the polypeptides described herein (or that cannot be phosphorylated to the extent that a TSC2 polypeptide having a serine residue at these positions can be phosphorylated) do not express endogenous, wild-type TSC2. For example, the nucleic acid sequence encoding wild-type TSC2 can be modified by any of a variety of genetic manipulation techniques known in the art including, but not limited to, CRISPR-based methods, TALEN-based methods, and other genetic targeting or recombination methods (see, *e.g.,* Roth et al., 2018 Nature 559:405-409).

TSC2 polypeptides disclosed herein for reference can be engineered at amino acid positions S1365 and/or S1366 of the mouse TSC2 polypeptide sequence (SEQ ID NO: 6), or S1366 and/or S1367 of the rat TSC2 polypeptide sequence (SEQ ID NO: 7) with any mutation or modification that results in TSC2 being unable to be phosphorylated (or that results in a decreased ability of TSC2 to be phosphorylated) at the respective positions in human (S1364, S1365), mouse (S1365, S1366), or rat (S1366, S1367). In some cases, TSC2 polypeptides from other species (*e.g*., monkey) can be engineered with any mutation or modification (*e.g*., a residue corresponding to human S1364 and/or S1365, mouse S1365 and/or S1366, and/or rat S1366 and/or S1367) that results in TSC2 being unable to be phosphorylated (or that results in a decreased ability of TSC2 to be phosphorylated).

Provided herein are also nucleic acids encoding engineered TSC2 polypeptides that cannot be phosphorylated at positions corresponding to a serine residue in the wild-type TSC2 polypeptide sequence, or that cannot be phosphorylated to the extent that a non-engineered TSC2 polypeptide having the serine residue can be phosphorylated. Provided herein are nucleic acids encoding engineered TSC2 polypeptides having an amino acid substitution at a residue corresponding to the serine residue at position S1365 of the human TSC2 polypeptide sequence (SEQ ID NO: 5) to alanine or glutamic acid and optionally a further substitution at position S1364 to the same amino acid. In some embodiments, a nucleic acid sequence encoding an engineered TSC2 polypeptide having an amino acid substitution at the serine residue at position S1364 and S1365 of the human TSC2 polypeptide sequence is provided (*e.g*., the nucleic acid sequence of SEQ ID NO: 22).

Disclosed herein for reference are nucleic acids encoding engineered TSC2 polypeptides having an amino acid substitution at a residue corresponding to the serine residue at position S1365 and/or S1366 of the mouse TSC2 polypeptide sequence (SEQ ID NO: 6), or position S1366 and/or S1367 of the rat TSC2 polypeptide sequence (SEQ ID NO: 7). In some embodiments, a nucleic acid sequence encoding an engineered TSC2 polypeptide having an amino acid substitution at the serine residue at position S1365 of the human TSC2 polypeptide sequence is provided (e.g., the nucleic acid sequence of SEQ ID NO: 3). Disclosed herein for reference, nucleic acids provided herein encode engineered TSC2 polypeptides in which the serine residue at position S1365 of SEQ ID NO: 5, or a serine residue in a TSC2 polypeptide that corresponds to the serine residues at this position, is substituted with an amino acid with an aliphatic side chain other than alanine and glutamic acid. In some disclosures, nucleic acids provided herein encode engineered TSC2 polypeptides in which the serine residue at position S1364 and/or S1365 of SEQ ID NO: 5 (other than alanine or glutamic acid), or position S1365 and/or 51366 of SEQ ID NO: 6, or position S13666 and/or S1367 of SEQ ID NO: 7, or a serine residue in a TSC2 polypeptide that corresponds to the serine residues at these positions, are substituted with an amino acid with an aliphatic side chain. In some disclosures, nucleic acids provided herein encode engineered TSC2 polypeptides in which the serine residue at position S1364 and/or S1365 of SEQ ID NO: 5, or a serine residue in a TSC2 polypeptide that corresponds to the serine residues at this position, is substituted with a methionine residue, a valine residue, a leucine residue, an isoleucine residue, or a phenylalanine residue. In some disclosed herein for reference, nucleic acids provided herein encode engineered TSC2 polypeptides in which the serine residue at position S1365 and/or S1366 of SEQ ID NO: 6, or position S1366 and/or S1367 of SEQ ID NO: 7, or a serine residue in a TSC2 polypeptide that corresponds to the serine residues at these positions, are substituted with a methionine residue, an alanine residue, a valine residue, a leucine residue, an isoleucine residue, or a phenylalanine residueIn some embodiments, nucleic acids disclosed herein encode engineered TSC2 polypeptides that are phosphorylated to an extent that is about 5%, 10%, 15%, 20%, 25%, 30%, 35%, 40%, 45%, 50%, 55%, 60%, 65%, 70%, 75%, 80%, 85%, 90%, 95% or less than a non-engineered TSC2 polypeptide having the serine residue(s) can be phosphorylated. In some embodiments, nucleic acids disclosed herein encode engineered TSC2 polypeptides that cannot be phosphorylated.

### Cells Having Engineered TSC2 Polypeptides that Exhibit Reduced Ability to be Phosphorylated For Use in Treating Disease

### Cells having engineered TSC2 polypeptides that Exhibit Reduced Ability to be Phosphorylated

Provided herein are cells expressing engineered TSC2 polypeptides that cannot be phosphorylated at position corresponding to a serine residue in the wild-type TSC2 polypeptide sequence, or that cannot be phosphorylated to the full extent as that of a wild-type TSC2 polypeptide having the serine residue.

The present invention provides cells expressing engineered TSC2 polypeptides having an amino acid substitution to alanine or glutamic acid at a position corresponding to the serine residue at position S1365, or at both S1364 and S1365, of the human TSC2 polypeptide sequence (SEQ ID NO: 5).. In some embodiments, cells disclosed herein express an engineered TSC2 polypeptide (*e.g*., SEQ ID NO: 1) having an amino acid substitution at the serine residue at position S1365 of the human TSC2 polypeptide sequence. In some embodiments, cells provided herein express engineered TSC2 polypeptides that cannot be phosphorylated to the extent that a non-engineered TSC2 polypeptide having the serine residue(s) can be phosphorylated. In some embodiments, cells provided herein express engineered TSC2 polypeptides that are phosphorylated to an extent that is about 5%, 10%, 15%, 20%, 25%, 30%, 35%, 40%, 45%, 50%, 55%, 60%, 65%, 70%, 75%, 80%, 85%, 90%, 95% or less that a non-engineered TSC2 polypeptide having the serine residue(s). In some embodiments, cells provided herein express engineered TSC2 polypeptides that cannot be phosphorylated.

Disclosed herein for reference are also cells expressing engineered TSC2 polypeptides having an amino acid substitution at a residue corresponding to the serine residue at position S1366, or at both S1365 and S1366, of the mouse TSC2 polypeptide sequence (SEQ ID NO: 6) such that engineered TSC2 polypeptides cannot be phosphorylated at the engineered residue, or cannot be phosphorylated to the extent that a non-engineered TSC2 polypeptide having the serine residue can be phosphorylated. In some disclosuress, cells provided herein express an engineered TSC2 polypeptide in which the serine residue at position S1366 of SEQ ID NO: 6, or a serine residue in a TSC2 polypeptide that corresponds to the serine residue at position S1366 of SEQ ID NO: 6, is substituted with an amino acid with an aliphatic side chain. In some disclosuress, cells provided herein express an engineered TSC2 polypeptide in which the serine residue at position S1365 and/or S1366 of SEQ ID NO: 6, or a serine residue in a TSC2 polypeptide that corresponds to the serine residue at position S1365 and/or S1366 of SEQ ID NO: 6, is substituted with an amino acid with an aliphatic side chain. In some disclosures, cells provided herein express an engineered TSC2 polypeptide in which the serine residue at position S1366 of SEQ ID NO: 6, or a serine residue in a polypeptide that corresponds to the serine residue at position S1366 of SEQ ID NO: 6, is substituted with a methionine residue, an alanine residue, a valine residue, a leucine residue, an isoleucine residue, or a phenylalanine residue. In some disclosures, cells provided herein express an engineered TSC2 polypeptide in which the serine residue at position S1365 and/or S 1366 of SEQ ID NO: 6, or a serine residue in a polypeptide that corresponds to the serine residue at position S1365 and/or S1366 of SEQ ID NO: 6, is substituted with a methionine residue, an alanine residue, a valine residue, a leucine residue, an isoleucine residue, or a phenylalanine residue. In some disclosuress, cells provided herein express an engineered TSC2 polypeptide (*e.g*., SEQ ID NO: 8) having an amino acid substitution at the serine residue at position S1366 of the mouse TSC2 polypeptide sequence. In some disclosuress, cells provided herein express an engineered TSC2 polypeptide (e.g., SEQ ID NO: 24) having amino acid substitutions at the serine residue positions S1365 and S1366 of the mouse TSC2 polypeptide sequence. In some disclosures, cells provided herein express engineered TSC2 polypeptides that cannot be phosphorylated to the extent that a non-engineered TSC2 polypeptide having the serine residue(s) can be phosphorylated. In some disclosures, cells provided herein express engineered TSC2 polypeptides that are phosphorylated to an extent that is about 5%, 10%, 15%, 20%, 25%, 30%, 35%, 40%, 45%, 50%, 55%, 60%, 65%, 70%, 75%, 80%, 85%, 90%, 95% or less that a non-engineered TSC2 polypeptide having the serine residue(s). In some disclosures, cells provided herein express engineered TSC2 polypeptides that cannot be phosphorylated.

Disclosed herein for reference are also cells expressing an engineered TSC2 polypeptide having an amino acid substitution at a residue corresponding to the serine residue at position S1367, or at both S1366 and S1367, of the rat TSC2 polypeptide sequence (SEQ ID NO: 7) such that engineered TSC2 polypeptides cannot be phosphorylated at the engineered residue, or cannot be phosphorylated to the extent that a non-engineered TSC2 polypeptide having the serine residue can be phosphorylated. In some disclosures, cells provided herein express an engineered TSC2 polypeptide in which the serine residue at position S1367 of SEQ ID NO: 7, or a serine residue in a TSC2 polypeptide that corresponds to the serine residue at position S1367 of SEQ ID NO: 7, is substituted with an amino acid with an aliphatic side chain. In some disclosures, cells provided herein express an engineered TSC2 polypeptide in which the serine residue at position S1366 and/or S1367 of SEQ ID NO: 7, or a serine residue in a TSC2 polypeptide that corresponds to the serine residue at position S1366 and/or S1367 of SEQ ID NO: 7, is substituted with an amino acid with an aliphatic side chain. In some disclosurets, cells provided herein express an engineered TSC2 polypeptide in which the serine residue at position S1367 of SEQ ID NO: 7, or a serine residue in a polypeptide that corresponds to the serine residue at position S1367 of SEQ ID NO: 7, is substituted with a methionine residue, an alanine residue, a valine residue, a leucine residue, an isoleucine residue, or a phenylalanine residue. In some disclosures, cells provided herein express an engineered TSC2 polypeptide in which the serine residue at position S1366 and/or S1367 of SEQ ID NO: 7, or a serine residue in a polypeptide that corresponds to the serine residue at position S1366 and/or S1367 of SEQ ID NO: 7, is substituted with a methionine residue, an alanine residue, a valine residue, a leucine residue, an isoleucine residue, or a phenylalanine residue. In some disclosures, cells provided herein express an engineered TSC2 polypeptide (*e.g*., SEQ ID NO: 9) having an amino acid substitution at the serine residue at position S1367 of the rat TSC2 polypeptide sequence. In some disclosures, cells provided herein express an engineered TSC2 polypeptide (e.g., SEQ ID NO: 26) having amino acid substitutions at the serine residue positions S1366 and S1367 of the rat TSC2 polypeptide sequence. In some disclosures, cells provided herein express engineered TSC2 polypeptides that cannot be phosphorylated to the extent that a non-engineered TSC2 polypeptide having the serine residue(s) can be phosphorylated. In some disclosurets, cells provided herein express engineered TSC2 polypeptides that are phosphorylated to an extent that is about 5%, 10%, 15%, 20%, 25%, 30%, 35%, 40%, 45%, 50%, 55%, 60%, 65%, 70%, 75%, 80%, 85%, 90%, 95% or less that a non-engineered TSC2 polypeptide having the serine residue(s). In some disclosures, cells provided herein express engineered TSC2 polypeptides that cannot be phosphorylated.

The present invention further provides cells harboring vectors that include nucleic acid sequences that encode polypeptides that cannot be phosphorylated at a residue corresponding to S1364 and/or S1365 of the human TSC2 polypeptide sequence (SEQ ID NO: 5), wherein the nucleic acid sequences encode a polypeptide comprising any one of SEQ ID NO: 1; SEQ ID NO: 20; SEQ ID NO: 2 and SEQ ID NO: 21. A vector that includes nucleic acid sequences that encode polypeptides that cannot be phosphorylated at position corresponding to S1364 and/or S1365 of the human TSC2 polypeptide sequence (SEQ ID NO: 5) can be introduced into a cell using any appropriate methods and/or techniques.

A vector can be introduced into a cell in a transient manner (*e.g*., maintained as a vector) or in a stable manner (*e.g*., integrated into the genome). Examples of methods and/or techniques that can be used to introduce one or more vectors into a cell include, without limitation, transfection, transduction, electroporation, and infection. In some embodiments, nucleic acids encoding engineered TSC2 polypeptides are operably linked to nucleic acids that drive expression of the engineered TSC2 polypeptides in the vectors (*e.g*., promoter sequences).

In some embodiments, cells having engineered TSC2 polypeptides that cannot be phosphorylated, or that cannot be phosphorylated to the extent of a wild-type TSC2 polypeptide can have, or can express, endogenous TSC2 proteins. For example, a cell having an engineered TSC2 polypeptide also can have an endogenous wild-type nucleic acid sequence encoding a wild-type TSC2 polypeptide. In some embodiments of cells having (*e.g*., expressing) both: 1) engineered TSC2 polypeptides that cannot be phosphorylated, or that cannot be phosphorylated to the extent of a wild-type TSC2 polypeptide, and 2) an endogenous TSC2 protein, the cells exhibit the same or similar activity as a corresponding cell lacking (*e.g*., not expressing) the endogenous TSC2 protein.

In some embodiments, cells having engineered TSC2 polypeptides that cannot be phosphorylated, or that cannot be phosphorylated to the extent of a wild-type TSC2 polypeptide, do not have, or do not express, endogenous, wild-type TSC2 proteins. For example, a cell having an engineered TSC2 polypeptide can have a genetic alteration in which a wild-type nucleic acid sequence encoding the TSC2 polypeptide has been rendered inactive. In some embodiments, the nucleic acid sequence encoding wild-type TSC2 can be modified by any of a variety of genetic manipulation techniques known in the art including, but not limited to, CRISPR-based methods, TALEN-based methods, and other genetic targeting or recombination methods (see, *e.g.,* Roth et al., 2018 Nature 559:405-409). In some embodiments, a wild-type nucleic acid sequence encoding a TSC2 polypeptide can be rendered inactive by removing, replacing, or mutating a nucleic acid sequence that contributes to expression of the TSC2 polypeptide including, but not limited to, a promoter sequence, an enhancer sequence, a coding sequence of a transcription factor that regulates expression of TSC2, the coding sequence of the TSC2 polypeptide itself, or combinations thereof. In some embodiments, a wild-type nucleic acid sequence encoding a TSC2 polypeptide can be rendered inactive via a frameshift caused by one or more modifications or mutations in the nucleic acid sequence encoding the TSC2 polypeptide.

In some embodiments, cells that can be engineered to include an engineered TSC2 polypeptide that cannot be phosphorylated at a position that corresponds to a wild-type serine residue (or that cannot be phosphorylated to the extent that a TSC2 polypeptide having a serine residue at these positions can be phosphorylated) include immune cells. For example, the immune cells can be CD4+ T cells, CD8+ T cells, Natural Killer cells (NK cells), macrophages, neutrophils, regulatory T cells (Tregs), helper T cells, or any other immune cells and/or inflammatory cells known in the art.

CD8+ T cells have been investigated in T cell-based therapies for their role in cellular immune responses. Tumor-specific CD8+ T cells have been found in patients with hematologic malignancies and solid tumors and within the pool of tumor-infiltrating lymphocytes. (Zanetti M, Tapping CD4 T cells for cancer immunotherapy: the choice of personalized genomics, J Immunol. 2015 Mar 1;194(5):2049-56). The role of CD8+ T cells has been demonstrated in mouse models of cancer. The presence of CD8+ T cells in tumors has also been shown in human. It has also been shown that the engagement of checkpoint receptors on activated CD8+ T cells represents a major mechanism of tumorinduced immunosuppression. In addition, a high density of CD8+ T cells has been found to be associated with longer patient survival when tumors display high densities of tertiary lymphoid structures in lung, colorectal, and renal cell cancers (see, *e.g.,* Teng et al., 2015 J Clin Invest. 125:3338-46).

In some embodiments, CD8+ effector T cells can be generated by stimulating the splenocytes and expanding them in effector promoting conditions with IL-2. Strong IL-2 signaling preferentially skews CD8+ T cells to differentiate into effector T cells. After some time, CD8+ cultures can be processed to remove non-viable cells and assessed for effector function by re-stimulating viable cells with PMAand Ionomycin along with a Golgi blocker to capture cytokines within the cells for later flow cytometry analysis. Cells can then be processed and stained to assess cytokine function. High expression of Interferon gamma (IFNg), tumor necrosis factor alpha (TNFa), and Interleukin-2 (IL-2) are indicators of CD8+ effector T cells.

CD4+ T cells are also known to play a role in adaptive immune responses. Various types of CD4+ T cells contribute to anti-tumor immunity through their diverse functions. For example, CD4+ T cells facilitate B cells with isotype switching and affinity maturation. CD4+ T cells are also involved in facilitating the activation and expansion of CD8+ T cells, and the generation and maintenance of memory CD8+ T cells. CD4+ T cells are further involved in tumor protection. For example, activated CD4+ T cells have been found to induce delayed-type hypersensitivity-like reactions and attract inflammatory cells including macrophages, granulocytes, eosinophils, and NK cells in or around the tumor.

A T regulatory cell or "Treg cell" refers to a cell that can modulate a T cell response. Regulatory T cells are known for their ability to downregulate the function of other T cells. (Zanetti M, Tapping CD4 T cells for cancer immunotherapy: the choice of personalized genomics, J Immunol. 2015 Mar 1;194(5):2049-56). Treg cells express the transcription factor Foxp3, which is not upregulated upon T cell activation and discriminates Tregs from activated effector cells. Tregs are identified by the cell surface markers CD25, CTLA4, and GITR. Several Treg subsets have been identified that have the ability to inhibit autoimmune and chronic inflammatory responses and to maintain immune tolerance in tumor-bearing hosts. These subsets include interleukin 10- (IL-10-) secreting T regulatory type 1 (Tr1) cells, transforming growth factor-β- (TGF-β-) secreting T helper type 3 (Th3) cells, and "natural" CD4+/CD25+ Tregs (Trn).

In some embodiments, an immune cell that is engineered to include an engineered TSC2 polypeptide can be a cytotoxic T cell. In some embodiments, a cytotoxic T cell can be engineered to express a chimeric antigen receptor ("CAR") or a T cell receptor ("TCR").

In some embodiments, an immune cell that is engineered to include an engineered TSC2 polypeptide can be a B cell. B cells are known to be involved in immune responses and T cell activation. For example, B cells can act as antigen-specific antigen presenting cells. A signal through binding of an antigen to membrane Ig can enhance B cell antigen presentation and T-cell-dependent B cell activation. As a result of helper T cell recognition of antigen on the B cell surface, the T cell becomes activated and then activates the B cell.

In some embodiments, an immune cell that is engineered to include an engineered TSC2 polypeptide can be expanded (*e.g*., clonally expanded). For example, an immune cell that is engineered to include an engineered TSC2 polypeptide can be clonally expanded ex vivo (*e.g*., for use in an adoptive cell therapy). In cases where an immune cell that is engineered to include an engineered TSC2 polypeptide is used in an adoptive cell therapy, the adoptive cell therapy can be any appropriate adoptive cell therapy. Examples of adoptive cell therapies include, without limitation, dendritic cell therapy and synthetic dendritic cell therapy. In some cases, adoptive cell therapy can include the extraction of tumor infiltrating lymphocytes.

In some embodiments, increased mTORC1 signaling can be determined by any of a variety of techniques or methods known in the art. For example, mTORC1 signaling typically results in increased growth, decreased autophagy, and phosphorylation of Ulk1 (Unc-51-like kinase-1), p70S6K, and 4EBP1 (elF4E binding protein-1).

### Cancers

Compositions and methods disclosed herein can be used to treat cancer. For example, an immune cell that is engineered to include an engineered TSC2 polypeptide (*e.g*., expressed from a vector introduced into the cell, which vector includes a nucleic acid sequence that encodes the engineered TSC2 polypeptide) that cannot be phosphorylated at a residue corresponding to S1364 and/or S1365 of the human TSC2 polypeptide sequence (SEQ ID NO: 5), S1365 and/or S1366 of the mouse TSC2 polypeptide sequence (SEQ ID NO: 6), or S1366 and/or S1367 of the rat TSC2 polypeptides sequence (SEQ ID NO: 7), or that cannot be phosphorylated to the full extent as that of a wild-type TSC2 polypeptide, can be administered to a subject having cancer (*e.g*., in an adoptive cell therapy) such that the cancer is treated. In some embodiments, the engineered immune cell administered to the subject does not have or express an endogenous, wild type TSC polypeptide. In some embodiments, the engineered immune cell administered to the subject does have or express an endogenous, wild type TSC2 polypeptide. The present invention provides an immune cell that is engineered to include a polypeptide comprising any one of SEQ ID NO: 1, SEQ ID NO: 2, SEQ ID NO:20, and SEQ ID NO:21. In some embodiments, a CD8+ T effector cell that recognizes a cancer cell (*e.g*., via a specific antigen on the cancer cell surface) can be engineered to include a TSC2 polypeptide that cannot be phosphorylated, or that cannot be phosphorylated to the full extent as that of a wild-type TSC2 polypeptide, which CD8+ T effector cell is then administered to a subject that has such a cancer cell. In some embodiments, a CD8+ T effector cell that is engineered to include a TSC2 polypeptide that cannot be phosphorylated, or that cannot be phosphorylated to the full extent as that of a wild-type TSC2 polypeptide, is also engineered to express a chimeric antigen receptor or a T cell receptor. In some embodiments, an immune cell that is engineered to include an engineered TSC2 polypeptide that cannot be phosphorylated, or that cannot be phosphorylated to the full extent as that of a wild-type TSC2 polypeptide, is more effective in treating cancer in a subject than an immune cell that lacks the engineered TSC2 polypeptide.

Cancer types that can be treated include, without limitation, lung cancer (*e.g*., small cell lung carcinoma or non-small cell lung carcinoma), papillary thyroid cancer, medullary thyroid cancer, differentiated thyroid cancer, recurrent thyroid cancer, refractory differentiated thyroid cancer, lung adenocarcinoma, bronchioles lung cell carcinoma, multiple endocrine neoplasia type 2A or 2B (MEN2A or MEN2B, respectively), pheochromocytoma, parathyroid hyperplasia, breast cancer, colorectal cancer (*e.g*., metastatic colorectal cancer), papillary renal cell carcinoma, ganglioneuromatosis of the gastroenteric mucosa, inflammatory myofibroblastic tumor, or cervical cancer, acute lymphoblastic leukemia (ALL), acute myeloid leukemia (AML), cancer in adolescents, adrenal cancer, adrenocortical carcinoma, anal cancer, appendix cancer, astrocytoma, atypical teratoid/rhabdoid tumor, basal cell carcinoma, bile duct cancer, bladder cancer, bone cancer, brain stem glioma, brain tumor, breast cancer, bronchial tumor, Burkitt lymphoma, carcinoid tumor, unknown primary carcinoma, cardiac tumors, cervical cancer, childhood cancers, chordoma, chronic lymphocytic leukemia (CLL), chronic myelogenous leukemia (CML), chronic myeloproliferative neoplasms, colon cancer, colorectal cancer, craniopharyngioma, cutaneous T-cell lymphoma, bile duct cancer, ductal carcinoma in situ, embryonal tumors, endometrial cancer, ependymoma, esophageal cancer, esthesioneuroblastoma, Ewing sarcoma, extracranial germ cell tumor, extragonadal germ cell tumor, extrahepatic bile duct cancer, eye cancer, fallopian tube cancer, fibrous histiocytoma of bone, gallbladder cancer, gastric cancer, gastrointestinal carcinoid tumor, gastrointestinal stromal tumors (GIST), germ cell tumor, gestational trophoblastic disease, glioma, hairy cell tumor, hairy cell leukemia, head and neck cancer, heart cancer, hepatocellular cancer, histiocytosis, Hodgkin's lymphoma, hypopharyngeal cancer, intraocular melanoma, islet cell tumors, pancreatic neuroendocrine tumors, Kaposi sarcoma, kidney cancer, Langerhans cell histiocytosis, laryngeal cancer, leukemia, lip and oral cavity cancer, liver cancer, lung cancer, lymphoma, macroglobulinemia, malignant fibrous histiocytoma of bone, osteocarcinoma, melanoma, Merkel cell carcinoma, mesothelioma, metastatic squamous neck cancer, midline tract carcinoma, mouth cancer, multiple endocrine neoplasia syndromes, multiple myeloma, mycosis fungoides, myelodysplastic syndromes, myelodysplastic/myeloproliferative neoplasms, myelogenous leukemia, myeloid leukemia, multiple myeloma, myeloproliferative neoplasms, nasal cavity and paranasal sinus cancer, nasopharyngeal cancer, neuroblastoma, non-Hodgkin's lymphoma, non-small cell lung cancer, oral cancer, oral cavity cancer, lip cancer, oropharyngeal cancer, osteosarcoma, ovarian cancer, pancreatic cancer, papillomatosis, paraganglioma, paranasal sinus and nasal cavity cancer, parathyroid cancer, penile cancer, pharyngeal cancer, pheochromosytoma, pituitary cancer, plasma cell neoplasm, pleuropulmonary blastoma, pregnancy and breast cancer, primary central nervous system lymphoma, primary peritoneal cancer, prostate cancer, rectal cancer, renal cell cancer, retinoblastoma, rhabdomyosarcoma, salivary gland cancer, sarcoma, Sezary syndrome, skin cancer, small cell lung cancer, small intestine cancer, soft tissue sarcoma, squamous cell carcinoma, squamous neck cancer, stomach cancer, T-cell lymphoma, testicular cancer, throat cancer, thymoma and thymic carcinoma, thyroid cancer, transitional cell cancer of the renal pelvis and ureter, unknown primary carcinoma, urethral cancer, uterine cancer, uterine sarcoma, vaginal cancer, vulvar cancer, Waldenstrom Macroglobulinemia, and Wilms' tumor.

### Viral Diseases

Any of the compositions disclosed herein can be used to treat viral diseases. Viral diseases that can be treated include, without limitation, diseases resulting from infection by an adenovirus, a herpesvirus (*e.g*., HSV-I, HSV-II, CMV, or VZV), a poxvirus (*e.g*., an orthopoxvirus such as variola or vaccinia, or molluscum contagiosum), a picomavirus (*e.g*., rhinovirus or enterovirus), an orthomyxovirus (*e.g*., influenza virus), a paramyxovirus (*e.g*., parainfluenzavirus, mumps virus, measles virus, and respiratory syncytial virus (RSV)), a coronavirus (*e.g*., SARS), a papovavirus (*e.g*., papillomaviruses, such as those that cause genital warts, common warts, or plantar warts), a hepadnavirus (*e.g.,* hepatitis B virus), a flavivirus (*e.g*., hepatitis C virus or Dengue virus), or a retrovirus (*e.g*., a lentivirus such as HIV). Viral diseases that can be treated also include viral skin diseases, such as Herpes or shingles, and systemic viral diseases such as influenza, the common cold, and encephalitis.

### Bacterial Diseases

Any of the compositions and methods disclosed herein can be used to treat bacterial diseases. Bacterial diseases that can be treated include, without limitation, diseases resulting from infection by bacteria of, for example, the genus Escherichia, Enterobacter, Salmonella, Staphylococcus, Shigella, Listeria, Aerobacter, Helicobacter, Klebsiella, Proteus, Pseudomonas, Streptococcus, Chlamydia, Mycoplasma, Pneumococcus, Neisseria, Clostridium, Bacillus, Corynebacterium, Mycobacterium, Campylobacter, Vibrio, Serratia, Providencia, Chromobacterium, Brucella, Yersinia, Haemophilus, Bordetella, or Borrelia.

### Fungal Diseases

Any of the compositions and methods disclosed herein can be used to treat fungal diseases. Fungal diseases that can be treated include, without limitation, candidiasis, aspergillosis, histoplasmosis, and cryptococcal meningitis.

### Parasitic Diseases

Any of the compositions and methods disclosed herein can be used to treat parasitic diseases. Parasitic diseases that can be treated include, without limitation, malaria, pneumocystis carnii pneumonia, leishmaniasis, cryptosporidiosis, toxoplasmosis, and trypanosome infection.

### An Agent for use in the treatment of a Disease that Result in Reduced Phosphorylation of TSC2 Polypeptides in Immune Cells

The present invention provides an engineered immune cell comprising a vector for use in the treatment of a disease in a subject;
wherein the vector comprises a nucleic acid encoding a polypeptide comprising SEQ ID NO: 1 or SEQ ID NO: 20 operably linked to a promoter that drives expression of the polypeptide in the T cell; and
wherein upon recognizing an antigen associated with the disease, the engineered immune cell exhibits increased activity as compared to a reference immune cell that lacks the vector; wherein the increased activity comprises increased mTORC1 signalling.

Disclosed herein for reference are also methods of treating a disease (*e.g*., any of the variety of cancers, viral diseases, bacterial diseases, fungal diseases, or parasitic diseases disclosed herein) in a subject by administering one or more agents that result in reduced phosphorylation of TSC2 polypeptides in an immune cells. In some disclosures, administration of an agent that results in reduced phosphorylation of TSC2 polypeptides in immune cells results in reduced phosphorylation of a TSC2 polypeptide at a serine residue at position S1365 of the human TSC2 polypeptide sequence (SEQ ID NO: 5). In some disclosures, administration of an agent that results in reduced phosphorylation of TSC2 polypeptides in immune cells results in reduced phosphorylation of a TSC2 polypeptide at serine residue positions S1364 and S1365 of the human TSC2 polypeptide sequence (SEQ ID NO: 5). In some disclosures, administration of an agent that results in reduced phosphorylation of TSC2 polypeptides in immune cells results in reduced phosphorylation of a TSC2 polypeptide at a serine residue at position S1366 of the mouse TSC2 polypeptide sequence (SEQ ID NO: 6). In some disclosures, administration of an agent that results in reduced phosphorylation of TSC2 polypeptides in immune cells results in reduced phosphorylation of a TSC2 polypeptide at serine residue positions S1365 and S1366 of the mouse TSC2 polypeptide sequence (SEQ ID NO: 6). In some disclosures, administration of an agent that results in reduced phosphorylation of TSC2 polypeptides in immune cells results in reduced phosphorylation of a TSC2 polypeptide at a serine residue at position S1367 of the rat TSC2 polypeptide sequence (SEQ ID NO: 7). In some disclosures, administration of an agent that results in reduced phosphorylation of TSC2 polypeptides in immune cells results in reduced phosphorylation of a TSC2 polypeptide at serine residue at positions S1366 and S1367 of the rat TSC2 polypeptide sequence (SEQ ID NO: 7). In some disclosures, administration of an agent to a subject that results in reduced phosphorylation of TSC2 polypeptides in immune cells results in a population of TSC2 polypeptides in the immune cell that exhibit decreased phosphorylation as compared to a population of TSC2 polypeptides in an immune cell in a reference subject that has not been administered the agent. For example, administration of an agent to a subject that results in reduced phosphorylation of TSC2 polypeptides in immune cells can result in a population of TSC2 polypeptides in the immune cell wherein at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, at least 99% or more of the TSC2 polypeptides in the cell are not phosphorylated (*e.g*., a serine residue at position S1364 and/or S1365 of the human TSC2 polypeptide sequence (SEQ ID NO: 5), at a serine residue at position S1365 and/or S1366 of the mouse TSC2 polypeptide sequence (SEQ ID NO: 6), or at a serine residue at position S1366 and/or S1367 of the rat TSC2 polypeptide sequence (SEQ ID NO: 7) as compared to a population of TSC2 polypeptides in an immune cell in a reference subject, wherein the reference subject has not been administered the agent. Methods of determining whether a TSC2 polypeptide or population of TSC2 polypeptides are phosphorylated are known in the art. For example, TSC2 polypeptides from an immune cell(s) from a subject that has been administered the agent or from an immune cell(s) that has been contacted with the agent *in vitro* can be isolated (*e.g*., with a TSC2-specific antibody) and the phosphorylation state of the TSC2 polypeptides can be assayed with a phospho-specific antibody. Those of ordinary skill in the art will be aware of other suitable methods for determining whether a TSC2 polypeptide or population of TSC2 polypeptides are phosphorylated.

In some disclosures, an agent that results in reduced phosphorylation of TSC2 polypeptides in immune cells is a kinase inhibitor. A variety of kinases are known that can be inhibited, including without limitation: AKT1, AKT2, AKT3, CRIK, DMPK1, DMPK2, MRCKa, MRCKb, ROCK1, ROCK2, BARK1, BARK2, GPRK4, GPRK5, GPRK6, GPRK7, RHOK, MAST1, MAST2, MAST3, MAST4, MASTL, LATS1, LATS2, NDR1, NDR2, PDK1, PKACa, PKACb, PKACg, PRKX, PRKY, PKC (e.g., PKCa, PKCb, PKCg, PKCd, PKCt, PKCe, PKCh, PKCi, or PKCz), PKN1, PKN2, PKN3, MSK1, MSK2, p70S6K, p70S6Kb, RSK1, RSK2, RSK3, RSK4, RSKL1, RSKL2, SgK494, SGK1, SGK2, SGK3, YANK1, YANK2, YANK3, ADCK3, ADCK4, ADCK1, ADCK2, ADCK5, AlphaK1, AlphaK2, AlphaK3, ChaK1, ChaK2, eEF2K, BAZ1A, BAZ1B, ABR, BCR, BLVRA, BRD2, BRD3, BRD4, BRDT, Col4A3BP, FASTK, G11, GTF2F1, BCKDK, PDHK1, PDHK2, PDHK3, PDHK4, ATM, ATR, DNAPK, FRAP, SMG1, TRRAP, RIOK1, RIOK2, RIOK3, TAF1, TAF1L, TIF1D, TIF1a, TIF1b, TIF1g, VACAMKL, CaMK1a, CaMK1b, CaMK1d, CaMK1g, CaMK4, CaMK2a, CaMK2b, CaMK2d, CaMK2g, AMPKa1, AMPKa2, BRSK1, BRSK2, CHK1, HUNK, LKB1, MARK1, MARK2, MARK3, MARK4, MELK, NIM1, NuaK1, NuaK2, PASK, QIK, QSK, SIK, SNRK, CASK, DAPK1, DAPK2, DAPK3, DRAK1, DRAK2, DCLK1, DCLK2, DCLK3, MAPKAPK2, MAPKAPK3, MAPKAPK5, MNK1, MNK2, SgK085, TTN, caMLCK, skMLCK, smMLCK, PHKg1, PHKg2, PIM1, PIM2, PIM3, PKD1, PKD2, PKD3, PSKH1, PSKH2, CHK2, STK33, SgK495, SSTK, TSSK1, TSSK2, TSSK3, TSSK4, Trb1, Trb2, Trb3, Obscn, SPEG, Trad, Trio, CK1a, CK1a2, CK1d, CK1e, CK1g1, CK1g2, CK1g3, TTBK1, TTBK2, VRK1, VRK2, VRK3, CCRK, CDC2, CDK10, CDK11a, CDK11b, CDK2, CDK3, CDK4, CDK6, CDK5, CDK7, CDK19, CDK8, CDK9, CHED, CRK7, PCTAIRE1, PCTAIRE2, PCTAIRE3, PFTAIRE1, PFTAIRE2, CDKL1, CDKL2, CDKL3, CDKL4, CDKL5, CK2a1, CK2a2, CLK1, CLK2, CLK3, CLK4, DYRK1A, DYRK1B, DYRK2, DYRK3, DYRK4, HIPK1, HIPK2, HIPK3, HIPK4, PRP4, GSK3A, GSK3B, Erk1, Erk2, Erk3, Erk4, Erk5, Erk7, JNK1, JNK2, JNK3, NLK, p38 *(e.g.,* p38a, p38b, p38d, or p38g), ICK, MAK, MOK, MSSK1, SRPK1, SRPK2, NME1A, NME1B, NME3, NME4, NME5, NME6, NME7a, TXNDC3, TXNDC6, AurA, AurB, AurC, BUB1, BUBR1, PRPK, CaMKK1, CaMKK2, CDC7, Dusty, Haspin, IKKa, IKKb, IKKe, TBK1, IRE1, IRE2, KIS, MOS, AAK1, BIKE, GAK, MPSK1, NEK1, NEK3, NEK5, NEK10, NEK11, NEK2, NEK4, NEK6, NEK7, NEK8, NEK9, SBK, SgK069, SgK110, PINK1, SgK223, SgK269, CLIK1, CLIK1L, SgK307, NRBP1, NRBP2, RNAseL, SgK196, SgK396, PAN3, GCN2, HRI, PEK, PKR, PLK1, PLK2, PLK3, PLK4, SCYL1, SCYL2, SCYL3, SgK071, SgK493, Slob, TBCK, TLK1, TLK2, PBK, TTK, Fused, ULK1, ULK2, ULK3, ULK4, PIK3R4, MYT1, Wee1, Wee1B, Wnk1, Wnk2, Wnk3, Wnk4, FAM198A, FAM198B, FAM20A, FAM20B, FAM20C, FJB1, ANPa, ANPb, CYGD, CYGF, HSER, COT, NIK, MAP3K5, MAP3K6, MAP3K7, MAP3K1, MEKK15, MAP3K2, MAP3K3, MAP3K4, OSR1, STLK3, GCK, HPK1, KHS1, KHS2, HGK, MINK, NRK, TNIK, MST1, MST2, MYO3A, MYO3B, PAK1, PAK2, PAK3, PAK4, PAK5, PAK6, LOK, SLK, STLK5, STLK6, TAO1, TAO2, TAO3, MST3, MST4, YSK1, MAP2K1, MAP2K2, MAP2K3, MAP2K6, MAP2K4, MAP2K5, MAP2K7, ALK, LTK, ABL1, ABL2, ACK, TNK1, AXL, MER, TYRO3, CCK4, CSK, CTK, DDR1, DDR2, EGFR, ErbB2, ErbB3, ErbB4, EphA1, EphA10, EphA2, EphA3, EphA4, EphA5, EphA6, EphA7, EphA8, EphB1, EphB2, EphB3, EphB4, EphB6, FAK, PYK2, FGFR1, FGFR2, FGFR3, FGFR4, FER, FES, IGF1R, INSR, IRR, JAK1, JAK2, JAK3, TYK2, LMR1, LMR2, LMR3, MET, RON, MUSK, FLT3, FMS, KIT, PDGFRa, PDGFRb, RET, ROR1, ROR2, RYK, ROS, FRK, BRK, SRM, FGR, FYN, SRC, YES, BLK, HCK, LCK, LYN, SYK, ZAP70, SuRTK106, BMX, BTK, ITK, TEC, TXK, TIE1, TIE2, TRKA, TRKB, TRKC, FLT1, FLT4, KDR, IRAK1, IRAK2, IRAK3, IRAK4, LIMK1, LIMK2, TESK1, TESK2, LRRK1, LRRK2, HH498, ILK, DLK, LZK, MLK1, MLK2, MLK3, MLK4, TAK1, ZAK, KSR1, KSR2, ARAF, BRAF, RAF1, ANKRD3, RIPK1, RIPK2, RIPK3, SgK288, ALK1, ALK2, ALK4, ALK7, BMPR1A, BMPR1B, TGFbR1, ACTR2, ACTR2B, BMPR2, MISR2, TGFbR2, MLKL. In some disclosures, a kinase to be inhibited can be one or more of: PKC, p38, MK2 or MK3.

In some disclosures, the agent is a kinase inhibitor (*e.g*., a kinase inhibitor that inhibits one or more of the kinases disclosed herein). In some disclosures, the agent includes two or more kinase inhibitors. In some disclosures, the agent includes a kinase inhibitor in combination with at least one other second agent (*e.g*., a second agent that increases the immune response of an immune cell). A variety of kinase inhibitors are known in the art. Non-limiting examples of kinase inhibitors include: MK-5108, palbociclib, capmatinib, rabusertib, SCH-900776, PF-477736, PF-477736, volitinib, crenolanib, pacritinib, adavosertib, afatinib, axitinib, bosutinib, cetuximab, cobimetinib, crizotinib, cabozantinib, dasatinib, entrectinib, erdafitinib, erlotinib, fostamatinib, gefitinib, ibrutinib, imatinib, lapatinib, lenvatinib, mubritinib, nilotinib, pazopanib, pegaptanib, ruxolitinib, sorafenib, sunitinib, SU6656, vandetanib, and vemurafenib. In some disclosures, a kinase inhibitor to be administered to a subject to treat a disease inhibits one or more of PKC, p38, MK2 or MK3. Non-limiting examples of kinase inhibitors that inhibit one or more of PKC, p38, MK2 and/or MK3 include: ruboxistaurin, chelerythrine, miyabenol C, myricitrin, gossypol, verbascoside, bryostatin 1, pamapimod, PH-797804, BIRB 796, VX-702, SB239063, SB202190, SB203580, SCIO 469, and BMS 582949. In some disclosures, a kinase inhibitor can be as described elsewhere (see, *e.g.,* Klaeger et al., 2017 Science 358:1148).

In some embodiments, administration to a subject of an agent that results in reduced phosphorylation of TSC2 polypeptides in immune cells is effective in the treatment of a disease (*e.g*., any of the variety of cancers, viral diseases, bacterial diseases, fungal diseases, or parasitic diseases disclosed herein). In some disclosures, administration to a subject of an agent that results in reduced phosphorylation of TSC2 polypeptides in immune cells results in increased mTORC1 signaling in the immune cells such that the immune cells exhibit increased immune activity as compared to a reference immune cell from a reference subject that has not been administered the agent. In some disclosures, administration of an agent to a subject that results in reduced phosphorylation of TSC2 polypeptides in immune cells results in reduced phosphorylation of TSC2 polypeptides (*e.g*., a population of TSC2 polypeptides in the cell in which the number of TSC2 polypeptides in the population that are phosphorylate is reduced as compared to a population of TSC2 polypeptides in a reference immune cell from a reference subject that has not been administered the agent) in one or more of CD4+ T cells, CD8+ T cells, Natural Killer cells (NK cells), macrophages, neutrophils, regulatory T cells (Tregs), helper T cells, or any other immune and inflammatory cells known in the art. In some disclosures, administration of an agent to a subject that results in reduced phosphorylation of TSC2 polypeptides in immune cells results in clonal expansion, enhanced synthesis and release of cytokines (*e.g*., cytokines known in the art to be associated with an increased immune response, including but not limited to TNFα, IFN-γ, IFN-α, IFN-β, TGF-β, IL-2, IL-3, IL-4, IL-5, IL-6, IL-10, IL-12, IL-13, and GM-CSF), and other downstream effects known to be associated with an increased immune response. A person of ordinary skill in the art will be aware of such downstream effects known to be associated with an increased immune response and will be able to determine whether such downstream effects are occurring or have occurred. In some disclosures, a clinical outcome can be determined in a subject that has been administered an agent that results in reduced phosphorylation of TSC2 polypeptides. Non-limiting examples of clinical outcomes include, increased survival (*e.g*., number of days, months, or years), increased progression-free survival (*e.g*., number of days, months, or years), increased overall response rate, decreased numbers of cancer cells, decreased tumor burden, and/or decreased numbers of pathogens (*e.g*., bacteria, viruses, fungi) as compared to a reference subject that has not been administered the agent.

An agent (*e.g*., a kinase inhibitor, one or more kinase inhibitors, or a kinase inhibitor in combination with a second agent) can be administered to a subject once or multiple times over a period of time ranging from days to weeks. In some cases, one or more agents can be formulated into a pharmaceutically acceptable composition for administration to a subject having a disease (*e.g*., cancer). For example, a therapeutically effective amount of an agent can be formulated together with one or more pharmaceutically acceptable carriers (additives) and/or diluents. A pharmaceutical composition can be formulated for administration in solid or liquid form including, without limitation, sterile solutions, suspensions, sustained-release formulations, tablets, capsules, pills, powders, and granules.

Pharmaceutically acceptable carriers, fillers, and vehicles that may be used in a pharmaceutical composition described herein include, without limitation, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

A pharmaceutical composition containing one or more agents can be designed for oral or parenteral (including subcutaneous, intramuscular, intravenous, and intradermal) administration. When being administered orally, a pharmaceutical composition can be in the form of a pill, tablet, or capsule. Compositions suitable for parenteral administration include aqueous and non-aqueous sterile injection solutions that can contain antioxidants, buffers, bacteriostats, and solutes that render the formulation isotonic with the blood of the intended recipient. The formulations can be presented in unit-dose or multidose containers, for example, sealed ampules and vials, and may be stored in a freeze dried (lyophilized) condition requiring only the addition of the sterile liquid carrier, for example, water for injections, immediately prior to use. Extemporaneous injection solutions and suspensions may be prepared from sterile powders, granules, and tablets.

In some cases, a pharmaceutically acceptable composition including one or more agents can be administered locally or systemically. For example, a composition provided herein can be administered locally by injection into tumors. In some cases, a composition provided herein can be administered systemically, orally, or by injection to a subject (*e.g*., a human).

Effective doses can vary depending on the severity of the disease, the route of administration, the age and general health condition of the subject, excipient usage, the possibility of co-usage with other therapeutic treatments such as use of other agents, and the judgment of the treating physician.

When referring to cancer, an effective amount of a composition containing one or more agents can be any amount that reduces the number of cancer cells present within the subject without producing significant toxicity to the subject. For example, an effective amount of dosage of an agent can be in the range of from about 0.1 mg/kg to about 100 mg/kg of body weight/day, for example, from about 1.0 mg/kg to about 50 mg/kg of body weight/day. In some embodiments, the dosage of an agent is in the range of from about 0.1 mg/kg to about 1.0 mg/kg of body weight/day; from about 0.1 mg/kg to about 5 mg/kg of body weight/day; from about 0.1 mg/kg to about 10 mg/kg of body weight/day; from about 0.1 mg/kg to about 25 mg/kg of body weight/day; from about 0.1 mg/kg to about 50 mg/kg of body weight/day; from about 1.0 mg/kg to about 5.0 mg/kg of body weight/day; from about 1.0 mg/kg to about 10 mg/kg of body weight/day; from about 1.0 mg/kg to about 20 mg/kg of body weight/day; from about 1.0 mg/kg to about 25 mg/kg of body weight/day; from about 1.0 mg/kg to about 40 mg/kg of body weight/day; from about 1.0 mg/kg to about 100 mg/kg of body weight/day; from about 10 mg/kg to about 100 mg/kg of body weight/day; from about 25 mg/kg to about 100 mg/kg of body weight/day; from about 50 mg/kg to about 100 mg/kg of body weight/day; from about 5.0 mg/kg to about 50 mg/kg of body weight/day; from about 10 mg/kg to about 50 mg/kg of body weight/day; or from about 25 mg/kg to about 50 mg/kg of body weight/day.

If a particular subject fails to respond to a particular amount, then the amount of an agent can be increased by, for example, two fold. After receiving this higher amount, the subject can be monitored for both responsiveness to the treatment and toxicity symptoms, and adjustments made accordingly. The effective amount can remain constant or can be adjusted as a sliding scale or variable dose depending on the subject's response to treatment. Various factors can influence the actual effective amount used for a particular application. For example, the frequency of administration, duration of treatment, use of multiple treatment agents, route of administration, and severity of the condition (*e.g*., cancer) may require an increase or decrease in the actual effective amount administered.

The frequency of administration of an agent can be any amount that reduces the number of cancer cells present within the subject without producing significant toxicity to the subject. For example, the frequency of administration of an agent can be from about two to about three times a week to about two to about three times a month. The frequency of administration of an agent can remain constant or can be variable during the duration of treatment. A course of treatment with a composition containing an agent can include rest periods. For example, a composition containing one or more agents can be administered daily over a two week period followed by a two week rest period, and such a regimen can be repeated multiple times. As with the effective amount, various factors can influence the actual frequency of administration used for a particular application. For example, the effective amount, duration of treatment, use of multiple treatment agents, route of administration, and severity of the condition (*e.g*., cancer) may require an increase or decrease in administration frequency.

An effective duration for administering a composition containing one or more agents can be any duration that reduces the severity of the condition (*e.g*., the number of cancer cells present within the subject) without producing significant toxicity to the subject. In some cases, the effective duration can vary from several days to several weeks. In general, the effective duration can range in duration from about one week to about four weeks. Multiple factors can influence the actual effective duration used for a particular treatment. For example, an effective duration can vary with the frequency of administration, effective amount, use of multiple treatment agents, route of administration, and severity of the condition being treated.

In some embodiments, an agent can be contacted with an immune cell *in vitro* for any of a variety of purposes including, without limitation, testing specific cell types, testing the effective, maximum, and/or minimum dosage of the agent, and/or testing duration of contact of the agent. In some embodiments, a test agent can be contacted with an immune cell *in vitro* to determine whether it is effective. Any suitable immune cell can be contacted *in vitro* with the agent including, without limitation, CD4+ T cells, CD8+ T cells, Natural Killer cells (NK cells), macrophages, neutrophils, regulatory T cells (Tregs), helper T cells, or any other immune and inflammatory cells known in the art. In some embodiments, the *in vitro* agent is a kinase inhibitor. In some embodiments, the *in vitro* agent includes two or more kinase inhibitors. In some embodiments, the *in vitro* agent includes a kinase inhibitor in combination with at least one other second agent (*e.g*., a second agent that increases the immune response of an immune cell). Effectiveness of the *in vitro* agent(s) on stimulating the immune cell can be assessed by any of a variety of techniques known in the art. In some embodiments, clonal expansion is assessed. In some embodiments, enhanced synthesis and release of cytokines (*e.g*., cytokines known in the art to be associated with an increased immune response, including but not limited to TNFα, IFN-γ, TGF-β, IL-4, IL-10, IL-13) is assessed. Those of ordinary skill in the art will be aware of and will be able to employ other suitable assessment methods for determining the effectiveness of an agent(s) on an immune cell *in vitro.*

### Engineered TSC2 Polypeptides that are Pseudophosphorylated

Disclosed herein are also engineered TSC2 polypeptides that act as if they are constitutively phosphorylated. Such engineered TSC2 polypeptides can be considered to be "pseudo-phosphorylated". In some disclosures, provided herein are pseudophosphorylated TSC2 polypeptides having an amino acid substitution at a residue corresponding to the serine residue at position S1365 of the human TSC2 polypeptide sequence (SEQ ID NO: 5). In some disclosures, provided herein are pseudophosphorylated TSC2 polypeptides having an amino acid substitution at a residue corresponding to the serine residue at position S1364 and/or S1365 of the human TSC2 polypeptide sequence (SEQ ID NO: 5). In some disclosures, the serine residue at position S1365 of SEQ ID NO: 5, or a serine residue in a polypeptide that corresponds to the serine residue at position S1365 of SEQ ID NO: 5, is substituted with an aspartic acid or a glutamic acid residue. In some disclosures, the serine residue at position S1364 and/or S1365 of SEQ ID NO: 5, or a serine residue in a polypeptide that corresponds to the serine residue at position S1364 and/or S1365 of SEQ ID NO: 5, is substituted with an aspartic acid or a glutamic acid residue. In one aspect of the present invention, a pseudophosphorylated TSC2 polypeptide having a glutamic acid substitution at the serine residue at position S1365 of the human TSC2 polypeptide sequence is provided, wherein the polypeptide sequence comprises SEQ ID NO: 2. In another aspect of the present invention, a pseudo-phosphorylated TSC2 polypeptide having glutamic acid substitutions at the serine residue positions S1364 and S1365 of the human TSC2 polypeptide sequence is provided, wherein the polypeptide sequence comprises SEQ ID NO: 21.

Disclosed herein are also pseudo-phosphorylated TSC2 polypeptides having an amino acid substitution at a residue corresponding to the serine residue at position S1366, or at both S1365 and S1366, of the mouse TSC2 polypeptide sequence (SEQ ID NO: 6) such that engineered TSC2 polypeptides act as if they are constitutively phosphorylated. In some disclosures, the serine residue at position S1366 of SEQ ID NO: 6, or a serine residue in a polypeptide that corresponds to the serine residue at position S1366 of SEQ ID NO: 6, is substituted with an aspartic acid or a glutamic acid residue. In some disclosures, the serine residue at position S1365 and/or S1366 of SEQ ID NO: 6, or a serine residue in a polypeptide that corresponds to the serine residue at position S1365 and/or S1366 of SEQ ID NO: 6, is substituted with an aspartic acid or a glutamic acid residue. In some disclosures, a pseudo-phosphorylated TSC2 polypeptide having an amino acid substitution at the serine residue at position S1366 of the mouse TSC2 polypeptide sequence is provided (e.g., SEQ ID NO: 10). In some disclosures, a pseudophosphorylated TSC2 polypeptide having amino acid substitutions at the serine residue positions S1365 and S1366 of the mouse TSC2 polypeptide sequence is provided (*e.g*., SEQ ID NO: 25).

Disclosed herein are also pseudo-phosphorylated TSC2 polypeptides having an amino acid substitution at a residue corresponding to the serine residue at position S1367, or at both S1366 and S1367, of the rat TSC2 polypeptide sequence (SEQ ID NO: 7) such that engineered TSC2 polypeptides act as if they are constitutively phosphorylated. In some disclosures, the serine residue at position S1367 of SEQ ID NO: 7, or a serine residue in a polypeptide that corresponds to the serine residue at position S1367 of SEQ ID NO: 7, is substituted with an aspartic acid or a glutamic acid residue. In some disclosures, the serine residue at position S1366 and/or S1367 of SEQ ID NO: 7, or a serine residue in a polypeptide that corresponds to the serine residue at position S1366 and/or S1367 of SEQ ID NO: 7, is substituted with an aspartic acid or a glutamic acid residue. In some disclosures, a pseudo-phosphorylated TSC2 polypeptide having an amino acid substitution at the serine residue at position S1367 of the rat TSC2 polypeptide sequence is provided (*e.g.,* SEQ ID NO: 11). In some disclosures, a pseudophosphorylated TSC2 polypeptide having amino acid substitutions at the serine residue at positions S1366 and S1367 of the rat TSC2 polypeptide sequence is provided (*e.g.,* SEQ ID NO: 27).

In some disclosures, pseudo-phosphorylated TSC2 polypeptides exhibit increased activity in their ability to down-regulate the mTORC1 pathway as compared to wild-type TSC2 polypeptides that are not phosphorylated at the engineered amino acid position(s).

Disclosed herein are also vectors that include nucleic acid sequences that encode polypeptides that act as if they are constitutively phosphorylated at a residue corresponding to S1364 and/or S1365 of the human TSC2 polypeptide sequence (SEQ ID NO: 5), S1365 and/or S1366 of the mouse TSC2 polypeptide sequence (SEQ ID NO: 6), or S1366 and/or S1367 of the rat TSC2 polypeptides sequence (SEQ ID NO: 7), and cells having such vectors. A vector that includes nucleic acid sequences that encode polypeptides that act as if they are constitutively phosphorylated at a residue corresponding to S1364 and/or S1365 of the human TSC2 polypeptide sequence (SEQ ID NO: 5), S1365 and/or S1366 of the mouse TSC2 polypeptide sequence (SEQ ID NO: 6), or S1366 and/or S1367 of the rat TSC2 polypeptides sequence (SEQ ID NO: 7) can be any appropriate type of vector. The present invention provides a vector comprising a nucleic acid sequence encoding a polypeptide comprising any one of SEQ ID NO: 1, SEQ ID NO: 20, SEQ ID NO: 2, and SEQ ID NO: 21. Examples of vectors include, without limitation, plasmids (*e.g*., expression plasmids) and vectors (*e.g*., viral vectors such as lentiviral vectors, retroviral vectors, adenovirus vectors, and adeno-associated virus vectors). In some cases, a vector that includes nucleic acid sequences that encode polypeptides that act as if they are constitutively phosphorylated at a residue corresponding to S1364 and/or S1365 of the human TSC2 polypeptide sequence (SEQ ID NO: 5), S1365 and/or S1366 of the mouse TSC2 polypeptide sequence (SEQ ID NO: 6), or S1366 and/or S1367 of the rat TSC2 polypeptides sequence (SEQ ID NO: 7) can be a lentiviral vector. A vector that includes nucleic acid sequences that encode polypeptides that act as if they are constitutively phosphorylated at a residue corresponding to S1364 and/or S1365 of the human TSC2 polypeptide sequence (SEQ ID NO: 5), S1365 and/or S1366 of the mouse TSC2 polypeptide sequence (SEQ ID NO: 6), or S1366 and/or S1367 of the rat TSC2 polypeptides sequence (SEQ ID NO: 7) also can include one or more addition features (*e.g*., one or more additional features to modulate polypeptide expression). Examples of features that can modulate polypeptide expression include, without limitation, an origin of replication, a promoter, a polyA tail, a terminator, and a microRNA response element. In some cases, when a vector described herein also includes a promoter, the promoter can operably linked to a nucleic acid sequence that encodes polypeptides that act as if they are constitutively phosphorylated at a residue corresponding to S1364 and/or S1365 of the human TSC2 polypeptide sequence (SEQ ID NO: 5), S1365 and/or S1366 of the mouse TSC2 polypeptide sequence (SEQ ID NO: 6), or S1366 and/or S1367 of the rat TSC2 polypeptides sequence (SEQ ID NO: 7) (*e.g.,* such that the promoter can drive expression of the nucleic acid sequence). A promoter can be any appropriate promoter. In some cases, a promoter can be a constitutive promoter. In some cases, a promoter can be a viral promoter. In some cases, a promoter can be an inducible promoter. In some cases, a promoter can be a cell-specific and/or tissue-specific promoter. Examples of promoters that can be used to drive expression of nucleic acid sequences that encode polypeptides that act as if they are constitutively phosphorylated at a residue corresponding to S1364 and/or S1365 of the human TSC2 polypeptide sequence (SEQ ID NO: 5), S1365 and/or S1366 of the mouse TSC2 polypeptide sequence (SEQ ID NO: 6), or S1366 and/or S1367 of the rat TSC2 polypeptides sequence (SEQ ID NO: 7) include, without limitation, CMV In some embodiments, a promoter can be as described elsewhere (see, *e.g.,* Morgan et al., 2016 Biomedicines. 4:9).

In some embodiments, cells having vectors that include nucleic acid sequences that encode polypeptides that act as if they are constitutively phosphorylated at a serine residue in any of the polypeptides described herein do not express endogenous, wild-type TSC2. For example, the nucleic acid sequence encoding wild-type TSC2 can be modified by any of a variety of genetic manipulation techniques known in the art including, but not limited to, CRISPR-based methods, TALEN-based methods, and other genetic targeting or recombination methods (see, *e.g.,* Roth et al., 2018 Nature 559:405-409). In some embodiments, cells having vectors that include nucleic acid sequences that encode engineered pseudo-phosphorylated TSC2 polypeptides that act as if they are constitutively phosphorylated at a position that corresponds to a wild-type serine residue in any of the polypeptides described herein do express endogenous, wild-type TSC2. In some embodiments of cells having both: 1) vectors that include nucleic acid sequences that encode engineered TSC2 polypeptides that act as if they are constitutively phosphorylated, and 2) a nucleic acid sequence encoding an endogenous TSC2 protein, the cells exhibit the same or similar activity as a corresponding cell lacking the nucleic acid sequence encoding the endogenous TSC2 protein.

TSC2 polypeptides disclosed herein can be engineered at S1365 of the human TSC2 polypeptide sequence (SEQ ID NO: 5), S1366 of the mouse TSC2 polypeptide sequence (SEQ ID NO: 6), or S1367 of the rat TSC2 polypeptide sequence (SEQ ID NO: 7) with any mutation or modification that results in TSC2 mimicking constitutive phosphorylation at the respective positions in human (S1365), mouse (S1366), or rat (S1367). Additionally, TSC2 polypeptides disclosed herein can be engineered at S1364 and S1365 of the human TSC2 polypeptide sequence (SEQ ID NO: 5), S1365 and S1366 of the mouse TSC2 polypeptide sequence (SEQ ID NO: 6), or S1366 and S1367 of the rat TSC2 polypeptide sequence (SEQ ID NO: 7) with any mutation or modification that results in TSC2 mimicking constitutive phosphorylation at the respective positions in human (S1364 and S1365), mouse (S1365 and S1366), or rat (S1366 and S1367). In some cases, TSC2 polypeptides from other species (*e.g*., monkey) can be engineered with any mutation or modification (*e.g*., a residue corresponding to human S1364 and/or S1365, mouse S1365 and/or S1366, and/or rat S1366 and/or S1367) that results in TSC2 mimicking constitutive phosphorylation.

Disclosed herein are also nucleic acids encoding engineered TSC2 polypeptides that act as if they are constitutively phosphorylated at a position corresponding to a serine residue in a wild-type TSC2 polypeptide sequence. In some embodiments, nucleic acids provided herein encode pseudo-phosphorylated TSC2 polypeptides that exhibit increased activity in their ability to down-regulate the mTORC1 pathway as compared to wild-type TSC2 polypeptides that are not phosphorylated at the engineered position. In some disclosures, provided herein are nucleic acids encoding pseudo-phosphorylated TSC2 polypeptides having an amino acid substitution at a residue corresponding to the serine residue at position S1365 of the human TSC2 polypeptide sequence (SEQ ID NO: 5), or position S1366 of the mouse TSC2 polypeptide sequence (SEQ ID NO: 6), or position S1367 of the rat TSC2 polypeptide sequence (SEQ ID NO: 7). In some disclosures, provided herein are nucleic acids encoding pseudo-phosphorylated TSC2 polypeptides having an amino acid substitution at a residue corresponding to the serine residue at positions S1364 and S1365 of the human TSC2 polypeptide sequence (SEQ ID NO: 5), or positions S1365 and S1366 of the mouse TSC2 polypeptide sequence (SEQ ID NO: 6), or positions S1366 and S1367 of the rat TSC2 polypeptide sequence (SEQ ID NO: 7). In some embodiments, nucleic acids provided herein encode pseudo-phosphorylated TSC2 polypeptides in which the serine residue at position S1365 of SEQ ID NO: 5, position S1366 of SEQ ID NO: 6, or position 1367 of SEQ ID NO: 7, or a serine residue in a TSC2 polypeptide that corresponds to the serine residues at those positions, is substituted with an aspartic acid or a glutamic acid residue. In some embodiments, nucleic acids provided herein encode pseudo-phosphorylated TSC2 polypeptides in which the serine residue at positions S1364 and/or S1365 of SEQ ID NO: 5, positions S1365 and/or S1366 of SEQ ID NO: 6, or positions S1366 and/or S1367 of SEQ ID NO: 7, or a serine residue in a TSC2 polypeptide that corresponds to the serine residues at those positions, is substituted with an aspartic acid or a glutamic acid residue. In some embodiments, nucleic acids provided herein include the genetic codons GAA or GAG at positions that are translated to a glutamic acid residue at positions S1364 and/or S1365 of the human TSC2 polypeptide sequence (SEQ ID NO: 5), positions S1365 and/or S1366 of the mouse TSC2 polypeptide sequence (SEQ ID NO: 6), or positions S1366 and/or S1367 of the rat TSC2 polypeptide sequence (SEQ ID NO: 7). In some embodiments, nucleic acids provided herein include the genetic codons GAT or GAC at positions that are translated to an aspartic acid residue at positions S1364 and/or S1365 of the human TSC2 polypeptide sequence (SEQ ID NO: 5), positions S1365 and/or S1366 of the mouse TSC2 polypeptide sequence (SEQ ID NO: 6), or positions S1366 and/or S1367 of the rat TSC2 polypeptide sequence (SEQ ID NO: 7). In some embodiments, a nucleic acid sequence encoding an engineered TSC2 polypeptide having a glutamic acid substitution at the serine residue at position S1365 of the human TSC2 polypeptide sequence is provided (*e.g*., a nucleic acid sequence of SEQ ID NO: 4). In some disclosures, a nucleic acid sequence encoding an engineered TSC2 polypeptide having glutamic acid substitutions at the serine residue positions S1364 and S1365 of the human TSC2 polypeptide sequence is provided (e.g., a nucleic acid sequence of SEQ ID NO: 23).

### Cells Having Engineered TSC2 Polypeptides that are Pseudophosphorylated for Use in Treating a Disease

### Cells having engineered TSC2 polypeptides that are Pseudo-phosphorylated

Provided herein are cells expressing engineered TSC2 polypeptides that act as if they are constitutively phosphorylated at a position corresponding to a serine residue in the wild-type TSC2 polypeptide sequence. In some disclosures, cells provided herein include pseudo-phosphorylated TSC2 polypeptides that exhibit increased activity in their ability to down-regulate the mTORC1 pathway as compared to wild-type TSC2 polypeptides that are not phosphorylated at the engineered position. In some embodiments, cells provided herein express pseudo-phosphorylated TSC2 polypeptides in which the serine residue at position S1365 of SEQ ID NO: 5, or a serine residue in a polypeptide that corresponds to the serine residue at position S1365 of SEQ ID NO: 5, is substituted with an aspartic acid or a glutamic acid residue. In some embodiments, cells provided herein express pseudo-phosphorylated TSC2 polypeptides in which the serine residue positions S1364 and S1365 of SEQ ID NO: 5, or a serine residue in a polypeptide that corresponds to the serine residue at positions S1364 and S1365 of SEQ ID NO: 5, are substituted with an aspartic acid or a glutamic acid residue. In one aspect of the present invention are cells expressing pseudo-phosphorylated TSC2 polypeptides having a glutamic acid substitution at a residue corresponding to the serine residue at position S1365 of the human TSC2 polypeptide sequence, such as comprising SEQ ID NO: 2. In another aspect of the present invention, cells provided herein express an engineered TSC2 polypeptide having glutamic acid substitutions at the serine residue positions S1364 and S1365 of the human TSC2 polypeptide sequence, such as comprising SEQ ID NO: 21.

Disclosed herein for reference are also cells expressing pseudo-phosphorylated TSC2 polypeptides having an amino acid substitution at a residue corresponding to the serine residue at position S1366, or both S1365 and S1366, of the mouse TSC2 polypeptide sequence (SEQ ID NO: 6) such that engineered TSC2 polypeptides act as if they are constitutively phosphorylated at the serine residue. In some disclosures, the cells provided herein express a pseudo-phosphorylated TSC2 polypeptide in which the serine residue at position S1366 of SEQ ID NO: 6, or a serine residue in a polypeptide that corresponds to the serine residue at position S1366 of SEQ ID NO: 6, is substituted with an aspartic acid or a glutamic acid residue. In some disclosures, the cells provided herein express a pseudo-phosphorylated TSC2 polypeptide in which the serine residue at positions S1365 and S1366 of SEQ ID NO: 6, or a serine residue in a polypeptide that corresponds to the serine residue at positions S1365 and S1366 of SEQ ID NO: 6, are substituted with an aspartic acid or a glutamic acid residue. In some disclosures, cells provided herein express a pseudo-phosphorylated TSC2 polypeptide having a glutamic acid substitution at the serine residue at position S1366 of the mouse TSC2 polypeptide sequence (*e.g*., SEQ ID NO: 10). In some disclosures, cells provided herein express an engineered TSC2 polypeptide having glutamic acid substitutions at the serine residue positions S1366 and S1366 of the mouse TSC2 polypeptide sequence (e.g., SEQ ID NO: 25).

Disclosed herein for reference are also cells expressing a pseudo-phosphorylated TSC2 polypeptide having an amino acid substitution at a residue corresponding to the serine residue at position S1367, or both S1366 and S1367, of the rat TSC2 polypeptide sequence (SEQ ID NO: 7) such that engineered TSC2 polypeptides act as if they are constitutively phosphorylated at the serine residue. In some disclosures, cells provided herein express a pseudo-phosphorylated TSC2 polypeptide in which the serine residue at position S1367 of SEQ ID NO: 7, or a serine residue in a polypeptide that corresponds to the serine residue at position S1367 of SEQ ID NO: 7, is substituted with a aspartic acid or a glutamic acid residue. In some disclosures, cells provided herein express a pseudophosphorylated TSC2 polypeptide in which the serine residue at positions S1366 and S1367 of SEQ ID NO: 7, or a serine residue in a polypeptide that corresponds to the serine residue at positions S1366 and S1367 of SEQ ID NO: 7, are substituted with a aspartic acid or a glutamic acid residue. In some disclosures, cells provided herein express an engineered TSC2 polypeptide having a glutamic acid substitution at the serine residue at position S1367 of the rat TSC2 polypeptide sequence (*e.g*., SEQ ID NO: 11). In some disclosures, cells provided herein express an engineered TSC2 polypeptide having glutamic acid substitutions at the serine residue positions S1366 and S1367 of the rat TSC2 polypeptide sequence (*e.g*., SEQ ID NO: 27).

Disclosed herein for reference are also cells harboring vectors that include nucleic acids that encode polypeptides that act as if they are constitutively phosphorylated at a serine residue corresponding to S1365, or both S1364 and S1365, of the human TSC2 polypeptide sequence (SEQ ID NO: 5), S1366, or both S1365 and S1366, of the mouse TSC2 polypeptide sequence (SEQ ID NO: 6), or S1367, or both S1366 and S1367, of the rat TSC2 polypeptides sequence (SEQ ID NO: 7). A vector that includes nucleic acid sequences that encode polypeptides that act as if they are constitutively phosphorylated at a residue corresponding to S1365, or both S1364 and S1365, of the human TSC2 polypeptide sequence (SEQ ID NO: 5), S1366, or both S1365 and S1366, of the mouse TSC2 polypeptide sequence (SEQ ID NO: 6), or S1367, or both S1366 and S1367, of the rat TSC2 polypeptides sequence (SEQ ID NO: 7) can be introduced into a cell using any appropriate methods and/or techniques. The present invention provides a vector comprising a nucleic acid sequence which encodes a polypeptide comprising any one of SEQ ID NO: 1, SEQ ID NO: 20, SEQ ID NO: 2, and SEQ ID NO: 21. A vector can be introduced into a cell in a transient manner (*e.g*., maintained as a vector) or in a stable manner (*e.g*., integrated into the genome). Examples of methods and/or techniques that can be used to introduce one or more vectors into a cell include, without limitation, transfection, transduction, electroporation, and infection. In some embodiments, nucleic acids encoding pseudo-phosphorylated TSC2 polypeptides are operably linked to nucleic acids that drive expression of pseudo-phosphorylated TSC2 polypeptides in the vectors (*e.g*., promoter sequences).

In some embodiments, cells having pseudo-phosphorylated TSC2 polypeptides that act as if they are constitutively phosphorylated can have, or can express, endogenous, TSC2 proteins. For example, a cell having a pseudo-phosphorylated TSC2 polypeptide also can have an endogenous wild-type nucleic acid sequence encoding a wild-type TSC2 polypeptide. In some embodiments of cells having (*e.g*., expressing) both: 1) engineered pseudo-phosphorylated TSC2 polypeptides that act as if they are constitutively phosphorylated, and 2) an endogenous TSC2 protein, the cells exhibit the same or similar activity as a corresponding cell lacking (*e.g*., not expressing) the endogenous TSC2 protein.

In some embodiments, cells having pseudo-phosphorylated TSC2 polypeptides that act as if they are constitutively phosphorylated do not have, or do not express, endogenous, wild-type TSC2 proteins. For example, a cell having a pseudophosphorylated TSC2 polypeptide can have a genetic alteration in which a wild-type nucleic acid sequence encoding the TSC2 polypeptide has been rendered inactive. In some embodiments, the nucleic acid sequence encoding wild-type TSC2 can be modified by any of a variety of genetic manipulation techniques known in the art including, but not limited to, CRISPR-based methods, TALEN-based methods, and other genetic targeting or recombination methods (see, *e.g.,* Roth et al., 2018 Nature 559:405-409). In some embodiments, a wild-type nucleic acid sequence encoding a TSC2 polypeptide can be rendered inactive by removing, replacing, or mutating a nucleic acid sequence that contributes to expression of the TSC2 polypeptide including, but not limited to, a promoter sequence, an enhancer sequence, a coding sequence of a transcription factor that regulates expression of TSC2, the coding sequence of the TSC2 polypeptide itself, or combinations thereof. In some embodiments, a wild-type nucleic acid sequence encoding a TSC2 polypeptide can be rendered inactive via a frameshift caused by one or more modifications or mutations in the nucleic acid sequence encoding the TSC2 polypeptide.

In some embodiments, cells that can be engineered to include a pseudophosphorylated TSC2 polypeptide include immune cells. For example, the immune cells can be CD4+ T cells, CD8+ T cells, Natural Killer cells (NK cells), macrophages, neutrophils, regulatory T cells (Tregs), helper T cells, B cells, or any other immune cells and/or inflammatory cells known in the art. Relevant aspects of certain of these cells are disclosed elsewhere herein.

In some embodiments, an immune cell that is engineered to include an engineered TSC2 polypeptide can be a cytotoxic T cell. In some embodiments, a cytotoxic T cell can be engineered to express a CAR or a TCR.

In some embodiments, an immune cell that is engineered to include an engineered TSC2 polypeptide can be expanded (*e.g*., clonally expanded). For example, an immune cell that is engineered to include an engineered TSC2 polypeptide can be clonally expanded ex vivo *(e.g.,* for use in an adoptive cell therapy). In cases where an immune cell that is engineered to include an engineered TSC2 polypeptide is used in an adoptive cell therapy, the adoptive cell therapy can be any appropriate adoptive cell therapy. Examples of adoptive cell therapies include, without limitation, dendritic cell therapy and synthetic dendritic cell therapy. In some cases, adoptive cell therapy can include the extraction of tumor infiltrating lymphocytes.

In some embodiments, decreased mTORC1 signaling can be determined by any of a variety of techniques or methods known in the art. For example, inhibited mTORC1 signaling typically results in decreased growth, increased autophagy, and less phosphorylation of Ulk1 (Unc-51-like kinase-1), p70S6K (ribosomal protein S6 kinase), and 4EBP1 (elF4E binding protein-1).

### Cancers

Compositions and methods disclosed herein can be used to treat cancer. For example, an immune cell that is engineered to include an engineered TSC2 polypeptide (*e.g*., expressed from a vector introduced into the cell, which vector includes a nucleic acid sequence that encodes the engineered TSC2 polypeptide) that acts as if it is constitutively phosphorylated at a residue corresponding to S1365, or both S1364 and S1365, of the human TSC2 polypeptide sequence (SEQ ID NO: 5), S1366, or both S1365 and S1366, of the mouse TSC2 polypeptide sequence (SEQ ID NO: 6), or S1367, or both S1366 and S1367, of the rat TSC2 polypeptides sequence (SEQ ID NO: 7) can be administered to a subject having cancer (*e.g*., in an adoptive cell therapy) such that the cancer is treated. In some embodiments, the engineered immune cell administered to the subject does not have or express an endogenous, wild type TSC polypeptide. In some embodiments, the engineered immune cell administered to the subject does have or express an endogenous, wild type TSC2 polypeptide. In some embodiments, a CD8+ T effector cell that recognizes a cancer cell (*e.g*., via a specific antigen on the cancer cell surface) can be engineered to include a TSC2 polypeptide that acts as if it is constitutively non-phosphorylated, which CD8+ T effector cell is then administered to a subject that has such a cancer cell. This enhances mTORC1 activation with stimulation to increase effector function. In some embodiments, a CD8+ T effector cell that is engineered to include a TSC2 polypeptide acts as if it is constitutively non-phosphorylated is also engineered to express a chimeric antigen receptor or a T cell receptor. In some embodiments, an immune cell that is engineered to include an engineered TSC2 polypeptide that acts as if it is constitutively phosphorylated is more effective in treating cancer in a subject than an immune cell that lacks the engineered TSC2 polypeptide.

Cancer types that can be treated include, without limitation, lung cancer (*e.g*., small cell lung carcinoma or non-small cell lung carcinoma), papillary thyroid cancer, medullary thyroid cancer, differentiated thyroid cancer, recurrent thyroid cancer, refractory differentiated thyroid cancer, lung adenocarcinoma, bronchioles lung cell carcinoma, MEN2A, MEN2B, pheochromocytoma, parathyroid hyperplasia, breast cancer, colorectal cancer (*e.g*., metastatic colorectal cancer), papillary renal cell carcinoma, ganglioneuromatosis of the gastroenteric mucosa, inflammatory myofibroblastic tumor, or cervical cancer, ALL, AML, cancer in adolescents, adrenal cancer, adrenocortical carcinoma, anal cancer, appendix cancer, astrocytoma, atypical teratoid/rhabdoid tumor, basal cell carcinoma, bile duct cancer, bladder cancer, bone cancer, brain stem glioma, brain tumor, breast cancer, bronchial tumor, Burkitt lymphoma, carcinoid tumor, unknown primary carcinoma, cardiac tumors, cervical cancer, childhood cancers, chordoma, CLL, CML, chronic myeloproliferative neoplasms, colon cancer, colorectal cancer, craniopharyngioma, cutaneous T-cell lymphoma, bile duct cancer, ductal carcinoma in situ, embryonal tumors, endometrial cancer, ependymoma, esophageal cancer, esthesioneuroblastoma, Ewing sarcoma, extracranial germ cell tumor, extragonadal germ cell tumor, extrahepatic bile duct cancer, eye cancer, fallopian tube cancer, fibrous histiocytoma of bone, gallbladder cancer, gastric cancer, gastrointestinal carcinoid tumor, GIST, germ cell tumor, gestational trophoblastic disease, glioma, hairy cell tumor, hairy cell leukemia, head and neck cancer, heart cancer, hepatocellular cancer, histiocytosis, Hodgkin's lymphoma, hypopharyngeal cancer, intraocular melanoma, islet cell tumors, pancreatic neuroendocrine tumors, Kaposi sarcoma, kidney cancer, Langerhans cell histiocytosis, laryngeal cancer, leukemia, lip and oral cavity cancer, liver cancer, lung cancer, lymphoma, macroglobulinemia, malignant fibrous histiocytoma of bone, osteocarcinoma, melanoma, Merkel cell carcinoma, mesothelioma, metastatic squamous neck cancer, midline tract carcinoma, mouth cancer, multiple endocrine neoplasia syndromes, multiple myeloma, mycosis fungoides, myelodysplastic syndromes, myelodysplastic/myeloproliferative neoplasms, myelogenous leukemia, myeloid leukemia, multiple myeloma, myeloproliferative neoplasms, nasal cavity and paranasal sinus cancer, nasopharyngeal cancer, neuroblastoma, non-Hodgkin's lymphoma, non-small cell lung cancer, oral cancer, oral cavity cancer, lip cancer, oropharyngeal cancer, osteosarcoma, ovarian cancer, pancreatic cancer, papillomatosis, paraganglioma, paranasal sinus and nasal cavity cancer, parathyroid cancer, penile cancer, pharyngeal cancer, pheochromosytoma, pituitary cancer, plasma cell neoplasm, pleuropulmonary blastoma, pregnancy and breast cancer, primary central nervous system lymphoma, primary peritoneal cancer, prostate cancer, rectal cancer, renal cell cancer, retinoblastoma, rhabdomyosarcoma, salivary gland cancer, sarcoma, Sezary syndrome, skin cancer, small cell lung cancer, small intestine cancer, soft tissue sarcoma, squamous cell carcinoma, squamous neck cancer, stomach cancer, T-cell lymphoma, testicular cancer, throat cancer, thymoma and thymic carcinoma, thyroid cancer, transitional cell cancer of the renal pelvis and ureter, unknown primary carcinoma, urethral cancer, uterine cancer, uterine sarcoma, vaginal cancer, vulvar cancer, Waldenstrom Macroglobulinemia, and Wilms' tumor.

### Other diseases

In some embodiments, the compositions and methods disclosed herein can be are useful for other situations and/or can be used to treat other diseases. Examples of other situations where the compositions and methods provided herein can be useful include, without limitation, situations of tissue, skin, and organ transplantation. Examples of other diseases that the compositions and methods can be used to treat include, without limitation, graft-versus-host disease (GVHD), allergies, asthma, autoimmune diseases (such as systemic lupus erythematosus and rheumatoid arthritis), multiple sclerosis, and inflammatory bowel disease. In some embodiments, cells comprising a pseudophosphorylated TSC2 polypeptide can be administered to patients in need (*e.g*., in an adoptive cell therapy) resulting in decreased mTORC1 activities and thus treating diseases including situations of tissue, skin and organ transplantation, in GVHD, or allergies, or in autoimmune diseases such as systemic lupus erythematosus and multiple sclerosis.

### Methods of Generating a Persistent T Cell in a Subject

Also disclosed herein are methods of generating a persistent T cell a subject. In some disclosures, methods of generating a persistent T cell in a subject include administering to a subject (*e.g*., in an adoptive cell therapy) an engineered immune cell comprising a vector. In some embodiments, the vector comprises a nucleic acid encoding a polypeptide comprising SEQ ID NO: 2 that is operably linked to a nucleic acid that drives expression of the polypeptide in the engineered immune cell. In some embodiments, the engineered immune cell recognizes an antigen. In some embodiments, upon recognizing the antigen, the engineered immune cell exhibits decreased effector cell activity as compared to a reference T cell that lacks the vector. In some embodiments, upon recognizing the antigen, the engineered immune cell exhibits long-term persistence and memory activity as compared to a reference T cell that lacks the vector. In some embodiments, upon recognizing the antigen, the engineered immune cell becomes a persistent T cell.

In some embodiments, the decreased effector activity of the engineered immune cell can include a decline in mTORC1 signaling.

In some embodiments, the engineered immune cell is derived from an endogenous immune cell obtained from the subject. As used herein, the phrase "derived from" means that the endogenous immune cell is obtained from the subject, after which it is modified (e.g., via introduction of a vector having an nucleic acid sequence encoding a modified TSC2 polypeptide as described herein) to generate the engineered immune cell.

In some embodiments, the engineered immune cell is a CD8+ T cell. In some embodiments, the persistent T cell is a memory T cell. In some embodiments, the CD8+ T cell is further engineered to express a CAR or a TCR. In some embodiments, the engineered immune cell is a regulatory T cell. In some embodiments, the persistent T cell is a persistent T regulatory cell.

### Methods of Generating a Persistent T Cell in vitro

In an aspect of the present invention are methods of generating a persistent T cell *in vitro* comprising:
introducing into an immune cell a nucleic acid encoding a polypeptide comprising SEQ ID NO: 2 or SEQ ID NO: 21 operably linked to a promoter that drives expression of the polypeptide in the immune cell, thereby generating an engineered immune cell;
wherein the engineered immune cell exhibits decreased mTORC1 signaling as compared to a reference immune cell that lacks the vector;
contacting the engineered immune cell with an antigen that is recognized by the engineered immune cell; and
culturing the engineered immune cell under conditions and for a time sufficient such that the engineered immune cell becomes the persistent T cell.

In some embodiments, the engineered immune cell exhibits decreased mTORC1 signaling as compared to a reference immune cell that does not comprise the vector. In some embodiments, the engineered immune cell expresses one or more polypeptides that contain a targeted pseudophosphorylation modification (e.g., that can result in decreased mTORC1 signaling) as compared to a reference immune cell that does not comprise the vector.

In some embodiments, the immune cell is a CD8+ T cell, and the generated persistent T cell is a memory T cell. In some embodiments, the CD8+ T cell is further engineered to express a chimeric antigen receptor or a T cell receptor.

In some embodiments, a persistent T cell generated *in vitro* is for use in treatinga disease. In some embodiments, the disease is cancer, a viral disease, a bacterial disease, fungal disease, or a parasitic disease (*e.g*., any of the cancers, viral diseases, bacterial diseases, fungal diseases, or parasitic diseases disclosed herein).

The immune cell may be obtained from the subject to be treated (*e.g*., an autologous cell). The immune cell may be obtained from a subject other than the subject to be treated (*e.g*., an allogenic cell). In some embodiments, the immune cell is a regulatory T cell, and the persistent T cell is a persistent T regulatory cell. In some embodiments, the disease is asthma, an autoimmune disease, or graft vs. host disease.

The invention will be further described in the following examples, which do not limit the scope of the invention described in the claims.

### EXAMPLES

### Example 1: Substitution of murine TSC2 S1366 (human TSC2 S1365) modulates mTORC1 signaling

### Materials and Methods

### Neonatal rat cardiomyocyte studies (NRCMs)

NRCMs are freshly isolated from pregnant female rates, cells isolated and cultured for 24 hours in DMEM with 10% FBS and antibiotics prior to study, as described elsewhere (see, *e.g.,* Lee et al., 2015 Nature 519:472-476). Cells were also transfected with plasmids expressing human TSC2 polypeptides: TSC2-WT, TSC2-S1364A, TSC2-S1364E, TSC2-S1365A, of TSC2-S1365E performed with Takara Clontech Xfect reagent per manufacturer protocol. After 24-36 hours, cells were stimulated with endothelin 1 (ET1, 10 nM, Sigma) or vehicle for 15 minutes or for 48 hours, in serum-free DMEM supplemented with 0.1% Insulin-Transferrin-Selenium (Life Technologies).

### Gene expression - qRT-PCR

Total RNA was isolated from left ventricular myocardium or cultured NRCMs using Trizol Reagent (Invitrogen), followed by reverse transcription to cDNA using a High Capacity RNA-to-cDNA Kit (Applied Biosystems, Life Technologies). cDNA underwent PCR amplification using TaqMan probes for brain or B-type natriuretic peptide (BNP) (mouse # Mm01255770_g1, rat #Rn00580641_m1), and glyceraldehyde-3-phosphate dehydrogenase (GAPDH) (mouse #99999915_g1, rat #Rn01775763_g1) (Applied Biosystems). The threshold cycle value was determined using the crossing point method. Samples were normalized to the GAPDH value for each run.

### Protein analysis

Whole cell lysate was extracted (Cell Signaling Technology #9803) and protein concentration determined by BCA method (Pierce). Samples were prepared in SDS Tris-Glycine buffer (Life Technologies) and run on Novex 8-16% Tris-Glycine Gels (Life Technologies) or TGX 7.5% and 4-20% Tris-Glycine Gels (Bio-Rad) and blotted onto a nitrocellulose membrane. The following primary antibodies were used in this study: phospho-70 S6K (T389) #9205 lot 21 used at 1:1,000, p70 S6K #9202 lot 20 used at 1:1,000, phospho-4EBP1 (S65) #9451 lot 14 used at 1:1,000, 4EBP1 #9452 lot 12 used at 1:1,000, GAPDH #2118 clone 14C10 lot 10 used at 1:1,000, TSC2 #3612 clone D93F12 lot 5 used at 1:1,000, and α-tubulin #3873 clone DM1A lot 12 used at 1:1,000 (Cell Signaling Technology), phospho-TSC2 (S1365) #120718 lotNFSA12072OAH used at 1:500 (NovoPro Labs), LC3 #ab192890 lot GR321-3 used at 1:1,000. Antibody binding was visualized by infrared imaging (Odyssey, Licor) and quantified with Licor Image Studio Software 3.1.

### Tandem fluorescent LC3 probe analysis

NRCMs were infected with an adenovirus (10 MOI) expressing a tandem fluorescent (GFP-RFP) tagged LC3¹⁵. This expresses LC3 with both green and red fluorescence as the autophagosomal membrane is forming; but upon merging with the acidic lysosome (autolysosome), the GFP signal is quenched, leaving RFP. The rise in RFP provides a marker of autophagic flux. In some studies, myocytes were further transfected with plasmid encoding for WT-TSC2, or SA or SE mutant TSC2, and further stimulated for 48 hours with endothelin 1 (10 nM). Dot counts for both colors/cell were determined using Image J software (Ver 1.52a, NIH).

### In vitro protein kinase G activity

PKG activity was assessed by in vitro colorimetric assay (Cyclex, Cat #CY-1161, Nagano, Japan) following the manufacturer's instructions. The assay provides cGMP substrate, and a kinase-specific peptide-target to assess phosphorylation activity.

### Results

To examine whether PKG activates autophagic flux in isolated cardiac myocytes, NRVMs were infected with an adenovirus (10 MOI) expressing a tandem fluorescent (GFP-RFP) tagged LC315 (LC3-GFP-RFP). This adenovirus expresses LC3 with both green and red fluorescence as the autophagosomal membrane is forming; but upon merging with the acidic lysosome (autolysosome), the GFP signal is quenched, leaving RFP. The rise in RFP provides a marker of autophagic flux. NRVMs were then stimulated with endothelin-1 (ET1), and in turn treated with either a protein kinase G activator (cGMP) or inhibitor (DT3). ET1 stimulates myocyte growth (hypertrophy) and this is markedly reduced by protein kinase G stimulation. These cells display an increase in red dots indicating enhanced autophagy. By contrast, inhibiting PKG increases cell size and markedly reduces autophagy as reflected by the fall in both green and red dots (Figure 1).

A phospho-site map for TSC2, with numbering based on human sequence, is shown in Figure 2. A serine duplet is present in TSC2 numbered as residues S1364 and S1365 in the human TSC2 sequence set forth in SEQ ID NO: 5. These sites are all highly homologous between human and other mammalian species, though the numbering is slightly different for the T1271 AMPK site (mouse it is T1270), and for all sites identified at and above S1364 (mouse number would be one higher for each). For example, residues S1364 and S1365 in the human TSC2 sequence set forth in SEQ ID NO: 5 would be numbered a S1365 and S1366 in the mouse TSC2 sequence set forth in SEQ ID NO: 6.

The activity of TSC2 when modifying either the first serine in the doublet (hsS1364, mmS1364) or the second serine in the doublet (hsS1365, mmS1366) was compared. Myocytes were transfected with plasmid encoding human WT TSC2, huS1364A TSC2, hsS1364E TSC2, hsS1365A TSC2, or hsS1365E TSC2.

To evaluate phosphorylation of TSC2, myocytes expressing either WT TSC2 or mutated TSC2 were exposed to ET1 (10 nM) for 48 hours, stimulating phosphorylation of TSC2 as detected by the antibody on when wild-type TSC2 protein was expressed. Control cells were stimulated with vehicle for 48 hours. The cell lysates were probed for phosphorylated TSC2 signal (Figure 3). Phosphorylation was observed when WT protein is expressed, but is equally blunted by site mutagenesis at either the first serine (hsS1364) or second serine (hsS1365).

To evaluate PKG activation, mRNA expression of a pathological hypertrophy gene marker (*Nppb*) was measured. Myocytes expressing either WT TSC2 or mutated TSC2 were exposed to ET1 for 48 hours to induce hypertrophy and cell lysates were examined for *Nppb* mRNA (Figure 4). Activation of PKG by sildenafil (Sil) reduces Nppb increase in WT expressing cells, but not those with SA or SE. SE expression depresses *Nppb* rise, whereas SA expression enhances it. This demonstrated that functionally, modifying either the first or second serine of the duplet at hsTSC21364, S1365 is equally effective in either attenuating (SE) or amplifying (SA) the hypertrophic signal stimulated in cardiac myocytes.

To evaluate mTORC1 activation, mTORC1 signaling proteins (p70 p70S6K and 4EBP1) and markers of autophagy (p62 and LC3-II) were measured. Myocytes expressing either WT TSC2 or mutated TSC2 were exposed to ET1 for 48 hours to induce hypertrophy, and cell lysates were examined for mTORC1 signaling proteins and markers of autophagy (Figure 5). Endothelin 1 stimulates mTORC1 activation depicted by increased phosphorylation of p70 p70S6K and 4EBP1), while also increasing both LC3-II and p62 - all consistent with mTORC1 activation and stimulated autophagy. Expression of hsS1364E or hsS1365E reduces mTORC1 activation and p62, while further increasing LC3-II. This is consistent with enhanced autophagy and reduced growth stimulation. By contrast, expression of huS1364A or huS1365A further increases mTORC1 activation (more p70S6K and 4EBP1 phosphorylation, increased p62, reduced LC3-II). These data demonstrate that gene modification of either the first or second serine of the discovered duplet is sufficient to incur amplification (SA) or attenuation (SE) of mTORC1 stimulated activity with associated changes in growth signaling and autophagy.

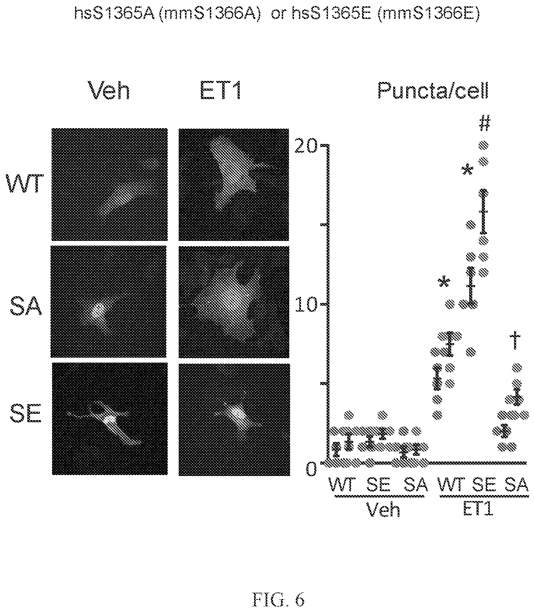

To assess the impact of altering TSC2 with either the relevant targeted phosphorsilencing or phosphor-mimetic mutations, we transfected rat neonatal myocytes with TSC2-wild type, or hsS1365A or hsS1365E TSC2 mutants, and the LC3-GFP-RFP autophagic flux florescent reporter, and then subjected them to endothelin-1 (ET1) stimulation (Figure 6). Cells expressing WT-TSC2 enlarged upon exposure to ET1 and displayed a rise in both green and red punctae reflecting the generation of autophagosomes, and merging of these vesicles with the lysosome (completed autophagy). Cells expressing hsS1365A-TSC2 displayed enhanced hypertrophy with ET1 exposure as compared to WT, and less green and red punctae consistent with reduced autophagy. Cells expressing hsS1365E-TSC2 displayed much less hypertrophy with ET1 exposure as compared to WT or SA, and much more green and red puncae, indicating enhanced autophagy. The relative differences in autophagy are fully compatible with the rise in mTORC1 stimulation, elevated p62 and blunted LC3-II in hsS1365A expressing cells, and suppressed mTORC1 stimulation, reduced p62 and enhanced LC3-II in hsS1365E expressing cells (as shown in Figure 5, lower right and left panels).

## Claims

1. A polypeptide comprising any one of SEQ ID NO: 1; SEQ ID NO: 20; SEQ ID NO: 2 and SEQ ID NO: 21.

2. A nucleic acid encoding a polypeptide of claim 1.

3. A cell comprising a vector comprising the nucleic acid of claim 2, wherein the nucleic acid is operably linked to a nucleic acid that drives expression of the polypeptide in the cell.

4. An engineered immune cell comprising a vector for use in the treatment of a disease in a subj ect;
wherein the vector comprises a nucleic acid encoding a polypeptide comprising SEQ ID NO: 1 or SEQ ID NO: 20 operably linked to a promoter that drives expression of the polypeptide in the T cell; and
wherein upon recognizing an antigen associated with the disease, the engineered immune cell exhibits increased activity as compared to a reference immune cell that lacks the vector; wherein the increased activity comprises increased mTORC1 signalling.

5. The engineered immune cell for use in accordance with claim 4, wherein the engineered immune cell is selected from the group consisting of: a cytotoxic T cell, a chimeric antigen receptor T cell (CAR-T cell), a helper T cell, a regulatory T cell, a macrophage and a neutrophil.

6. The engineered immune cell for use in accordance with claim 5, wherein the increased activity comprises increased expression of one or more cytokines selected from the group consisting of: interferon gamma, tumor necrosis factor alpha, interleukin 2, and combinations thereof.

7. The engineered immune cell for use in accordance with any one of claims 4-6, wherein the engineered immune cell is derived from an endogenous immune cell obtained from the subject.

8. An engineered immune cell comprising a vector for use in generating a persistent T cell in a subject;
wherein the vector comprises a nucleic acid encoding a polypeptide comprising SEQ ID NO: 2 or SEQ ID NO: 21 operably linked to a nucleic acid promoter that drives expression of the polypeptide in the engineered immune cell;
wherein the engineered immune cell recognizes an antigen;
wherein upon recognizing the antigen, the engineered immune cell exhibits decreased activity as compared to a reference immune cell that lacks the vector, wherein the decreased activity of the engineered immune cell comprises decreased mTORC1 signaling; and
wherein upon recognizing the antigen, the engineered immune cell becomes the persistent T cell.

9. The engineered immune cell for use in accordance with claim 8, wherein
the engineered immune cell is derived from an endogenous immune cell obtained from the subject.

10. The engineered immune cell for use in accordance with claim 8 or claim 9, wherein the engineered immune cell is a CD8+ T cell, and wherein the persistent T cell is a memory T cell.

11. The engineered immune cell for use in accordance with claim 10, wherein the CD8+ T cell is further engineered to express a chimeric antigen receptor or a T cell receptor.

12. The engineered immune cell for use in accordance with any one of claims 8-10, wherein the engineered immune cell is a regulatory T cell, and wherein the persistent T cell is a persistent T regulatory cell.

13. A method of generating a persistent T cell *in vitro* comprising:
introducing into an immune cell a nucleic acid encoding a polypeptide comprising SEQ ID NO: 2 or SEQ ID NO: 21 operably linked to a promoter that drives expression of the polypeptide in the immune cell, thereby generating an engineered immune cell;
wherein the engineered immune cell exhibits decreased mTORC1 signaling as compared to a reference immune cell that lacks the vector;
contacting the engineered immune cell with an antigen that is recognized by the engineered immune cell; and
culturing the engineered immune cell under conditions and for a time sufficient such that the engineered immune cell becomes the persistent T cell.

## Patentansprüche

1. Ein Polypeptid, das eine beliebige von SEQ ID NO: 1, SEQ ID NO: 20, SEQ ID NO: 2 und SEQ ID NO: 21 beinhaltet.

2. Eine Nukleinsäure, die ein Polypeptid gemäß Anspruch 1 kodiert.

3. Eine Zelle, die einen Vektor beinhaltet, der die Nukleinsäure gemäß Anspruch 2 beinhaltet, wobei die Nukleinsäure funktionsfähig mit einer Nukleinsäure verbunden ist, die die Expression des Polypeptids in der Zelle antreibt.

4. Eine technisch veränderte Immunzelle, die einen Vektor beinhaltet, zur Verwendung bei der Behandlung einer Krankheit bei einem Individuum;
wobei der Vektor eine Nukleinsäure beinhaltet, die ein Polypeptid, das die SEQ ID NO: 1 oder SEQ ID NO: 20 beinhaltet, kodiert und funktionsfähig mit einem Promotor, der die Expression des Polypeptids in der T-Zelle antreibt, verbunden ist; und
wobei bei Erkennen eines mit der Krankheit assoziierten Antigens die technisch veränderte Immunzelle verglichen mit einer Referenzimmunzelle, der der Vektor fehlt, erhöhte Aktivität zeigt; wobei die erhöhte Aktivität erhöhte mTORC1 -Signalisierung beinhaltet.

5. Technisch veränderte Immunzelle zur Verwendung gemäß Anspruch 4, wobei die technisch veränderte Immunzelle aus der Gruppe ausgewählt ist, die aus Folgendem besteht: einer zytotoxischen T-Zelle, einer Chimärer-Antigenrezeptor-T-Zelle (CAR-T-Zelle), einer T-Helferzelle, einer regulatorischen T-Zelle, einem Makrophagen und einem Neutrophilen.

6. Technisch veränderte Immunzelle zur Verwendung gemäß Anspruch 5, wobei die erhöhte Aktivität erhöhte Expression von einem oder mehreren Zytokinen beinhaltet, die aus der Gruppe ausgewählt sind, die aus Folgendem besteht: Interferon-gamma, Tumornekrosefaktor-alpha, Interleukin-2 und Kombinationen davon.

7. Technisch veränderte Immunzelle zur Verwendung gemäß einem der Ansprüche 4-6, wobei die technisch veränderte Immunzelle von einer endogenen Immunzelle abgeleitet ist, die von dem Individuum erhalten wurde.

8. Eine technisch veränderte Immunzelle, die einen Vektor zur Verwendung beim Erzeugen einer persistierenden T-Zelle in einem Individuum beinhaltet;
wobei der Vektor eine Nukleinsäure beinhaltet, die ein Polypeptid, das die SEQ ID NO: 2 oder SEQ ID NO: 21 beinhaltet, kodiert und funktionsfähig mit einem Nukleinsäurepromotor, der die Expression des Polypeptids in der technisch veränderten Immunzelle antreibt, verbunden ist;
wobei die technisch veränderte Immunzelle ein Antigen erkennt;
wobei bei Erkennen des Antigens die technisch veränderte Immunzelle verglichen mit einer Referenzimmunzelle, der der Vektor fehlt, verringerte Aktivität zeigt, wobei die verringerte Aktivität der technisch veränderten Immunzelle verringerte mTORC1-Signalisierung beinhaltet; und
wobei bei Erkennen des Antigens die technisch veränderte Immunzelle zu der persistierenden T-Zelle wird.

9. Technisch veränderte Immunzelle zur Verwendung gemäß Anspruch 8, wobei die technisch veränderte Immunzelle von einer endogenen Immunzelle abgeleitet ist, die von dem Individuum erhalten wurde.

10. Technisch veränderte Immunzelle zur Verwendung gemäß Anspruch 8 oder Anspruch 9, wobei die technisch veränderte Immunzelle eine CD8+-T-Zelle ist und wobei die persistierende T-Zelle eine T-Gedächtniszelle ist.

11. Technisch veränderte Immunzelle zur Verwendung gemäß Anspruch 10, wobei die CD8+-T-Zelle ferner technisch verändert ist, um einen chimären Antigenrezeptor oder einen T-Zellrezeptor zu exprimieren.

12. Technisch veränderte Immunzelle zur Verwendung gemäß einem der Ansprüche 8-10, wobei die technisch veränderte Immunzelle eine regulatorische T-Zelle ist und wobei die persistierende T-Zelle eine persistierende regulatorische T-Zelle ist.

13. Ein Verfahren zum Erzeugen einer persistierenden T-Zelle *in vitro,* das Folgendes beinhaltet:
Einführen einer Nukleinsäure, die ein Polypeptid, das die SEQ ID NO: 2 oder SEQ ID NO: 21 beinhaltet, kodiert und funktionsfähig mit einem Promotor, der die Expression des Polypeptids in der Immunzelle antreibt, verbunden ist, in eine Immunzelle, wodurch eine technisch veränderte Immunzelle erzeugt wird;
wobei die technisch veränderte Immunzelle verglichen mit einer Referenzimmunzelle, der der Vektor fehlt, verringerte mTORC1-Signalisierung zeigt;
In-Kontakt-Bringen der technisch veränderten Immunzelle mit einem Antigen, das von der technisch veränderte Immunzelle erkannt wird; und
Kultivieren der technisch veränderten Immunzelle unter Bedingungen und für eine Zeitdauer, die ausreichen, sodass die technisch veränderte Immunzelle zu der persistierenden T-Zelle wird.

## Revendications

1. Un polypeptide comprenant l'une quelconque parmi SEQ ID N° : 1 ; SEQ ID N° : 20 ; SEQ ID N° : 2 et SEQ ID N° : 21.

2. Un acide nucléique codant pour un polypeptide de la revendication 1.

3. Une cellule comprenant un vecteur comprenant l'acide nucléique de la revendication 2, où l'acide nucléique est lié de façon opérationnelle à un acide nucléique qui conduit l'expression du polypeptide dans la cellule.

4. Une cellule immunitaire modifiée par ingénierie comprenant un vecteur pour son utilisation dans le traitement d'une maladie chez un sujet ;
où le vecteur comprend un acide nucléique codant pour un polypeptide comprenant SEQ ID N° : 1 ou SEQ ID N° : 20 lié de façon opérationnelle à un promoteur qui conduit l'expression du polypeptide dans la cellule T ; et
où après avoir reconnu un antigène associé à la maladie, la cellule immunitaire modifiée par ingénierie présente une activité accrue comparée à une cellule immunitaire de référence qui est dépourvue du vecteur ; où l'activité accrue comprend une signalisation de mTORC1 accrue.

5. La cellule immunitaire modifiée par ingénierie pour son utilisation conformément à la revendication 4, la cellule immunitaire modifiée par ingénierie étant sélectionnée dans le groupe constitué de : une cellule T cytotoxique, une cellule T portant un récepteur antigénique chimérique (cellule CAR-T), une cellule T auxiliaire, une cellule T régulatrice, un macrophage et un neutrophile.

6. La cellule immunitaire modifiée par ingénierie pour son utilisation conformément à la revendication 5, où l'activité accrue comprend l'expression accrue d'une ou de plusieurs cytokines sélectionnées dans le groupe constitué de : l'interféron gamma, le facteur de nécrose tumorale alpha, l'interleukine 2, et des combinaisons de ceux-ci.

7. La cellule immunitaire modifiée par ingénierie pour son utilisation conformément à l'une quelconque des revendications 4 à 6, la cellule immunitaire modifiée par ingénierie étant dérivée d'une cellule immunitaire endogène prélevée sur le sujet.

8. Une cellule immunitaire modifiée par ingénierie comprenant un vecteur pour son utilisation dans la génération d'une cellule T persistante chez un sujet ;
où le vecteur comprend un acide nucléique codant pour un polypeptide comprenant SEQ ID N° : 2 ou SEQ ID N° : 21 lié de façon opérationnelle à un acide nucléique promoteur qui conduit l'expression du polypeptide dans la cellule immunitaire modifiée par ingénierie ;
où la cellule immunitaire modifiée par ingénierie reconnaît un antigène ;
où après avoir reconnu l'antigène, la cellule immunitaire modifiée par ingénierie présente une activité décrue comparée à une cellule immunitaire de référence qui est dépourvue du vecteur, où l'activité décrue de la cellule immunitaire modifiée par ingénierie comprend une signalisation de mTORC1 décrue ; et
où après avoir reconnu l'antigène, la cellule immunitaire modifiée par ingénierie devient la cellule T persistante.

9. La cellule immunitaire modifiée par ingénierie pour son utilisation conformément à la revendication 8, la cellule immunitaire modifiée par ingénierie étant dérivée d'une cellule immunitaire endogène prélevée sur le sujet.

10. La cellule immunitaire modifiée par ingénierie pour son utilisation conformément à la revendication 8 ou à la revendication 9, la cellule immunitaire modifiée par ingénierie étant une cellule T CD8+, et la cellule T persistante étant une cellule T mémoire.

11. La cellule immunitaire modifiée par ingénierie pour son utilisation conformément à la revendication 10, où la cellule T CD8+ est modifiée plus avant par ingénierie afin d'exprimer un récepteur antigénique chimérique ou un récepteur des cellules T.

12. La cellule immunitaire modifiée par ingénierie pour son utilisation conformément à l'une quelconque des revendications 8 à 10, la cellule immunitaire modifiée par ingénierie étant une cellule T régulatrice, et la cellule T persistante étant une cellule T régulatrice persistante.

13. Un procédé de génération d'une cellule T persistante *in vitro* comprenant :
l'introduction au sein d'une cellule immunitaire d'un acide nucléique codant pour un polypeptide comprenant SEQ ID N° : 2 ou SEQ ID N° : 21 lié de façon opérationnelle à un promoteur qui conduit l'expression du polypeptide dans la cellule immunitaire, générant par là une cellule immunitaire modifiée par ingénierie ;
où la cellule immunitaire modifiée par ingénierie présente une signalisation de mTORC1 décrue comparée à une cellule immunitaire de référence qui est dépourvue du vecteur ;
la mise en contact de la cellule immunitaire modifiée par ingénierie avec un antigène qui est reconnu par la cellule immunitaire modifiée par ingénierie ; et
la mise en culture de la cellule immunitaire modifiée par ingénierie dans des conditions et pendant une durée suffisantes pour que la cellule immunitaire modifiée par ingénierie devienne la cellule T persistante.
